# FASCICULE DE BREVET EUROPEEN

(11) **EP 1 773 854 B1**
(45) Date de publication et mention de la délivrance du brevet: **21.09.2011**
(21) Numéro de dépôt: 05790838.6
(22) Date de dépôt: 20.07.2005
(51) Int. Cl.: C07H 3/06, A61K 31/7008, A61K 31/702, A61P 35/00

(54) **DERIVES D'AZASUCRE, INHIBITEURS D'HEPARANASES, LEUR PROCEDE DE PREPARATION, LES COMPOSITIONS EN CONTENANT, LEUR UTILISATION**
AZAZUCKER-DERIVATE, HEPARANASE-HEMMER, VERFAHREN ZU DEREN HERSTELLUNG, ZUSAMMENSETZUNGEN DAMIT UND VERWENDUNG
AZASUGAR DERIVATIVES, HEPARANASE INHIBITORS, METHOD FOR PREPARING SAME, COMPOSITIONS CONTAINING SAME, USE THEREOF

(30) Priorité: 23.07.2004 FR 0408160
(43) Date de publication de la demande: 18.04.2007
(73) Titulaire: SANOFI, 75013 Paris (FR)
(72) Inventeur: DRIGUEZ, Pierre, Alexandre, F-31500 Toulouse (FR); PETITOU, Maurice, F-75645 Paris Cedex 13 (FR)
(74) Mandataire: Jacobson, Claude
(86) Numéro de dépôt international: PCT/FR2005/001851
(87) Numéro de publication internationale: WO 2006/021653

(56) Documents cités:
- TAKAHASHI S ET AL: "Design and synthesis of a heparanase inhibitor with pseudodisaccharide structure" TETRAHEDRON, ELSEVIER SCIENCE PUBLISHERS, AMSTERDAM, NL, vol. 57, no. 32, 6 août 2001 (2001-08-06), pages 6915-6926, XP004275148 ISSN: 0040-4020
- IGARASHI Y ET AL: "Synthesis Of A Potent Inhibitor Of beta-Glucuronidase" TETRAHEDRON LETTERS, ELSEVIER SCIENCE PUBLISHERS, AMSTERDAM, NL, vol. 37, no. 16, 15 avril 1996 (1996-04-15), pages 2707-2708, XP004029775 ISSN: 0040-4039
- Y. ICHIKAWA ET AL.: "1-N-Iminosugars: Potent and selective inhibitors of beta-glycosidases" J. AM. CHEM. SOC., vol. 120, 1998, pages 3007-3018, XP002323260 cité dans la demande

## Description

L'invention a pour objet des dérivés d'azasucre, inhibiteurs d'héparanases, leur préparation, les compositions en contenant et leur application en thérapeutique.

Les héparanases sont des enzymes de type endoglucuronidases qui ont pour substrat des polysaccharides de la famille héparine/héparanes sulfate (HS). On connaît des héparanases de type I est II (McKenzie et al., Biochem. Biophys. Res. Commun., (2000), Viol.276, p. 1170-1177). Elles hydrolysent spécifiquement des liaisons de type β-1→4 entre une unité saccharidique de type acide D-glucuronique et une unité saccharidique de type D-glucosamine et libèrent des fragments d'HS d'environ 10 à 20 unités saccharidiques (Pikas, D.S et al., J. Biol. Chem., (1998), Vol.273, p.18770-18777). Les héparanases dégradent de la même façon les chaînes polysaccharidiques des protéoglycanes contenant des héparanes sulfates (HSPGs, pour Heparan Sulfate Proteoglycans) (Vlodavsky et Friedmann, J. Clin. Invest., (2001), Vol.108, p. 341-347). Les HSPGs sont constitués d'une protéine centrale à laquelle sont liées de façon covalente des chaînes linéaires d'HS (Kjellen et al., Annu. Rev. Biochem., (1991), Vol.60, p.443-475). Les HSPGs sont des macromolécules ubiquitaires. Comme les HS, les HSPGs sont présents à la surface de nombreux types cellulaires et dans la matrice extracellulaire (ECM pour Extracellular Matrix) (Kjellen *et al.,* (1991), *ibid. ;* Bernfield et al., Annu. Rev. Biochem., (1999), Vol.68, p.729-777 ; David et al., FASEB J., (1993), Vol.7, p.1023-1030 ; Lozzo et al., Annu. Rev. Biochem., (1998), Vol.67, p.609-652). La ECM, composant majeur des tissus conjonctifs des vertébrés et invertébrés, occupe l'environnement extracellulaire. Elle enveloppe les organes et entoure les endothéliums, notamment les endothéliums capillaires (Wight et al., Arteriosclerosis, (1989), Vol.9, p.1-20), jouant ainsi un rôle de maintien et de barrière de protection des organes et endothéliums (McKenzie et al., Biochem. J ; 2003 ; Vol.373; p.423-435). La ECM est également un modulateur clé, impliqué dans différents mécanismes cellulaires, notamment la différenciation et la réparation cellulaire (Folkman et al., Adv. Exp. Med. Biol., (1992), Vol. 313, p. 355-364).
Des composés inhibiteurs d'héparanases ont été décrits dans l'art antérieur. Par exemple, la demande de brevet internationale WO 02/0600374 décrit des dérivés benz-1,3-azole, la demande de brevet internationale WO 03/074516 concerne des dérivés d'acide phtalimide carboxylique et benzoxazole, la demande de brevet internationale WO 041013132 décrit des dérivés de furanethiazoles ou encore la demande de brevet internationale WO 041046123 décrit des dérivés benzoxazole, benzthiazole et benzimidazole.
La synthèse de dérivés d'azasucres à chaîne courte (2 motifs) dans lesquels l'atome d'azote remplace l'atome d'oxygène en position 5 a été décrite dans Takahashi et al, Chem. Lett., (1994), Vol.11, p. 2119; Takahashi et al, Tetrahedron, (2001), Vol.57, p.6915-6926). Cependant, leurs activités in vivo n'a pas été identifiée.

Des dérivés d'azasucres à un seul motif, de formule suivante : 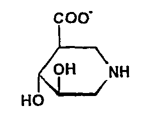 ont déjà été décrits (brevet US 6,583,158 ; Ichikawa et al., J. Amer. Chem. Soc., (1998), Vol. 120, p. 3007).

Il existe toujours une nécessité de trouver et de développer des produits présentant une bonne activité *in vitro* et *in vivo.*

Il a maintenant été trouvé, de manière surprenante, que des dérivés d'azasucre de synthèse présentent une bonne activité en tant qu'inhibiteurs d'héparanases. La présente invention concerne donc de nouveaux dérivés d'azasucre, inhibiteurs d'héparanases. Ces nouveaux composés présentent une bonne activité inhibitrice d'héparanases.

L'objet de l'invention concerne les composés répondant à la formule générale (I) : 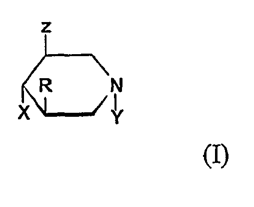 dans laquelle :
R représente un atome d'hydrogène, un radical hydroxyle, un radical -OSO₃⁻, un radical -O-(C₁-C₅)alkyle ou un radical -O-aralkyle ;
Z représente un radical COO⁻ ou un radical hydroxyle
X représente un radical hydroxyle ou une unité saccharidique de formule A : 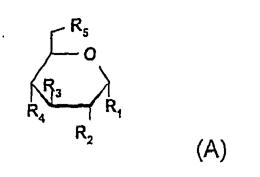 dans laquelle :
   - R₁ représente un atome d'oxygène, permettant à A de se lier à l'unité azasucre ou à une autre unité saccharidique,
   - R₂ représente un radical -NH₂, un radial -NHCO(C₁-C₅)alkyle, un radical -NHCOaryle, un radical -NHSO₃⁻, un radical hydroxyle, un radical -O-(C₁-C₅)alkyle, un radical -O-aralkyle ou un radical -OSO₃⁻,
   - R₃ représente un radical hydroxyle, un radical -OSO₃⁻, un radical -O-(C₁-C₅)alkyle ou un radical -O-aralkyle,
   - R₄ représente un radical hydroxyle, un radical -OSO₃⁻, un radical -O-(C₁-C₅)alkyle, un radical -O-aralkyle ou une unité saccharidique de formule B : 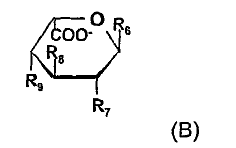 dans laquelle :
      - R₆ représente un atome d'oxygène, permettant à B de se lier à une autre unité saccharidique de formule A,
      - R₇ et R₈ ont la même définition que R₃ tel que défini précédemment,
      - R₉ représente un radical hydroxyle, un radical -OSO₃⁻, un radical -O-(C₁-C₅)alkyle, un radical -O-aralkyle ou une unité saccharidique de formule A telle que définie précédemment,
   - R₅ a la même définition que R₃ tel que défini précédemment ;
Y représente un atome d'hydrogène, un radical (C₁-C₅)alkyle ou une unité saccharidique de formule D 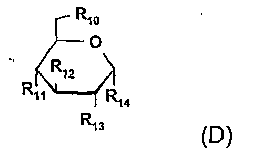 dans laquelle :
   - R₁₀, R₁₂ et R₁₃ ont respectivement les mêmes définitions que R₅, R₃ et R₂ tels que définis précédemment,
   - R₁₁ représente :
      - un radical (C₁-C₃)alkylène permettant à D de se greffer sur l'unité azasucre ou
      - un atome d'oxygène permettant à D de se greffer sur une autre unité saccharidique,
   - R₁₄ représente un radical -O-(C₁-C₅)alkyle ou un radical -O-E dans laquelle E représente un radical de formule suivante : 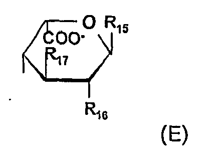 dans laquelle :
      - R₁₅ représente un radical -O-(C₁-C₅)alkyle, un radical -O-aralkyle ou une unité saccharidique de formule D dans laquelle R₁₁ représente un atome d'oxygène,
      - R₁₆ et R₁₇ ont la même définition que R₃ tel que défini précédemment,
à la condition toutefois que lorsque X et R représentent chacun un radical hydroxyle, Y ne représente pas un atome d'hydrogène,
et étant entendu que le nombre d'unités saccharidiques comportées par le composé de formule (I) est compris entre 1 et 10,
sous forme libre ou sous forme de sels formés avec une base ou un acide pharmaceutiquement acceptables ainsi que sous forme de solvats ou d'hydrates.

Selon un de ses aspects préférés, l'invention concerne les composés de formule générale (I): 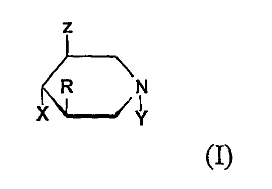 dans laquelle :
R représente un radical hydroxyle ;
Z représente un radical COO⁻ ou un radical hydroxyle
X représente un radical hydroxyle ou une unité saccharidique de formule A : 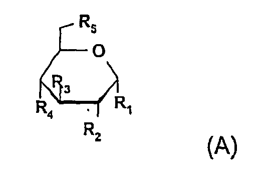 dans laquelle :
   - R₁ représente un atome d'oxygène,
   - R₂ représente un radical -NHCOCH₃, un radical -NHSO₃⁻, un radical -OSO₃⁻,
   - R₃ représente un radical hydroxyle ou un radical -O-(C₁-C₅)alkyle,
   - R₄ représente un radical hydroxyle, un radical -O-aralkyle ou une unité saccharidique de formule B : 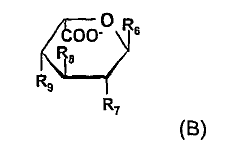 dans laquelle :
      - R₆ représente un atome d'oxygène,
      - R₇ représente un radical -OSO₃⁻,
      - R₈ représente un radical hydroxyle, un radical -O-(C₁-C₅)alkyle ou un radical -O-aralkyle,
      - R₉ représente un radical -OSO₃⁻, un radical -O-aralkyle, un radical -O(C₁-C₅)alkyle ou une unité saccharidique de formule A telle que définie précédemment,
   - R₅ représente un radical -OSO₃⁻,
Y représente un atome d'hydrogène ou une unité saccharidique de formule D : 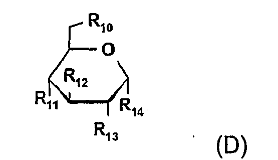 dans laquelle :
   - R₁₀ a la même définition que R₅ tel que défini précédemment,
   - R₁₂ représente un radical hydroxyle ou un radical -OSO₃⁻,
   - R₁₃ représente un radical -NHSO₃⁻,
   - R₁₁ représente un radical méthylène lié à l'unité azasucre ou un atome d'oxygène lié à E,
   - R₁₄ un radical -OCH₃ ou un radical de formule -O-E dans laquelle E représente un radical de formule : 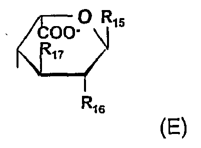 dans laquelle :
      - R₁₅ représente une unité D dans laquelle R₁₁ représente un atome d'oxygène permettant de lier E à D,
      - R₁₆ représente un radical -OSO₃⁻,
      - R₁₇ représente un radical hydroxyle,
étant entendu que le nombre d'unités saccharidiques comportées par le composé de formule (I) est compris entre 2 et 10,
sous forme libres ou sous forme de sels avec une base ou un acide pharmaceutiquement acceptables ainsi que sous forme de solvats ou d'hydrates.

Des composés particulièrement préférés sont des composés de formule 1 dans laquelle Y est un atome d'hydrogène.

L'invention englobe les dérivés azasucres sous leur forme acide ou sous la forme de l'un quelconque de leurs sels pharmaceutiquement acceptables. Dans la forme acide, les fonctions -COO⁻ et -SO₃⁻ sont respectivement sous forme -COOH et -SO₃H.

Selon un de ses aspects particulièrement préférés, la présente invention concerne les composés suivant :
- (2,4-di-O-sodium sulfonato-α-idopyranosyluronate de sodium)-(1-4)-(2-acétamido-2-désoxy-6-O-sodium sulfonato-α-D-glucopyranosyl)-(1-4)-(2-O-sodium sulfonato-α-L-idopyranosyluronate de sodium)-(1-4)-(2-acétamido-2-désoxy-6-O-sodium sulfonato-α-D-glucopyranosyl)-(1-4)-(5-(hydroxy)-4-oxypipéridine-3-carboxylate de sodium (3S, 4R, 5R)) (composé n° 20)
- (2,4-di-O-sodium sulfonato-α-L-idopyranosyluronate de sodium)-(1-4)-(2-N-sodium sulfonato-2-désoxy-6-O-sodium sulfonato-α-D-glucopyranosyl)-(1-4)-(2-O-sodium sulfonato-α-L-idopyranosyluronate de sodium)-(1-4)-(2-N-sodium sulfonato-2-désoxy-6-O-sodium sulfonato-α-D-glucopyranosyl)-(1-4)-(5-(hydroxy)-4-oxypipéridine-3-carboxylate de sodium (3S, 4R, 5R)) (composé n°27)
- (3-O-méthyl-2,4-di-O-sodium sulfonato-α-L-idopyranosyluronate de sodium)-(1-4)-(3-O-méthyl-2,6-di-O-sodium sulfonato-α-D-glucopyranosyl)-(1-4)-(3-O-méthyl-2-O-sodium sulfonato-α-L-idopyranosyluronate de sodium)-(1-4)-(3-O-méthyl-2,6-di-O-sodium sulfonato-α-D-glucopyranosyl)-(1-4)-(5-(hydroxy)-4-oxypipéridine-3-carboxylate de sodium (3S, 4R, 5R)) (composé n°47)
- (2,4-di-O-sodium sulfonato-α-L-idopyranosyluronate de sodium)-(1-4)-(2,6-di-O-sodium sulfonato-α-D-glucopyranosyl)-(1-4)-(2-O-sodium sulfonato-α-L-idopyranosyluronate de sodium)-(1-4)-(2,6-di-O-sodium sulfonato-α-D-glucopyranosyl)-(1-4)-(5-(hydroxy)-4-oxypipéridine-3-carboxylate de sodium (3S, 4R, 5R)) (composé n° 69)
- (4-O-propyl-2-O-sodium sulfonato-α-L-idopyranosyluronate de sodium)-(1-4)-(2,6-di-O-sodium sulfonato-α-D-glucopyranosyl)-(1-4)-(2-O-sodium suffonato-α-L-idopyranosyluronate de sodium)-(1-4)-(2,6-di-O-sodium sulfonato-α-D-glucopyranosyl)-(1-4)-(5-(hydroxy)-4-oxypipéridine-3-carboxylate de sodium (3S, 4R, 5R)) (composé n°74).

- (2,4-di-O-sodium sulfonato-α-L-idopyranosyluronate de sodium)-(1-4)-(2-acétamido-2-désoxy-6-O-sodium sulfonato-α-D-glucopyranosyl)-(1-4)-(2-O-sodium sulfonato-α-L-idopyranosyluronate de sodium)-(1-4)-(2-acétamido-2-désoxy-6-O-sodium sulfonato-α-D-glucopyranosyl)-(1-4)-(3-(hydroxy)-5-hydroxyméthyl-4-oxypipéridine (3R, 4R, 5R)) (composé n°123).
- (4-O-phény(propyl-2-O-sodium sulfonato-α-L-idopyranosyluronate de sodium)-(1-4)-(2-acétamido-2-désoxy-6-O-sodium sulfonato-α-D-glucopyranosyl)-(1-4)-(2-O-sodium sulfonato-α-L-idopyranosyluronate de sodium)-(1-4)-(2-acétamido-2-désoxy-6-O-sodium sulfonato-α-D-glucopyranosyl)-(1-4)-(5-(hydroxy)-4-oxypipéridine-3-carboxylate de sodium (3S, 4R, 5R)) (composé n°124).

Dans le cadre de la présente invention :
- un radical (C₁-C₅)alkyle représente un radical aliphatique, pouvant comporter de 1 à 5 atomes de carbone, saturé linéaire ou ramifié. A titre d'exemples, on peut citer les radicaux méthyle, éthyle, propyle, isopropyle, butyle, isobutyle, terbutyle, ...,
- un radical -O-aralkyle représente un groupement alkyle tel que défini ci-dessus mais substitué par un radical aromatique pouvant lui même porter des substituants. A titre d'exemple on peut citer le p-methoxy-benzyl, phénymethyl, phényléthyl, phénylpropyl, ...,
- un radical (C₁-C₃)alkylène représente un groupe alkyle bivalent comportant de 1 à 3 atomes de carbone.
On entend par sel pharmaceutiquement acceptable des dérivées azasucres de l'invention, un dérivé azasucre dans lequel une ou plusieurs des fonctions -COO- ou/et -SO₃⁻ sont liées de façon ionique à un cation pharmaceutiquement acceptable. Les sels préférés selon l'invention sont ceux dont le cation est choisi parmi les cations des métaux alcalins et plus préférablement encore ceux dont le cation est Na⁺ ou K⁺.

Dans son principe le procédé de préparation des composés selon l'invention utilise des synthons de base mono-, di- ou oligosaccharidiques préparés comme précédemment rapporté dans la littérature et choisis en tenant compte notamment de l'orthogonalité des groupes protecteurs. On se réfèrera notamment à EP 300099, EP 529715, EP 621282 et EP 649854 ainsi qu'à C. van Boeckel, M. Petitou, Angew. Chem. Int. Ed. Engl., (1993), Vol.32, p.1671-1690. Ces synthons sont ensuite couplés les uns aux autres de façon à fournir un équivalent entièrement protégé d'un composé selon l'invention. Cet équivalent protégé est ensuite transformé en un composé selon l'invention en utilisant des procédés bien connus de l'homme de l'art.

Les composés de l'invention contenant en outre un motif azasucre (ou pipéridine substituée), leur synthèse requiert la préparation d'un précurseur de ce motif portant des groupements protecteurs compatibles avec les couplages ultérieurs à des mono-, di-, ou oligosaccharides. Les précurseurs des azasucres sont préparés selon des procédés décrits dans la littérature. On se réfèrera en particulier à l'ouvrage « Iminosugars as Glycosidase Inhibitors », AE Stütz, Wiley-VCH, 1999.

Lorsque les synthons nécessaires à l'assemblage de la chaîne sont disponibles, on procède aux couplages de ces synthons entre eux. Dans les réactions de couplage évoquées, un synthon "donneur", activé sur son carbone anomère, réagit avec un synthon "accepteur", possédant au moins un hydroxyle libre.

La présente invention concerne un procédé pour la préparation des composés de formule (I) caractérisé en ce que : dans une première étape, on prépare un équivalent complètement protégé du composé (I) désiré; dans une seconde étape, les groupes chargés négativement (carboxylates, sulfonates) ainsi que les hydroxyles libres sont introduits et/ou démasqués.

La synthèse du précurseur est réalisée selon des réactions bien connues de l'homme de l'art en utilisant en particulier les méthodes de la synthèse osidique (G.J. Boons, Tetrahedron, (1996), Vol.52, p.1095-1121 ; WO98/03554 et WO99/36443) selon lesquelles un oligosaccharide donneur de liaison glycosidique est couplé avec un oligosaccharide accepteur de liaison glycosidique pour conduire à un autre oligosaccharide dont la taille est égale à la somme des tailles des deux espèces réactives. Cette séquence est répétée jusqu'à l'obtention du composé de formule (I) désiré. La structure du composé final désiré détermine la nature des entités chimiques utilisées dans les différentes étapes de la synthèse, de façon à contrôler la stéréochimie et la régiosélectivité, selon les règles bien connues de l'homme de l'art.

Les composés de l'invention sont obtenus à partir de leurs précurseurs polysaccharidiques complètement protégés en utilisant, en général, l'enchaînement suivant de réactions :
- Libération et substitution des fonctions devant être transformées en groupes N-et/ou *O*-sulfonate.
- Libération des autres fonctions et assemblage éventuel des fragments entre eux, par exemple par une réaction d'amination réductrice.
La libération des fonctions acides carboxyliques peut être effectuée à différents stades de ce procédé.

Les composés de l'invention peuvent naturellement être préparés en utilisant différentes stratégies connues par l'homme de l'art de la synthèse osidique et organique.

Le procédé décrit ci-dessus est le procédé préféré de l'invention. Toutefois, les composés de formule (I) peuvent être préparés par d'autres méthodes bien connues de la chimie des sucres décrites par exemple dans « Monosaccharides, Their chemistry and their roles in natural products », P.M. Collins et R.J. Ferrier, J. Wiley & sons, 1995 et dans G.J. Boons, Tetrahedron, (1996), Vol.52, p.1095-1121.

Les groupes protecteurs utilisés dans le procédé de préparations des composés (I) sont ceux couramment utilisés dans la chimie des sucres par exemple dans Protective Groups in Organic Synthesis, TW Greene, PGM Wuts, John Wiley & sons, New-York, 1999.
Les groupes protecteurs sont avantageusement choisis par exemple parmi les groupes acétyle, halogénométhyle, benzoyle, lévulinyle, benzyle, benzyle substitué, trityle éventuellement substitué; carbamate, tétrahydropyranyle, allyle, pentenyle, tert-butyldiméthylsilyle (tBDMS) ou triméthylsilyléthyle.
Les groupes activateurs sont ceux classiquement utilisés en chimie des sucres selon par exemple G.J. Boons, Tetrahedron, (1996), Vol.52, p.1095-1121. Ces groupes activateurs sont choisis par exemple parmi les imidates, les thioglycosides, les penténylglycosides, les xanthates, les phosphites ou les halogénures.

Le procédé décrit ci-dessus permet d'obtenir les composés de l'invention sous forme de sels. Pour obtenir les acides correspondants, les composés de l'invention sous forme de sels, sont mis en contact avec une résine échangeuse de cations sous forme acide.
Les composés de l'invention sous forme d'acides peuvent être ensuite neutralisés par une base pour obtenir le sel souhaité. Pour la préparation des sels des composés de formule (I), on peut utiliser toute base minérale ou organique, donnant avec les composés de formule (I), des sels pharmaceutiquement acceptables. On utilise de manière préférentielle comme base l'hydroxyde de sodium, de potassium, de calcium ou de magnésium. Les sels de sodium et de calcium des composés de formule (I), sont les sels préférés.

Les composés selon l'invention ont fait l'objet d'études biochimiques et pharmacologiques. Les tests qui suivent, non limitatifs, illustrent la présente invention

On définit les termes suivants :
PET : polyethylene terephtalate,
AM : acetoxymethyl,
DMEM : milieu de Eagle modifié par Dulbecco,
EDTA : ethylenediaminetetraacetic acid,
Tris : tris(hydroxyméthyl)aminomethane,
AT : Antithrombine III,
nkat : nanokatal = unité de mesure enzymatique (donnée par le fabriquant), représentant la quantité de substrat catalysé par unité de temps.

### 1. Evaluation de l'activité des inhibiteurs d'héparanases dans un système enzymatique (détermination des CI₅₀ des composés selon l'invention).

L'activité de l'héparanase est mise en évidence par sa capacité à dégrader le fondaparinux. La concentration de fondaparinux est déterminée grâce à son activité anti-facteur Xa.

### A. Matériel et méthodes

L'héparanase est produit par Sanofi-Synthélabo (Labège, France).
Les réactifs de dosages du facteur Xa sont commercialisés par Chromogénix (Montpellier, France).

Des concentrations croissantes d'un composé selon l'invention, inhibiteur d'héparanases (dilutions variables: de 1nM à 10 µM) sont mélangées à une concentration fixe d'héparanase (pour chaque lot, des expériences préliminaires permettent de déterminer l'activité enzymatique suffisante à la dégradation de 0,45µg/ml de fondaparinux ajouté). Après 5 minutes à 37°C le mélange est mis en présence du fondaparinux et laissé 1 heure à 37°C. La réaction est arrêtée par chauffage à 95 °C pendant 5 minutes. La concentration de fondaparinux résiduelle est enfin mesurée par addition de facteur Xa et de son substrat chromogène spécifique (Ref. S2222).

Les différents mélanges sont effectués selon la procédure qui suit :

### a) Mélange réactionnel

On mélange 50 µl de tampon acétate de sodium (0.2 M, pH 4.2), avec 50 µl de fondaparinux (0.45 µg/ml) et 59 µl d'une solution d'héparanase). On incube 1 heure à 37 °C, puis 5 minutes à 95°C. On mélange ensuite 100 µl du mélange réactionnel avec 50 µl de tampon Tris 50 mM, NaCl 175 mM EDTA 75 mM, pH 14. On passe ainsi du pH 4.2 à pH 7.

L'activité anti-facteur Xa du fondaparinux est mesurée de la façon qui suit :

### b) Dosage de l'activité anti-facteur Xa du fondaparinux

On mélange 100 µl de la solution obtenue à l'étape a) avec 100 µl d'AT (0.5 µ/ml).
On maintient pendant 2 minutes à 37°C et on ajoute ensuite 100 µl de facteur Xa (7nkat/ml). On maintient pendant 2 minutes à 37°C et on ajoute ensuite 100 µl de substrat chromogénique (Ref. : S2222) (1 mM). On maintient pendant 2 minutes à 37 °C et on ajoute ensuite 100 µl d'acide acétique (50 %).
La lecture de la densité optique s'effectue à 405 nm.

Un pourcentage d'inhibition est déterminé par rapport au contrôle sans inhibiteur. Une courbe des pourcentages d'inhibition permet de calculer une CI₅₀.

### B. Résultats

Les composés selon la présente invention présentent des CI₅₀ comprises entre 10 nM et 10 µM.
Par exemple, le composé n° 27 présente une CI₅₀ de 11 ± 4 nM (moyenne ± SD, faite sur deux essais)

### 2. Effet des inhibiteurs d'héparanases sur l'invasion des cellules tumorales HT1080

L'effet des composés de formule (I), inhibiteurs d'héparanases, a été testé *in vitro* sur l'invasion des cellules tumorales HT1080.

### A. Matériel et méthodes :

### a) Culture cellulaire:

Les cellules issues de fibrosarcomes humains, HT1080 (ATCC CCL-121) sont cultivées dans un milieu DMEM (Ref.: GIBCO 11960-044) contenant 5% de Sérum de Veau Foetal, de la glutamine (2 mM) (Ref. : GIBCO 25030-024), du pyruvate de sodium (1 mM) (Ref. : GIBCO 11360-039) et des acides aminés non essentiels (1X) (Ref. : GIBCO 11140-035), sur flasks coatés collagène (Becton Dickinson 75 cm² ; Ref. 354523), jusqu'à 50 à 80% de confluence.

### b) Test d'invasion cellulaire

Les mesures d'invasion des HT1080 sont réalisées sur kit Becton Dickinson falcon HTS Fluoroblok Multiwell Insert System en plaques de 24 puits (Ref. : 351158).
Ces chambres de mesure fournissent aux cellules des conditions permettant d'évaluer leurs propriétés invasives *in vitro.*
Le kit est composé d'une plaque associée à des inserts de culture contenant une membrane PET trouée de pores de 8 microns sur laquelle on dépose une couche uniforme de Matrigel Matrix (Becton Dickinson ; Ref. 354230).
Le matrigel est une membrane basale soluble extraite de la tumeur EHS (Engelbreth-Holm-Swarm) qui, grâce à sa composition, forme en se solidifiant une structure équivalente à une membrane basale.
La couche de Matrigel occlue les pores de la membrane, bloquant ainsi la migration des cellules non-invasives au travers de la membrane. Au contraire, les cellules invasives (tumorales ou non tumorales) seront capables de se détacher et d'envahir la couche de Matrigel avant de migrer à travers la membrane.
La quantification de la migration cellulaire est réalisée par marquage à la calcéine AM (Molecular Probes C-3100). Le signal fluorescent émis est mesuré à l'aide d'un lecteur Perkin Elmer Wallac VICTOR 3 et peut être directement corrélé avec le nombre de cellules ayant envahi le gel de Matrigel. En les comparant à des contrôles effectués dans la même expérience que les produits étudiés (réponse en présence de 0% et 5 % de sérum de veau foetal), il est possible de déterminer un pourcentage d'inhibition de l'invasion cellulaire en présence des produits.

### B. Résultats

Une série de déterminations indépendantes (variant de 2 à 4, a permis de montrer qu'à une concentration comprise entre 1 nM et 10 µM, les composés selon l'invention inhibent en moyenne l'invasion cellulaire avec un pourcentage compris entre 8 et 60 %.

Par exemple, une série de quatre déterminations indépendantes a permis de montrer que 10 µM du composé n° 20 inhibent en moyenne l'invasion cellulaire avec un pourcentage égal à 40.3 ± 8.3 % (moyenne ± écart-type).
De plus, le composé n° 20 a un effet dose dépendant sur l'invasion cellulaire.
En effet :
- à une concentration de 0,3 µM, le composé n° 20 inhibe l'invasion cellulaire de 26 ± 3%,
- à une concentration de 1 µM, le composé n° 20 inhibe l'invasion cellulaire de 53 ± 11%.
Ces résultats mettent en évidence une augmentation de l'inhibition de l'invasion cellulaire en fonction de la dose du composé n° 20.

Les composés de formule (I) selon la présente invention présentent donc une bonne affinité pour les héparanases et présentent un effet inhibiteur des héparanases.

Il a été démontré chez les animaux et chez l'homme que l'augmentation de la sécrétion d'héparanases et la progression cancéreuse sont corrélées (Goldschmit et al., PNAS, (2002), Vol.99(15), p.10031-10036). Par exemple, un niveau d'héparanases élevé a été détecté dans le sérum d'animaux présentant des tumeurs métastatiques (Vlodavsky et al., Isr. J. Med. Sci., (1988), Vol.24(9-10), p.464-470) ou encore dans l'urine de patients atteints de cancer, ayant développé de nombreuses métastases (Vlodavsky et al., Curr. Biol., (1997), Vol.7(1), p.43-51). Des biopsies de tumeurs ont révélé la même corrélation (Vlodavsky et al., lsr. J. Med. Sci., (1988), Vol.24(9-10), p.464-470). Il existe donc une corrélation entre l'augmentation de sécrétion d'héparanases et le potentiel métastatsique des cellules tumorales (Vlodavsky et al., Invasion Matastasis, (1994), Vol.14, p.290-302 *;* Nature Medicine, (1999), Vol.5, p. 793-802).

Les héparanases, sécrétées par les cellules tumorales, dégradent les HSPGs et HS, composants majeurs de la ECM. La ECM ainsi perforée, laisse circuler les cellules tumorales et métastasiques et permet également l'invasion de vaisseaux sanguins néoformés (angiogénèse) (Suzanne A. Eccles, Nat. Med., (1999), Vol.5(7), p.793-809). L'angiogénèse est un processus de génération de nouveaux vaisseaux capillaires à partir de vaisseaux préexistants ou par mobilisation et différentiation de cellules de la moelle osseuse. Ainsi, à la fois une prolifération incontrôlée des cellules endothéliales et une mobilisation d'angioblastes à partir de la moelle osseuse sont observées dans les processus de néo-vascularisation des tumeurs.
Ainsi, les héparanases représentent des cibles pertinentes pour les thérapies visant à inhiber les processus d'invasion de cellules cancéreuses et de métastasisation d'une part, et d'angiogénèse d'autre part. On entend par cancer (ou carcinome) toute croissance cellulaire maligne de l'épithélium, présent dans la peau mais également et surtout dans la paroi des organes ainsi que l'apparition de cellules tumorales métastasiques telles que les mélanomes, mésothéliome, lymphome, leucémie, fibrosarcome, rhabdomyosarcome, mastocytome, mais également les carcinomes touchant un tissu tels que le colon, le rectum, la prostate, les poumons, les seins, le pancréas, l'intestin, les reins, les ovaires, l'utérus, le col de l'utérus, la vessie, le foie et l'estomac. Les carcinomes infiltrent les tissus adjacents et s'étendent (métastasent) à d'autres organes distants, par exemple, foie, poumons, cerveau ou les os. Un composé possédant une activité inhibitrice des héparanases tels que les composés de l'invention peut donc être utile pour le traitement de tels cancers (Fang J et al., Proc. Natl. Acad. Sci. USA, (2000), Vol.97(8), p. 3884-3889 ; Kondraganti et al., Cancer Res., (2000), Vol.60(24), p. 6851-6855), et notamment le cancer colorectal, de la prostate, du poumon, du sein, du pancréas, de rein, de la vessie et des ovaires.

Le récepteurs p75, récepteur des molécules de la famille des neurotrophines (NT), a été identifié comme étant un marqueur représentatif du phénomène de métastasisation dans le cerveau. Il a de plus été démontré que la sécrétion de NT implique une augmentation de la sécrétion d'héparanases (Marchetti D. et al, J. Cell. Biochem., (2004), Vol.91(1), p. 206-215). Ainsi, un composé possédant une activité inhibitrice des héparanases tels que les composés de l'invention peut donc être utile pour diminuer la métastasisation au niveau du système nerveux central en inhibant l'action de l'activation du récepteur P75 (Marchetti D. et al., Pathol. Oncol. Res., (2003), Vol.9(3), p. 147-158 et *Epub,* (2003), Review ; Walch ET. et al., Int. J. Cancer, (1999), Vol.82(1), p. 112-120 ; Menter DG. et al., Invasion Metastasis, (1994-95), Vol.14(1-6), p. 372-384 ; Marchetti D. et al., Int. J. Cancer, (1993), Vol. 55(4), p. 692-699).

L'activité des héparanases sur les HSPGs de la ECM semble également être corrélée avec le déclenchement de réactions inflammatoires et auto-immunes (Vlodavsky et al., Invasion metastasis, (1994), Vol.4, p. 290-302 ; Goldschmit et al., Med. Sci., (2002), Vol.99(15), p. 10031-10036). L'interaction des plaquettes, des granulocytes, des lymphocytes B et T, des macrophages et des mastocytes avec la ECM, est associée à la dégradation des HS par les héparanases (Vlodavsky et al., Invasion Mefastasis, (1992), Vol.12, p.112-127). Ainsi, un composé tel que les composés de l'invention, possédant une activité inhibitrice des héparanases, peut donc être utile pour le traitement de maladies inflammatoires, notamment les maladies inflammatoires chroniques comme l'arthrite rhumatoïde ou les IBD (Inflammatory Bowel Disease), comprenant deux formes de maladies inflammatoires chroniques de l'intestin : les UC (ulcerative colitis) et la maladie de Crohn's (CD) ou de maladies auto-immunes.

D'autres études laissent penser que les héparanases pourrait jouer un rôle dans le traitement de maladies cardiovasculaires (Journal of Pharmacological Sciences, (2004), 94, No. Supplement 1, pp. 160P, print. ; and Meeting Info.: 77th Annual Meeting of the Japanese Pharmacological Society. Osaka, Japan. March 08-10, 2004. Japanese Pharmacological Society ; and Miller, Heather Ann, Diss. Abstr. Int., (1984), B 2003, 64(5) ; Demir et al., Clin. Appl. Thromb. Hemost., (2001), Vol.7(2), p. 131-140), telles que la resténose post angioplastie, des maladies liées aux complications vasculaires du diabète comme les rétinopathies diabétiques, l'athérosclérose (Atherosclerosis, (1999), Vol.145, p. 97-106 ; J. Clin. Invest., (1997), Vol.100, p. 867-874) ou encore les maladies thromboemboliques et les thromboses artérielles. Ainsi, un composé, inhibiteur d'héparanases, selon l'invention peut présenter une thérapie de choix dans ces pathologies.

Par ailleurs, les héparanases sont connues pour être impliquées dans certains cas d'insuffisance rénale où les fonctions de filtration et de réabsorption rénales peuvent être altérées (FASEB J, (2004), Vol.18, p. 252-263). Ainsi, un composé, inhibiteur d'héparanases, selon l'invention peut présenter une thérapie de choix dans telles pathologies.

Les HSPGs de la ECM, semblent également jouer un rôle de régulateurs majeurs de la croissance et de l'activation cellulaire, via la modulation de facteurs de croissance, en particulier des FGFs (Fibroblast Growth Factors). Par exemple, l'activité des héparanases implique la libération de facteurs de croissance comme les FGFs, qui stimulent notamment l'angiogénèse et favorise la progression tumorale (Bashkin et al., Biochemistry, (1989), Vol.28, p. 1737-1743). Ainsi, les héparanases représentent des cibles pertinentes pour le traitement de maladies dans lesquelles les FGFs sont impliqués.

De manière générale, les FGFs sont impliqués de façon importante par l'intermédiaire de sécrétions autocrines, paracrines ou juxtacrines dans les phénomènes de dérégulation de la stimulation de la croissance des cellules cancéreuses. De plus, les FGFs initient l'angiogénèse tumorale qui joue un rôle prépondérant à la fois sur la croissance de la tumeur mais aussi sur les phénomènes de métastasisation.

L'angiogénèse est un processus de génération de nouveaux vaisseaux capillaires à partir de vaisseaux préexistants ou par mobilisation et différentiation de cellules de la moelle osseuse. Ainsi, à la fois une prolifération incontrôlée des cellules endothéliales et une mobilisation d'angioblastes à partir de la moelle osseuse sont observées dans les processus de néo-vascularisation des tumeurs. Il a été montré *in vitro* et *in vivo* que plusieurs facteurs de croissance stimulent la prolifération endothéliale, et notamment le FGF-1 ou a-FGF et le FGF-2 ou b-FGF.

Les a-FGF et b-FGF jouent par exemple, un rôle important dans la croissance et le maintien des cellules de la prostate (Doll JA. et al., Prostate, (2001), Vol.305, p.49-293.

Plusieurs travaux montrent la présence de a-FGF et b-FGF et de leurs récepteurs FGFRs à la fois dans les lignées tumorales humaines du sein (notamment MCF7) et dans des biopsies de tumeurs.

Les cellules de gliome produisent *in vitro* et *in vivo* du a-FGF et du b-FGF et possèdent à leur surface différents récepteurs FGFs.

Plus récemment le rôle potentiel d'agents pro angiogéniques et notamment des FGFs dans les leucémies et lymphomes a été documenté (Thomas DA. et al., Acta Haematol, (2001), Vol.207, p.106-190).

Il existe une corrélation entre le processus d'angiogénèse de la moelle osseuse et les "extramedullar disease" dans les CML (chronic myelomonocytic leukemia).

La prolifération et la migration de cellules musculaires lisses vasculaires contribuent à l'hypertrophie intimale des artères et joue ainsi un rôle prépondérant dans l'athérosclérose et dans la resténose après angioplastie et endoarterectomie. Des études *in vivo* montrent, après lésion de la carotide par "balloon injury", une production locale de a-FGF et de b-FGF.

Les troubles vasculaires dus au diabète se caractérisent par une altération de la réactivité vasculaire et du flux sanguin, une hyperperméabilité, une réponse proliférative exacerbée et une augmentation des dépôts de protéines matricielles. De manière plus précise le a-FGF et le b-FGF sont présents dans les membranes pré rétiniennes de patients ayant des rétinopathies diabétiques, dans les membranes des capillaires sous jacents et dans l'humeur vitrée de malades souffrants de rétinopathies prolifératives.

L'arthrite rhumatoïde (RA) est une maladie chronique avec une étiologie inconnue. Alors qu'elle affecte de nombreux organes, la forme la plus sévère de RA est une inflammation synoviale des articulations progressive aboutissant à la destruction. L'angiogénèse semble affecter de manière importante la progression de cette pathologie. Ainsi le a-FGF et le b-FGF ont été détectés dans le tissu synovial et dans le fluide articulaire de patients atteints de RA, indiquant que ce facteur de croissance intervient dans l'initiation et / ou la progression de cette pathologie. Dans des modèles de AIA (adjuvant-induced model of arthritis) chez le rat, il a été montré que la sur-expression de b-FGF augmente la sévérité de la maladie alors qu'un anticorps neutralisant anti b-FGF bloque la progression de la RA (Yamashita et al., J.Immunol., (2002), Vol.57, p. 168-450 ; Manabe et al., Rheumatol, (1999), Vol.20, p. 38-714).

L'angiogénèse et l'inflammation sont également des phénomènes majeurs intervenant dans les processus impliqués dans l"ostéoarthrite conduisant à une destruction de l'articulation associée à de la douleur. L'angiogénèse peut également jouer un rôle dans l'hypertrophie chondrocytaire et l'ossification contribuant ainsi aux modifications de l'articulation (Bonnet CS et a Rheumatology. (1) 7-16 2005).

Les IBD (inflammatory bowel disease) comprennent deux formes de maladies inflammatoires chroniques de l'intestin : les UC (ulcerative colitis) et la maladie de Crohn's (CD). Les IBD sont caractérisées par une dysfonction immunitaire se traduisant par une production inappropriée de cytokines inflammatoires induisant l'établissement d'un système micro-vasculaire local. Cette angiogénèse d'origine inflammatoire a pour conséquence une ischémie intestinale induite par vasoconstriction. Des taux circulants et locaux de b-FGF importants ont été mesurés chez des patients atteints de ces pathologies (Kanazawa et al., American Journal of Gastroenterology, (2001), Vol.28, p. 96-822 ; Thorn et al., Scandinavian Journal of Gastroenterology, (2000), Vol.12, p. 35-408).

Un composé possédant une activité inhibitrice des héparanases tels que les composés de l'invention, peut donc être utile pour le traitement de maladies liées à un une up régulation des FGFs et/ou de leurs récepteurs.

Grâce à leur faible toxicité et leurs propriétés pharmacologiques et biologiques, les composés de la présente invention trouvent leur application dans le traitement de tout carcinome ayant un degré de vascularisation important (poumon, sein, prostate, oesophage) ou induisant des métastases (colon, estomac, mélanome) ou étant sensibles au a-FGF ou au b-FGF de manière autocrine ou enfin dans des pathologies de type lymphomes et leucémies. Ces composés représentent une thérapie de choix soit seul soit en association avec une chimiothérapie ou une radiothérapie adaptée ou en association avec un traitement par des anti-angiogènes. Les composés selon l'invention trouvent également leur application dans le traitement de maladies cardiovasculaires comme l'athérosclérose, la resténose post angioplastie dans le traitement des maladies liés aux complications apparaissant suite à la pose de prothèses endovasculaires et/ou de pontages aorto-coronariens ou de complications vasculaires du diabète comme les rétinopathies diabétiques. Les composés selon l'invention trouvent également leur application dans le traitement de maladies inflammatoires chroniques comme l'arthrite rhumatoïde ou les IBD.

Les produits selon l'invention trouvent également leur application dans le traitement de la dégénérescence maculaire. Un caractère majeur de la perte de la vision chez l'adulte est la néo-vascularisation et les hémorragies consécutives qui causent des désordres fonctionnels importants au niveau de l'oeil et qui se traduisent par une cécité précoce. Récemment, l'étude des mécanismes impliqués dans les phénomènes de néo-vascularisation oculaire a permis de mettre en évidence l'implication de facteur pro-angiogéniques dans ces pathologies.

Les composés de l'invention peuvent également être utilisés en combinaison d'un ou plusieurs traitements anticancéreux, comme les traitements chirurgicaux la radiothérapie ou en association avec des composés bloquant l'angiogenèse. Par exemple, les composés de l'invention peuvent être utilisés seuls ou en association avec un autre principe actif tel que cisplatine, cyclophosphamide, methotrexate, 5-fluorouracile, paclitaxel, docetaxel, vincristine, vinblastine, vinorelbine, doxorubicine, tamoxifène, torémifène, acétate de megestrol, anastrozole, goserelin, capecitabine et raloxifène ou des molécules ayant une activité anti-angiogénique, pour le traitement de cancer.

Selon un autre de ses aspects, la présente invention a donc pour objet une composition pharmaceutique contenant, en tant que principe actif, un composé de formule (I) sous forme libre ou sous forme de sels formés avec une base ou un acide pharmaceutiquement acceptables, selon l'invention, éventuellement en association avec un ou plusieurs excipients inertes et appropriés.

Lesdits excipients sont choisis selon la forme pharmaceutique et le mode d'administration souhaités : orale, sublinguale, sous-cutanée, intramusculaire, intraveineuse, transdermique, trans-muqueux, locale ou rectale.

Dans chaque unité de dosage, le principe actif est présent dans les quantités adaptées aux doses journalières envisagées afin d'obtenir l'effet prophylactique ou thérapeutique désiré. Chaque unité de dosage peut contenir de 0,1 à 100 mg de principe actif, de préférence 0,5 à 50 mg.

Les compositions pharmaceutiques de l'invention peuvent être destinées à l'administration orale, sublinguale, sous-cutanée, intramusculaire, intraveineuse, intratrachéale, topique, intranasale, transdermique, rectale, intraoculaire, vaginale. Les formes unitaires d'administration peuvent être, par exemple, des comprimés, des gélules, des granules, des poudres, des solutions ou suspensions orales ou injectables, des timbres transdermiques ("patch"), des stylos injecteurs, des suppositoires. Pour l'administration locale on peut envisager des pommades, crêmes, lotions, collyres, gels, sprays, huile.
Lesdites formes unitaires sont dosées pour permettre une administration journalière de 1 à 100 mg de principe actif par kg de poids corporel, selon la forme galénique utilisée.

Pour préparer des comprimés on ajoute au principe actif, micronisé ou non, un véhicule pharmaceutique qui peut être composé de diluants, comme par exemple le lactose, la cellulose microcristalline, l'amidon, et des adjuvants de formulation comme des liants, (polyvinylpyrrolidone, hydroxypropylméthylcellulose, ...), des agents d'écoulement comme la silice, des lubrifiants comme le stéarate de magnésium, l'acide stéarique, le tribehenate de glycerol, le stéarylfumarate de sodium, Des agents mouillants ou tensioactifs tels que le laurylsulfate de sodium peuvent aussi être ajoutés.
Les techniques de réalisation peuvent être la compression directe, la granulation sèche, la granulation humide ou la fusion à chaud.
Les comprimés peuvent être nus, dragéifiés, par exemple par du saccharose, ou enrobés avec divers polymères ou autres matières appropriées. Il peuvent être conçus pour permettre une libération rapide, retardée ou prolongée du principe actif grâce à des matrices polymères ou à des polymères spécifiques utilisés dans l'enrobage.

Pour préparer des gélules on mélange le principe actif avec des véhicules pharmaceutiques secs (simple mélange, granulation sèche ou humide, ou fusion à chaud), liquides ou semi-solides.
Les gélules peuvent être dures ou molles, pelliculées ou non, de manière à avoir une activité rapide, prolongée ou retardée (par exemple pour une forme entérique).

Une composition sous forme de sirop ou d'élixir ou pour l'administration sous forme de gouttes peut contenir le principe actif conjointement à un édulcorant, de préférence acalorique, du méthylparaben ou du propylparaben comme antiseptique, un agent de sapidité et un colorant.

Les poudres et granules dispersibles dans de l'eau peuvent contenir le principe actif en mélange avec des agents de dispersion ou des agents mouillants, ou des agents dispersants comme la polyvinylpyrrolidone, de même qu'avec des édulcorants et des agents correcteurs de goût.

Pour l'administration rectale, on recourt à des suppositoires préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles injectables contenant des agents de dispersion et/ou des mouillants pharmacologiquement compatibles, par exemple le propylèneglycol ou le butylèneglycol.

Le principe actif peut être formulé également sous forme de micro capsules, éventuellement avec un ou plusieurs supports ou additifs, ou bien avec une matrice polymère ou avec une cyclodextrine (timbres transdermiques, formes à libération prolongée).

Les compositions à administration locale selon l'invention comprennent un milieu compatible avec la peau. Elles peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de micro émulsions, d'aérosols, ou encore sous forme de dispersions vésiculaires contenant des lipides ioniques et/ou non ioniques. Ces formes galéniques sont préparées selon les méthodes usuelles des domaines considérés.

Le principe actif peut être formulé également sous forme de micro capsules, éventuellement avec un ou plusieurs supports ou additifs, ou bien avec une matrice polymère ou avec une cyclodextrine (timbres transdermiques, formes à libération prolongée).

Les compositions locales selon l'invention comprennent un milieu compatible avec la peau. Elles peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de micro émulsions, d'aérosols, ou encore sous forme de dispersions vésiculaires contenant des lipides ioniques et/ou non ioniques. Ces formes galéniques sont préparées selon les méthodes usuelles des domaines considérés.

Les exemples qui suivent, donnés à titre non limitatif, illustrent la préparation de composés selon la présente invention.

Abréviations utilisées dans le texte qui suit :
- Ac =: acétyle
- All =: allyle
- Bn =: benzyle
- Me =: méthyle
- Ph =: phényle
- PMB =: (4-méthoxy)benzyle
- PMBBr =: (3-bromo-4-méthoxy)benzyle
- Z =: benzyloxycarbonyle
- CCM =: chromatographie sur couche mince

Les exemples qui suivent illustrent la préparation de composés selon l'invention, sans la limiter. Avant d'aborder la préparation de ces différents exemples, il est décrit ci-après la préparation de composés **(PREPARATION)** utiles à l'obtention de composés de l'invention ou à d'autres préparations.
Pour les préparations et exemples qui suivent, on utilise les méthodes expérimentales suivantes :

### METHODE 1

### Oxydation des alcools primaires en acide, puis transformation en ester de benzyle

Du TEMPO^{®} (0.02 équivalent molaire) et une solution aqueuse saturée d'hydrogénocarbonate de sodium (4 L/mol) sont ajoutés à une solution de composé à oxyder (1 équivalent molaire) dans le tétrahydrofurane (THF) (3.5 L/mol). Après refroidissement à 0 °C, du Bromodan (2 équivalents molaire) est ajouté goutte à goutte pendant 20 min. Après 3h d'agitation magnétique le mélange réactionnel est concentré et le résidu est séché par évaporation répétée de diméthylformamide (DMF) (4.95 L/mol). Le composé brut ainsi obtenu est utilisé tel quel dans l'étape suivante.
Une solution du composé précédent dans le diméthylformamide (13.1 L/mol) est traitée à température ambiante (1-15h) avec du bromure de benzyle (10 équivalents molaire), et de l'hydrogénocarbonate de potassium (5 équivalents molaire). Le mélange réactionnel est concentré puis le résidu est dissous dans l'acétate d'éthyle (35 L/mol), lavé à l'eau, séché (sulfate de sodium), et concentré. Une chromatographie sur colonne donne l'ester de benzyle attendu.

### METHODE 2

### Couplage aux imidates catalysé par le triflate de tert-butyldiméthylsilyle

Une solution de triflate de tert-butyldiméthylsilyle dans du dichlorométhane (0,1M, 0,15 mole par mole d'imidate) est ajoutée, sous argon, à - 20° C, à une solution de l'imidate et de l'accepteur de glycosyle dans un mélange dichlorométhane/éther diéthylique, (1:2, 22.5-45 L/mol) en présence de tamis moléculaire 4 Å. Après 10-45 minutes (CCM), on ajoute de l'hydrogénocarbonate de sodium solide. On filtre la solution, on lave à l'eau, on sèche et on évapore à sec.

### METHODE 3

### Méthode de saponification des esters.

A une solution de composé à saponifier dans du tétrahydrofurane (160 L/mol) sont additionnés successivement, à -5 °C, de l'eau oxygénée (H₂O₂) à 30% (7.16 L/mol ester), et une solution aqueuse 0.7 N d'hydroxyde de lithium (2.3 moles par mole d'ester). Après 1 h d'agitation à -5 °C, le milieu réactionnel est placé 4h à 0 °C, puis agité à température ambiante jusqu'à consommation des esters. Le brut réactionnel est alors éventuellement purifié sur colonne LH-20.

### METHODE 4

### Sulfonatation

On ajoute du complexe triéthylamine/trioxyde de soufre (5 moles par fonction hydroxyle) à une solution dans le diméthylformamide (90 L/mol) du composé à sulfater. Après 12 à 22 heures à 55° C, on ajoute à 0 °C du méthanol ou une solution aqueuse de d'hydrogénocarbonate de sodium, et après 0.5-24h d'agitation à température ambiante, le milieu réactionnel est purifié à l'aide d'une colonne LH-20, ou de deux colonnes Sephadex^{®} G-25 (éluées successivement par une solution aqueuse de chlorure de sodium 0,2 M, puis par de l'eau). Les fractions contenant le produit sont alors concentrées sous vide poussé pour conduire au composé désiré.

### METHODE 5

### Hydrogénolyse des éthers de benzyle et/ou des esters de benzyle

On laisse agiter pendant 6-16h (CCM) une solution du composé dans un mélange acide acétique glacial / eau / *tert*-butanol sous une atmosphère d'hydrogène (3-15 bars) en présence de palladium sur charbon (équivalent à 0.7-3 fois la masse du composé) à 5 ou 10 %. Après filtration, on dépose la solution au sommet d'une colonne de Sephadex^{®} G-25 éluée par du chlorure de sodium 0,2 M. On concentre les fractions contenant le produit et on dessale en utilisant la même colonne éluée par l'eau. Le composé final est obtenu après lyophilisation.

Des préparations utiles à l'obtention des composés selon l'invention sont décrites ci-dessous.

### PREPARATION 1 :

### Synthèse du 5-(benzyloxy)-4-hydroxypipéridine-1,3-dicarboxylate de dibenzyle (3S, 4R, 5R) (n° 6)

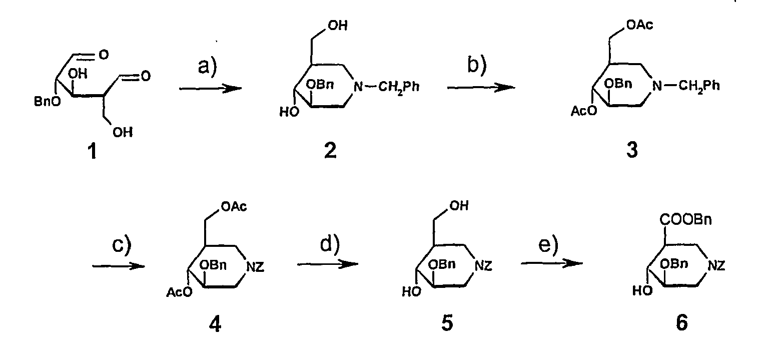

### Etape 1.a : Préparation du 1-benzyl-3-(benzyloxy)-5-(hydroxyméthyl)pipéridin-4-ol (3R, 4R, 5R) (n° 2)

La synthèse du composé 1 est décrite dans T.M. Jespersen and M. Bols, Tetrahedron (1994) 50 (47), 13449-13460 et dans le brevet US 5,844,102. La synthèse du composé 2 est décrite dans le brevet WO98/50359.
A une solution du composé 1 (10.8 g, 42.8 mmol) dans le méthanol (590 mL) sont ajoutés successivement du cyanoborohydrure de sodium (5.38 g, 2 équivalents molaires), puis de l'acide acétique (7.4 mL, 3 équivalents molaire) à -10 °C et une solution de benzylamine (5.1 mL, 1.1 equivalent) dans du méthanol (100 mL). Après retour à température ambiante, le mélange réactionnel est porté à 50 °C pendant 2h. Après retour à température ambiante, une solution d'hydrogénocarbonate de sodium 2% (85 mL) est additionnée. Le méthanol est concentré sous vide, puis le résidu est dilué par du dichlorométhane et la phase organique est lavée à l'eau, puis par une solution aqueuse de chlorure de sodium, séchée (Na₂SO₄), puis concentrée sous vide. Le résidu est engagé directement dans l'étape suivante sans purification.

### Etape 1.b : Préparation du [4-(acétyloxy)-1-benzyl-5-(benzyloxy)pipéridin-3-yl] acétate de méthyle (3R, 4R, 5R) (n° 3)

A une solution du composé brut **2** (10.8 g) obtenu à l'étape 1.a dans du dichlorométhane (345 mL), sont ajoutés successivement, à 0 °C, sous argon, de la triéthylamine (13.5 mL, 2.25 équivalents molaire), de la 4-(diméthylamino)pyridine (DMAP) (7.84 g, 1.5 équivalent), et de l'anhydride acétique (61 mL, 15 équivalents molaire). La température est maintenue à 0 °C pendant 10 min, puis le milieu réactionnel est placé à température ambiante pendant 16h. Le mélange réactionnel est alors concentré sous vide, et le résidu purifié sur gel de silice pour fournir le composé **3** (7.65g, 43%, 2 étapes).
RMN ¹H (CDCl₃) δ 7.36-7.18 (m, 10H, Ar), 4.60-4.42 (dd, 2H, OCH₂Ph), 2.02, 1.99 (2s, 6H, 2CH₃CO).

### Etape 1.c : Préparation du 4-(acétyloxy)-3-(acétyloxyméthyl)-5-(benzyloxy)pipéridine-1-carboxylate de benzyle (3R, 4R, 5R) (n° 4)

A une solution du composé **3** (2.31 g, 5.6 mmol) obtenu à l'étape 1.b dans le tétrahydrofurane (28 mL), est ajouté, sous argon, à -10 °C, du chlorure de benzyloxycarbonyle (2.4 mL, 3 équivalents molaire), puis le milieu réactionnel est laissé sous agitation à température ambiante pendant 18h. Le mélange réactionnel est alors concentré sous vide et le résidu est purifié sur gel de silice (1:9 éther diéthylique - éther diisopropylique) pour conduire au composé **4** (2.14 g, 84%).
Spectre de masse (ESI) *m*/*z* 478.3 [(M + Na)⁺].

### Etape 1.d : Préparation du 3-(benzyloxy)-4-hydroxy-5-(hydroxyméthyl)pipéridine-1-carboxylate de benzyle (3R, 4R, 5R) (n° 5)

A une solution du composé **4** (1.9 g, 4.2 mmol) obtenu à l'étape 1.c, dans le dioxane (25 mL), est ajouté, à 0 °C, une solution de 0.84 M d'hydroxyde de lithium monohydrate (25 mL, 5 équivalents molaire). Le milieu réactionnel est maintenu à 0 °C pendant 5 minutes, puis est placé à température ambiante, pendant 30 min. Après neutralisation par de l'acide chlorhydrique (HCl : 3 N), le milieu réactionnel est dilué dans du dichlorométhane, lavé à l'eau, séché (Na₂SO₄), filtré et concentré. Le résidu est purifié sur gel de silice (3:1 acétate d'éthyle - cyclohexane), pour conduire au composé **5** (1.59 g, 90%).
Spectre de masse (ESI) m/z 394.4 [(M + Na)⁺].

### Etape 1.e : Préparation du 5-(benzyloxy)-4-hydroxypipéridine-1,3-dicarboxylate de dibenzyle (3S, 4R, 5R) (n° 6)

Le composé **5** (3.18 g, 8.6 mmol) obtenu à l'étape 1.d, est traité selon la METHODE 1 pour conduire au composé **6** (2.92 g, 71%).
Spectre de masse (ESI) *m*/*z* 476.5 [(M + Na)⁺].

### PREPARATION 2 :

### Synthèse du (Benzyl 2,4-di-O-acétyl-3-O-benzyl-α-L-idopyranosyluronate)-(1-4)-(6-O-acétyl-2-azido-3-O-benzyl-2-désoxy-α,β-D-glucopyranose trichloroacétimidate) (n° 11)

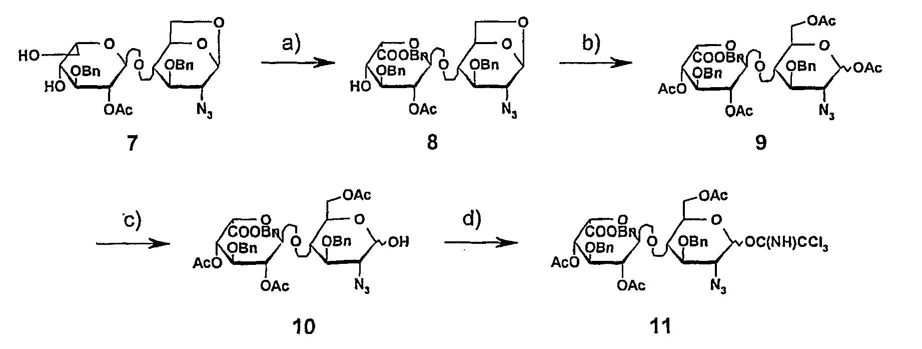

### Etape 2.a : Préparation du (Benzyl 2-O-acétyl-3-O-benzyl-α-L-idopyranosyluronate)-(1-4)-(1.6-anhydro-2-azido-3-O-benzyl-2-désoxy-β-D-glucopyranose) (n° 8)

Le composé **7** sous forme 2'-O-acétylée (18.0 g, 31.48 mmol), préparé de la même manière que le composé 2'-O-benzoylé décrit dans Y. Ichikawa et al., Tetrahedron Lett. (1986) 27 (5) 611-614*,* est traité selon la METHODE 1 pour conduire, après purification sur gel de silice (3:7 acétate d'éthyle - cyclohexane), au composé **8** (16.4 g, 77%).
Spectre de masse (ESI) *m*/*z* 698.3 [(M + Na)⁺].

### Etape 2.b : Préparation du (Benzyl 2,4-di-O-acétyl-3-O-benzyl-α-L-idopyranosyluronate)-(1-4)-(1,6-di-O-acétyl-2-azido-3-O-benzyl-2-désoxy-α,β-D-glucopyranose) (n° 9)

A une solution du composé **8** (3.74 g, 5.54 mmol) obtenu à l'étape 2.a, dans de l'anhydride acétique (52 mL, 100 équivalents molaire), est additionné, à 0 °C, de l'acide trifluoroacétique (TFA) (4.7 mL, 11 équivalents molaire). Après retour à température ambiante, le mélange réactionnel est agité pendant 16h, puis est concentré, coévaporé au toluène, et purifié sur gel de silice (4:1 toluène-acétate d'éthyle), pour donner le composé **9** (4.33 g, 95%).
Spectre de masse (ESI) *m*/*z* 842.2 [(M + Na)⁺].

### Etape 2.c : Préparation du (Benzyl 2,4-di-O-acétyl-3-O-benzyl-α-L-idopyranosyluronate)-(1-4)-(6-O-acétyl-2-azido-3-O-benzyl-2-désoxy-α,β-D-glucopyranose) (n° 10)

A une solution du composé **9** (4.3 g, 5.24 mmol) obtenu à l'étape 2.b, dans de l'éther diéthylique (210 mL), est additionnée, à 0 °C, de la benzylamine (BnNH₂) (22 mL, 38 équivalents molaire). Après 4.5h d'agitation à température ambiante, le milieu est acidifié avec de l'HCl 1N, puis est extrait à l'acétate d'éthyle, séché (Na₂SO₄), concentré, et purifié sur gel de silice (35:65 acétate d'éthyle - cyclohexane) pour conduire à **10** (3.4 g, 83%).
Spectre de masse (ESI) *m*/*z* 800.2 [(M + Na)⁺].

### Etape 2.d : Préparation du (Benzyl 2,4-di-O-acétyl-3-O-benzyl-α-L-idopyranosyluronate)-(1-4)-(6-O-acétyl-2-azido-3-O-benzyl-2-désoxy-α,β-D-glucopyranose trichloroacétimidate) (n° 11)

A une solution du composé **10** (3.38 g, 4.35 mmol) obtenu à l'étape 2.c, dans le dichlorométhane (82 mL), sont additionnés, sous argon, du carbonate de césium (Cs₂CO₃) (2.26 g, 1.6 équivalent molaire), puis du trichloroacétonitrile (CCl₃CN) (1.74 mL, 5.0 équivalents molaire). Après 1.5h d'agitation, le mélange réactionnel est filtré, puis concentré. Le résidu est purifié sur gel de silice (3:7 acétate d'éthylecyclohexane) pour donner 11 (2.96 g, 74%).
RMN ¹H (CDCl₃) δ 6.43 (d, H-1α Glc^{I}), 5.64 (d, H-1β Glc^{I}), 5.17 (d, IdoUA^{II}).

### PREPARATION 3 :

### Synthèse du (3-O-benzyl-2,4-di-O-sodium sulfonato-α-L-idopyranosyluronate de sodium)-(1-4)-(2-acétamido-3-O-benzyl-2-désoxy-6-O-sodium sulfonato-α-D-glucopyranosyl)-(1-4)-(3-O-benzyl-2-O-sodium sulfonato-α-L-idopyranosyluronate de sodium)-(1-4)-(2-acétamido-3-O-benzyl-2-désoxy-6-O-sodium sulfonato-α-D-glucopyranosyl)-(1-4)-(5-(benzyloxy)-4-oxypipéridine-1-carboxylate de benzyle-3-carboxylate de sodium (3S, 4R, 5R)) (n° 19)

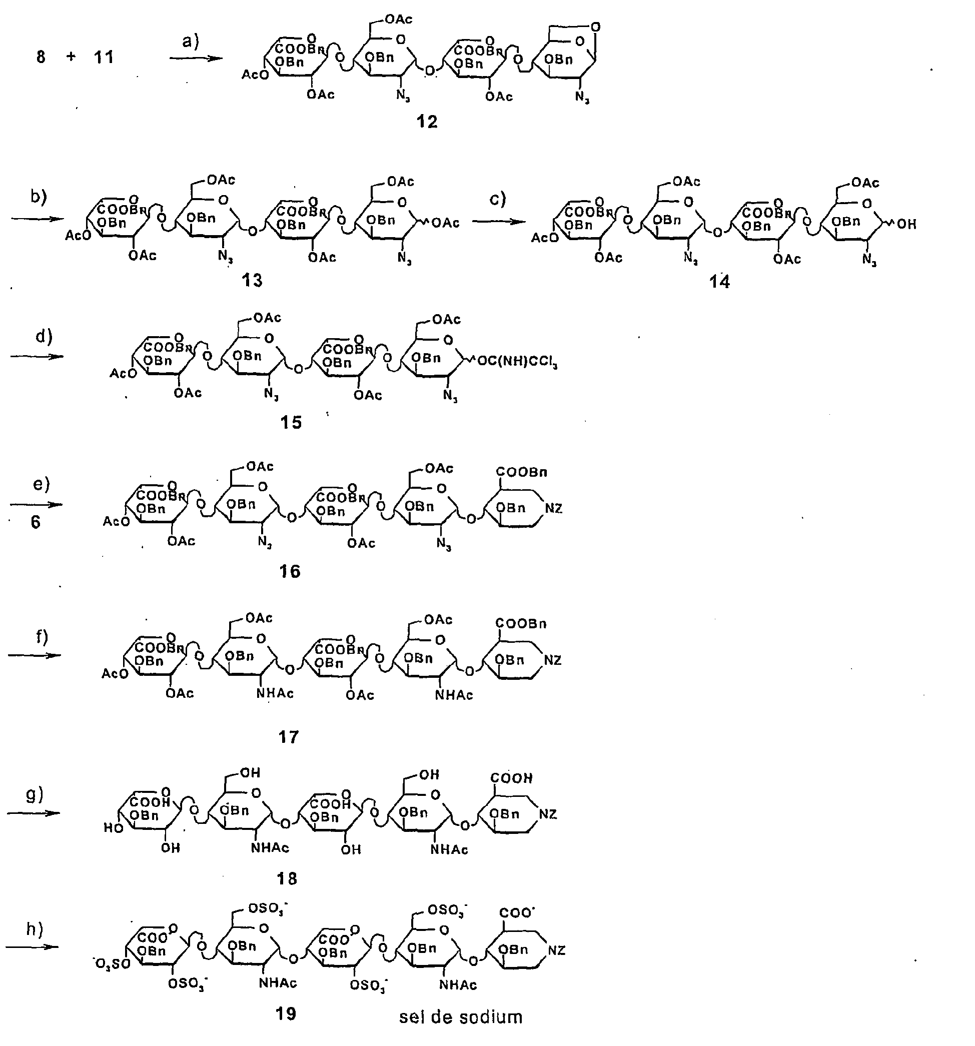

### Etape 3.a : Préparation du (benzyl 2,4-di-O-acétyl-3-O-benzyl-α-L-idopyranosyluronate)-(1-4)-(6-O-acétyl-2-azido-3-O-benzyl-2-désoxy-α-O-glucopyranosyl)-(1-4)-(benzyl 2-O-acétyl-3-O-benzyl-α-L-idopyranosyluronate)-(1-4)-(1,6-anhydro-2-azido-3-O-benzyl-2-désoxy-(3-D-glucopyranose) (n° 12)

Le composé **11** (455 mg, 0.50 mmol) obtenu à l'étape 2.d et le composé **8** (675 mg, 1 mmol) obtenu à l'étape 2.a sont traités selon la méthode 2 pour fournir, après purification, le composé **12** (385 mg, 54%).
Spectre de masse (ESI) *m*/*z* 1457.6 [(M + Na)⁺].

### Etape 3.b : Préparation du (benzyl 2,4-di-O-acétvl-3-0-benzvl-a.-L-idopyranosyluronate)-(1-4)-(6-O-acétyl-2-azido-3-O-benzyl-2-désoxy-α-D-glucogyranosyl)-(1-4)-(benzyl 2-O-acétyl-3-O-benzyl-α-L-idopyranosyluronate)-(1-4)-(1,6-di-O-acétyl-2-azido-3-O-benzyl-2-désoxv-(α,β-D-glucopyranose) (n° 13)

Le composé **12** (365 mg, 0.254 mmol) obtenu à l'étape 3.a, est traité comme pour la synthèse du composé **9** (étape 2.b) pour conduire, après purification sur gel de silice (1:1 Et₂O - éther diisopropylique), à **13** (376 mg, 97%).
Spectre de masse (ESI) m/z 1560.7 [(M + Na)⁺].

### Etape 3.c : Préparation du (benzyl 2,4-di-O-acétyl-3-O-benzyl-α-L-idopyranosyluronate)-(1-4)-(6-O-acétyl-2-azido-3-O-benzyl-2-désoxy-α-D-glucopyranosyl)-(1-4)-(benzyl 2-O-acétyl-3-O-benzyl-α-L-idopyranosyluronate)-(1-4)-(6-O-acétyl-2-azido-3-O-benzyl-2-désoxy-α,β-D-glucopyranose) (n° 14)

Le composé **13** (364 mg, 0.237 mmol) obtenu à l'étape 3.b, est traité comme pour la synthèse du composé **10** (étape 2.c) pour donner, après purification sur gel de silice (7:3 Et₂O-éther diisopropylique), le composé **14** (310 mg, 87%).
Spectre de masse (ESI) *m*/*z* 1518.8 [(M + Na)⁺].

### Etape 3.d : Préparation du (benzyl 2,4-di-O-acétyl-3-O-benzyl-α-L-idopyranosyluronate)-(1-4)-(6-O-acétyl-2-azido-3-O-benzyl-2-désoxy-α-D-glucopyranosyl)-(1-4)-(benzyl 2-O-acétyl-3-O-benzyl-α-L-idopyranosyluronate)-(1-4)-(6-O-acétyl)-2-azido-3-O-benzyl-2-désoxy-α,β-D-glucopyranose trichloroacétimidate) (n° 15)

Le composé **14** (279 mg, 0.187 mmol) obtenu à l'étape 3.c, est traité comme pour la synthèse du composé **11** (étape 2.d), pour fournir, après purification sur gel de silice (1:1 Et₂O - éther diisopropylique), **15** (230 mg, 75%).
Spectre de masse (ESI) *m*/*z* 1660.6 [(M + Na)⁺].

### Etape 3.e : Préparation du (benzyl 2,4-di-O-acétyl-3-O-benzyl-α-L-idopyranosyluronate)-(1-4)-(6-O-acétyl-2-azido-3-O-benzyl-2-désoxy-α-D-glucopyranosyl)-(1-4)-(benzyl 2-O-acétyl-3-O-benzyl-α-L-idopyranosyluronate)-(1-4)-(6-O-acétyl-2-azido-3-O-benzyl-2-désoxy-α-D-glucopyranosyl)-(1-4)-(5-(benzyloxy)-4-oxypipéridine-1,3-dicarboxylate de dibenzyle (3S, 4R, 5R)) (n° 16)

Les composés **15** (217 mg, 0.132 mmol) obtenu à l'étape 3.d, et 6 (126 mg, 0.264 mmol) obtenu à l'étape 1.e, sont traités selon la METHODE 2 pour fournir, après purification, le composé **16** (168 mg, 66%).
Spectre de masse (ESI) *m*/*z* 1976.0 [(M + Na)⁺].

### Etape 3.f : Préparation du (benzyl 2,4-di-O-acétvl-3-O-benzyl-α-L-idopyranosyluronate)-(1-4)-(6-O-acétyl-2-acétamido-3-O-benzyl-2-désoxy-α-D-glucopyranosyl)-(1-4)-(benzyl 2-O-acétyl-3-O-benzyl-α-L-idopyranosyluronate)-(1-4)-(6-O-acétyl-2-acétamido-3-O-benzyl-2-désoxy-α-D-glucopyranosyl)-(1-4)-(5-(benzyloxy)-4-oxypipéridine-1,3-dicarboxylate de dibenzyle (3S, 4R, 5R)) (n° 17)

Le composé **16** (138.5 mg, 70.9 µmol) obtenu à l'étape 3.e, est dissous dans la pyridine (1.16 mL), puis de l'acide thioacétique (1.14 mL, 225 équivalents molaire) est ajouté à 0 °C. Le milieu réactionnel est agité 14h à température ambiante, puis est concentré et purifié sur gel de silice (3:2 cyclohexane-acétate d'éthyle), pour conduire au composé **17** (118 mg, 84%).
Spectre de masse (ESI) *m*/*z* 2007.7 [(M + Na)⁺].

### Etape 3.g : Préparation de l'acide 3-O-benzyl-α-L-idopyranosyluronique)-(1-4)-(2-acétamido-3-O-benzyl-2-désoxy-α-D-glucopyranosyl)-(1-4)-(acide 3-O-benzyl-α-L-idopyranosyluronique)-(1-4)-(2-acétamido-3-O-benzyl-2-désoxy-α-D-glucopyranosyl)-(1-4)-(5-(benzyloxy)-4-oxypipéridine-3-acide carboxylique-1-carboxylate de benzyle (3S, 4R, 5R)) (n° 18)

Le composé **17** (101 mg, 50.9 µmol) obtenu à l'étape 3.f, est traité selon la METHODE 3, puis le milieu réactionnel est acidifié avec de l'acide chlorhydrique 6N (pH 1), et extrait au dichlorométhane. La phase organique est lavée au sulfite de sodium (Na₂SO₃) 5%, puis à l'eau. Après séchage, filtration et concentration, le résidu est engagé brut dans l'étape suivante.
Spectre de masse (ESI) *m*/*z* 1505.6 [(M + H)⁺].

### Etape 3.h : Préparation du (3-O-benzyl-2,4-di-O-sodium sulfonato-α-L-idopyranosyluronate de sodium)-(1-4)-(2-acétamido-3-O-benzyl-2-désoxy-6-O-sodium sulfonato-α-D-glucopyranosyl)-(1-4)-(3-O-benzyl-2-O-sodium sulfonato-α-L-idopyranosyluronate de sodium)-(1-4)-(2-acétamido-3-O-benzyl-2-désoxy-6-O-sodium sulfonato-α-D-glucopyranosyl)-(1-4)-(5-(benzyloxy)-4-oxypipéridine-1-carboxylate de benzyle-3-carboxylate de sodium (3S, 4R, 5R)) (n° 19)

Le composé 18 brut obtenu à l'étape 3.g, est traité d'après la METHODE 4, pour fournir le composé 19 (50 mg, 54% (2 étapes)).
Spectre de masse (ESI) *m*/*z* 2014 [(M - 3Na + 3H)⁻].

### PREPARATION 4 :

### Synthèse du (3-O-benzyl-2,4-di-O-sodium sulfonato-α-L-idopyranosyluronate de sodium)-(1-4)-(3-O-benzyl-2-désoxy-2-N-sodium sulfonato-6-O-sodium sutfonato-α-D-glucopyranosyl)-(1-4)-(3-O-benzyl-2-O-sodium sulfonato-α-L-idopyranosyluronate de sodium)-(1-4)-(3-O-benzyl-2-désoxy-2-N-sodium sulfonato-6-O-sodium sulfonato-α-D-glucopyranosyl)-(1-4)-(5-(benzyloxy)-4-oxypipéridinè-1-carboxylate de benzyle-3-carboxylate de sodium (3S, 4R, 5R)) (n° 26)

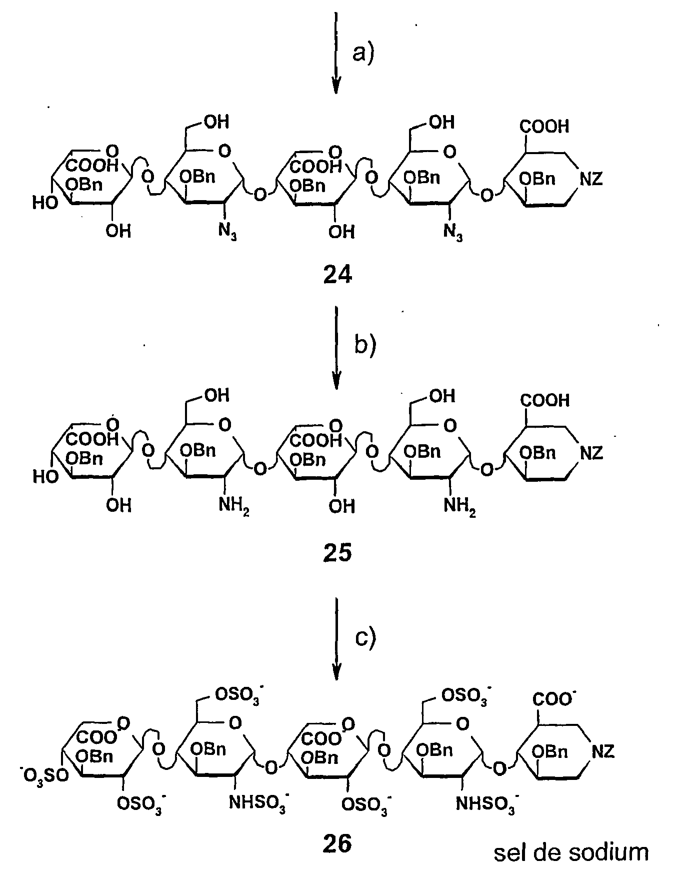

### Etape 4.a : Préparation du (acide 3-O-benzyl-α-L-idopyranosyluronique)-(1-4)-(2-azido-3-O-benzyl-2-désoxy-α-D-glucopyranosyl)-(1-4)-(acide3-O-benzy)-α-L-idopyranosyluronique)-(1-4)-(2-azido-3-O-benzyl-2-désoxy-α-D-glucopyranosyl)-(1-4)-(5-(benzyloxy)-4-oxypipéridine-1-carboxylate de benzyle-3-carboxylate de sodium (3S, 4R, 5R)) (n° 24)

Le composé **16** (175 mg, 89.6 µmol) obtenu à l'étape 3.e, est traité selon la METHODE 3, puis le milieu réactionnel est acidifié avec de l'acide chlorhydrique 6N (pH 2). Le mélange est alors purifié sur colonne Sephadex^{®} LH-20 (1:1 dichlorométhane - éthanol), pour conduire au composé **24** (100 mg, 76%).
Spectre de masse (ESI) *m*/*z* 1474.1 [(M + H)⁺].

### Etape 4.b : Préparation du (acide 3-O-benzyl-α-L-idopyranosyluronique)-(1-4)-(2-amino-3-O-benzyl-2-désoxy-α-D-glucopyranosyl)-(1-4)-(acide 3-O-benzyl-α-L-idopyranosyluronique)-(1-4)-(2-amino-3-O-benzyl-2-désoxy-α-D-glucopyranosyl)-(1-4)-(5-(benzyloxy)-4-oxypipéridine-1-carboxylate de benzyle-3-acide carboxylique (3S, 4R, 5R)) (n° 25)

A une solution du composé **24** (35 mg) obtenu à l'étape 4.a, dans un mélange 1:1 méthanol - tétrahydrofurane (1 mL), est ajouté un complexe 10% Pd/C/éthylènediamine préparé selon la méthode décrite dans H. Sajiki et al., J. Org. Chem. (1998), Vol.63, p. 7990-7992) (107 mg). Le milieu est alors placé sous pression d'H₂ (3 bars) à température ambiante pendant 16h. Après filtration et concentration, le brut réactionnel est remis en réaction dans les mêmes conditions, puis est purifié sur gel de silice (acétate d'éthyle - pyridine - acide acétique - eau, 6:2:0.6:1) pour conduire au composé 25 (12 mg, 44%).
Spectre de masse (ESI) *m*/*z* 1421.4 [(M + H)⁺].

### Etape 4.c : Préparation du (3-O-benzyl-2,4-di-O-sodium sulfonato-α-L-idopyranosyluronate de sodium)-(1-4)-(3-O-benzyl-2-désoxy-2-N-sodium sulfonato-6-O-sodium sulfonato-α-D-glucopyranosyl)-(1-4)-(3-O-benzyl-2-O-sodium sulfonato-α-L-idopyranosyluronate de sodium)-(1-4)-(3-O-benzyl-2-désoxy-2-N-sodium sulfonato-6-O-sodium sulfonato-α-D-glucopyranosyl)-(1-4)-(5-(benzyloxy)-4-oxypipéridine-1-carboxylate de benzyle-3-carboxylate de sodium (3S, 4R, 5R)) (n° 26)

Le composé **25** (8.5 mg, 5.98 µmol) obtenu à l'étape 4.b, est traité d'après la méthode 4, pour fournir le composé **26** (7 mg, 56%).
Spectre de masse (ESI) *mlz* 2133.8 [(M - H)⁻].

### PREPARATION 5 :

### Synthèse du (Benzyl 2-O-acétyl-4-O-lévulinoyl-3-O-méthyl-α-L-idopyranosyluronate)-(1-4)-(6-O-acétyl-2-O-(3-bromo-4-méthoxy)benzyl-3-O-méthyl-α,β-D-glucopyranose trichloroacétimidate) (n°40)

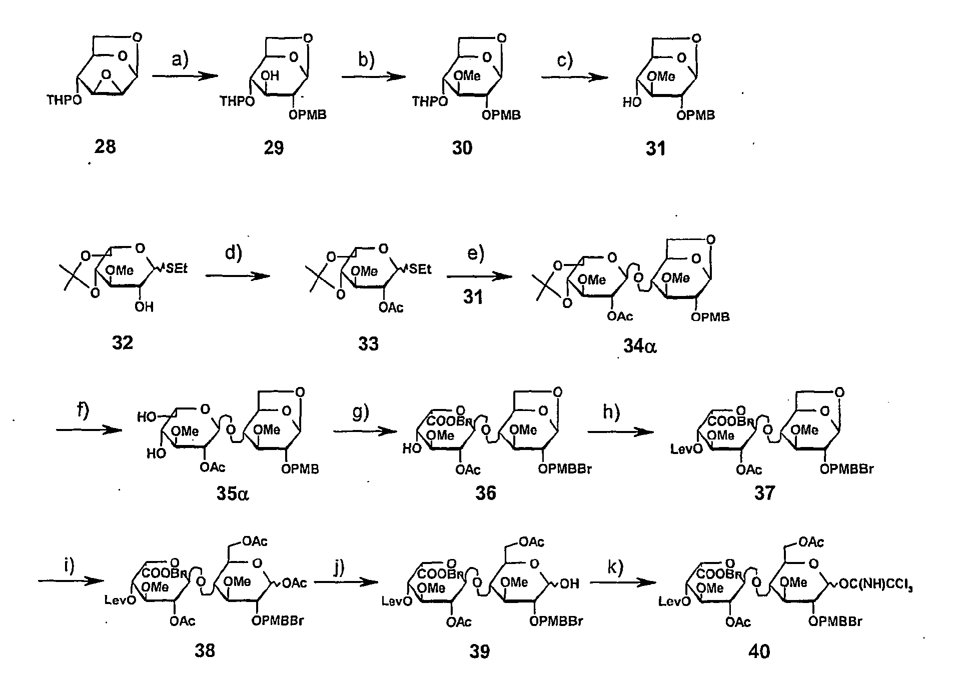

### Etape 5.a : Préparation du 1,6-anhydro-4-O-(tétrahydropyran-2-yl)-2-O-(4-méthoxy)benzol-O-D-glucopyranose (n° 29)

A une solution du composé **28** (41 g, 180 mmol) (préparé selon H. Paulsen and W. Stenzel, Chem. Ber. (1978) 111, 2348-57) dans l'alcool para-méthoxybenzylique (25 mL, 1.1 équivalent molaire) est ajouté, à 40 °C, du sodium (1.37 g, 0.33 équivalent molaire). Le mélange réactionnel est ensuite porté à 110 °C pendant 20 min, puis du méthanol (20 mL) est additionné à 0 °C, et l'agitation est maintenue 16h à température ambiante. Après concentration, une purification sur gel de silice (3:7 acétate d'éthyle-éther diisopropylique) conduit à 29 (20.1 g, 31%).
Spectre de masse (ESI) *m*/*z* 384.2 [(M + Na)⁺].

### Etape 5.b : Préparation du 1,6-anhydro-4-O-(tétrahydropyran-2-yl)-2-O-(4-méthoxy)benzyl-3-O-méthyl-β-D-glucopyranose (n° 30)

A une solution du composé 29 (13.1 g, 35.8 mmol) obtenu à l'étape 5.a, dans le diméthylformamide (107 mL) sont successivement ajoutés, à 0 °C et sous atmosphère d'argon, de l'iodure de méthyle (2.67 mL) et de l'hydrure de sodium (2.68 g). Après retour à température ambiante, le mélange réactionnel est agité 16h, puis du méthanol est ajouté à 0 °C, le milieu est extrait à l'acétate d'éthyle, séché (Na₂SO₄), filtré, et concentré, pour donner **30** qui est directement engagé dans l'étape suivante.
Spectre de masse (ESI) *m*/*z* 403.3 [(M + Na)⁺].

### Etape 5.c : Préparation du 1,6-anhydro-2-O-(4-méthoxy)benzyl-3-O-méthyl-β-D-glucopyranose (n° 31)

A une solution dans le méthanol (143 mL) du composé brut 30 obtenu à l'étape 5.b, est ajoutée une solution méthanolique 0.25 M d'acide camphorsulfonique (CSA) (1 équivalent molaire). Après 30 min d'agitation magnétique, le milieu est dilué au dichlorométhane, puis lavé à l'eau, avec une solution aqueuse à 2% d'hydrogénocarbonate de sodium, à l'eau, séché (Na₂SO₄), filtré, et concentré. Une purification sur gel de silice (3:7 acétate d'éthyle - cyclohexane) conduit au composé **31** (9.0 g, 85%).
Spectre de masse (ESI) *m*/*z* 319.1 [(M + Na)⁺].

### Etape 5.d : Préparation de l'éthyl 2-O-acétyl-4,6-O-isopropylidène-3-O-méthyl-1-thio-α,β-L-idopyranoside (n° 33)

A une solution du composé brut **32** (1.99 g, 7.1 mmol) (préparé selon P. Duchaussoy et al., Carbohydr. Res. (1999), 317, 63-84) dans le dichlorométhane (37 mL) sont ajoutés successivement, à 0 °C, sous argon, de la triéthylamine (2.2 mL), du DMAP (173 mg), et de l'anhydride acétique (1.34 mL). La température est maintenue à 0 °C pendant 10 min, puis le milieu réactionnel est placé à température ambiante pendant 16h. Le mélange réactionnel est alors concentré sous vide, et le résidu purifié sur silice (1:1 Et₂O-cyclohexane) pour fournir le composé **33** (2.1 g, 92%).
Spectre de masse (ESI) *m*/*z* 343.3 [(M + Na)⁺].

### Etape 5.e : Préparation du (2-O-acétyl-4 6-O-isopropylidène-3-O-méthyl-α-L-idopyranosyl)-(1-4)-(1,6-anhydro-2-O-(4-méthoxy)benzyl-3-O-méthyl-β-D-glucopyranose) (n° 34α) et du (2-O-acétyl-4,6-O-isopropylidène-3-O-méthyl-β-L-idopyranosyl)-(1-4)-(1,6-anhydro-2-O-(4-méthoxy)benzyl-3-O-méthyl-β-D-glucopyranose) (n° 34β)

A un mélange sous argon du composé 33 (1.86 g, 5.79 mmol) obtenu à l'étape 5.d, et du composé **31** (1.46 g, 4.94 mmol) obtenu à l'étape 5.c, en présence de tamis moléculaire 4 Å (2.89 g) dans du toluène (50 mL) est ajoutée, à -20 °C, une solution de N-iodosuccinimide (1.38 g), et d'acide trifluorométhanesulfonique (63.5 µL) dans un mélange 1:1 de dichlorométhane - dioxane (16.5 mL). Après 45 min d'agitation, de l'hydrogénocarbonate de sodium solide est ajouté au milieu réactionnel, et après filtration, le mélange est dilué au dichlorométhane, lavé avec une solution aqueuse de thiosulfate de sodium (Na₂S₂O₃) à 10%, et une solution aqueuse de chlorure de sodium saturée. Après séchage et concentration, le résidu est directement engagé dans l'étape suivante.
Spectre de masse (ESI) *m*/*z* 577.4 [(M + Na)⁺].

### Etape 5.f : Préparation du (2-O-acétyl-3-O-méthyl-α-L-idopyranosyl)-(1-4)-(1,6-anhydro-2-O-(4-méthoxy)benzyl-3-O-méthyl-β-D-glucopyranose) (n° 35α), et (2-O-acétyl-3-O-méthvl-β-L-idopyranosyl)-(1-4)-(1,6-anhydro-2-O-(4-méthoxy)benzyl-3-O-méthyl-β-D-glucopyranose) (n° 35β)

A une solution du composé mélange **34α** et **34β** obtenu à l'étape 5.e, dans du dichloro-1,2-éthane (12 mL), est ajouté de l'acide acétique à 70% (55 mL). Le mélange est chauffé à 60 °C pendant 50 min, puis concentré sous vide, et le résidu obtenu est purifié sur gel de silice (3:2 toluène - acétone) pour conduire au composé **35**α (1.56 g, 61%, deux étapes), et au composé **35**β (0.36 g, 14%, deux étapes).
Spectre de masse (ESI) *m*/*z* 537.5 [(M + Na)⁺].

### Etape 5.g : Préparation du (Benzyl 2-O-acétyl-3-O-méthyl-α-L-idopyranosyluronate)-(1-4)-(1,6-anhydro-2-O-(3-bromo-4-méthoxy)benzyl-3-O-méthyl-β-D-glucopyranose) (n° 36)

Le composé **35α** (0.975 g, 1.89 mmol) obtenu à l'étape 5f, est traité selon la METHODE 1 pour donner, après purification sur gel de silice (1:1 acétate d'éthylecyclohexane), le composé **36** (1.09 g, 83%).
RMN ¹H (CDCl₃) δ 7.54-6.86 (m, 8H, Ar).

### Etape 5.h : Préparation du (Benzyl 2-O-acétyl-4-O-lévulinoyl-3-O-méthyl-α-L-idopyranosyluronate)-(1-4)-(16-anhydro-2-O-(3-bromo-4-méthoxy)benzyl-3-O-méthyl-α-D-glucopyranose) (n° 37)

A une solution sous argon du composé **36** (1.09 g, 1.56 mmol) obtenu à l'étape 5.g, dans le dioxane (35 mL) sont ajoutés successivement du DMAP (43 mg, 0.2 équivalent molaire), de l'hydrochlorure de 1-(3-diméthylaminopropyl)-3-éthylcarbodiimide (EDCI) (0.675 g, 2 équivalents molaire.), et de l'acide lévulinique (0.361 mL, 2 équivalents molaire.), et après 16h d'agitation, le milieu réactionnel est lavé successivement par une solution d'hydrogénosulfate de potassium (KHSO₄) 10%, de l'eau, d'hydrogénocarbonate de sodium à 2%, puis la solution est séchée (Na₂SO₄), filtrée et concentrée pour donner un résidu qui est purifié sur gel de silice (acétate d'éthyle - dichlorométhane 2:3) conduisant au composé 37 (1.07 g, 86%).
Spectre de masse (ESI) *m*/*z* 819.4 [(M + Na)⁺].

### Etape 5.i : Préparation du (Benzyl 2-O-acétyl-4-O-lévulinoyl-3-O-méthyl-α-L-idopyranosyluronate)-(1-4)-(1,6-di-O-acétyl-2-O-(3-bromo-4-méthoxy)benzyl-3-O-méthyl-α,β-D-glucopyranose) (n° 38)

Le composé **37** (1.07 g, 1.34 mmol) obtenu à l'étape 5.h, est traité comme pour la synthèse du composé **9** (étape 2.b), pour donner, après purification sur gel de silice (acétate d'éthyle - dichlorométhane 2:3), le composé **38** (1.04 g, 87%).
Spectre de masse (ESI) *m*/*z* 921.4 [(M + Na)⁺].

### Etape 5.j : Préparation du (Benzyl 2-O-acétyl-4-O-lévulinoyl-3-O-méthyl-α-L-idopyranosyluronate)-(1-4)-(6-O-acétyl-2-O-(3-bromo-4-méthoxy)benzyl-3-O-méthyl-α,β-D-glucopyranose) (n° 39)

Le composé **38** (1.04 g, 1.16 mmol) obtenu à l'étape 5.i, est traité comme pour la synthèse du composé **10** (étape 2.c), pour fournir, après purification sur gel de silice (acétate d'éthyle-dichlorométhane 1:1), le composé **39** (740 mg, 74%).
Spectre de masse (ESI) *m*/*z* 877.3 [(M + Na)⁺].

### Etape 5.k : Préparation du (Benzyl 2-O-acétyl-4-O-lévulinoyl-3-O-méthyl-α-L-idopyranosyluronate)-(1-4)-(6-O-acétyl-2-O-(3-bromo-4-méthoxy)benzyl-3-O-méthyl-α,β-D-glucopyranose trichloroacétimidate) (n° 40)_{.}

Le composé 39 (740 mg, 860 µmol) obtenu à l'étape 5.j, est traité comme pour la synthèse du composé 11 pour donner, après purification sur gel de silice (acétate d'éthyle - dichlorométhane 3:7), le composé 40 (714 mg, 83%).
RMN ¹H (CDCl₃) δ 6.39 (d, H-1α Glc^{I}), 5.76 (d, H-1β Glc^{I}).

### PREPARATION 6 :

### Synthèse du (3-O-méthyl-2,4-di-O-sodium sulfonato-α-L-idopyranosyluronate de sodium)-(1-4)-(3-O-méthyl-2,6-di-O-sodium sulfonato-α-D-glucopyranosyl)-(1-4)-(3-O-méthyl-2-O-sodium sulfonato-α-L-idopyranosyluronate de sodium)-(1-4)-(3-O-méthyl-2,6-di-O-sodium sulfonato-α-D-glucopyranosyl)-(1-4)-(5-(benzyloxy)-4-oxypipéridine-1-carboxylate de benzyle-3-carboxylate de sodium (3S, 4R, 5R)) (composé n° 46)

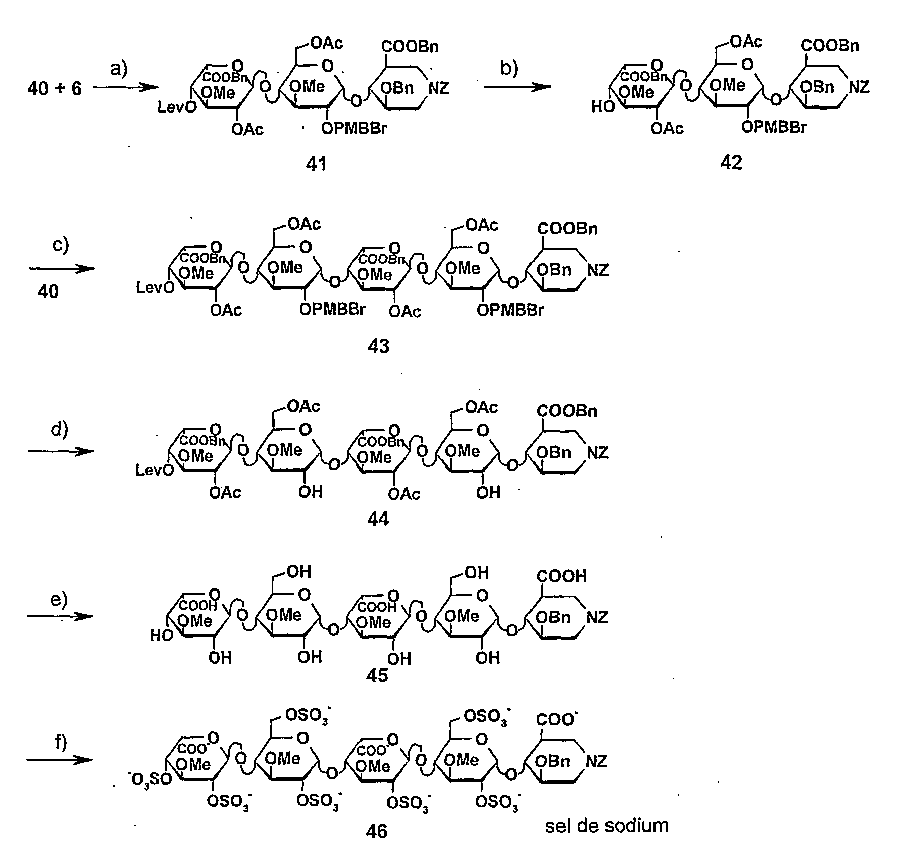

### Etape 6.a : Préparation du (benzyl 2-O-acétyl-4-O-lévulinoyl-3-O-méthyl-α-L-idopyranosyluronate)-(1-4)-(6-O-acétyl-2-O-(3-bromo-4-méthoxy)benzyl-3-O-méthyl-α-D-glucopyranosyl)-(1-4)-(5-(benzyloxy)-4-oxypipéridine-1,3-dicarboxylate de dibenzyle (3S, 4R, 5R)) (n° 41)

Un mélange du composé **40** (94 mg, 0.094 mmol, 1.0 équivalent molaire) obtenu à l'étape 5.k, et du composé **6** (111 mg, 0.234 mmol, 2.5 équivalent molaire) obtenu à l'étape 1.e, est traité selon la METHODE 2 pour conduire, après purification sur colonne Sephadex^{®} LH-20, puis sur gel de silice (dichlorométhane - acétate d'éthyle 9:1), au composé 41 (62 mg, 50%).
Spectre de masse (ESI) *m*/*z* 1336.5 [(M + Na)⁺].

### Etape 6.b : Préparation du (benzyl 2-O-acétyl-3-O-méthyl-α-L-idopyranosyluronate)-(1-4)-(6-O-acétyl-2-O-(3-bromo-4-méthoxy)benzyl-3-O-méthyl-α-D-glucopyranosyl)-(1-4)-(5-(benzyloxy)-4-oxypipéridine-1,3-dicarboxylate de dibenzyle (3S, 4R, 5R)) (n° 42)

A une solution du composé **41** (60 mg, 45.7 µmol) obtenu à l'étape 6.a, dans un mélange1:2 toluène/éthanol (9 mL) est ajouté de l'acétate d'hydrazine (21 mg, 10 équivalents molaire). Après 3h d'agitation magnétique, le mélange est concentré sous vide et le résidu est dilué au dichlorométhane, lavé avec une solution d'hydrogénocarbonate de sodium 2%, à l'eau, et la phase organique est séchée (Na₂SO₄), filtrée, puis concentrée. Le résidu est purifié sur gel de silice (acétate d'éthyle - cyclohexane 3:2) pour donner le composé **42** (48 mg, 88%).
Spectre de masse (ESI) *m*/*z* 1238.5 [(M + Na)⁺].

### Etape 6.c : Préparation du (benzyl 2-O-acétyl-4-O-lévulinoyl-3-O-méthyl-α-L-idopyranosyluronate)-(1-4)-(6-O-acétyl-2-O-(3-bromo-4-méthoxy)benzyl-3-O-méthyl-α-D-glucopyranosyl)-(1-4)-(benzyl 2-O-acétyl-3-O-méthyl-α-L-idopyranosyluronate)-(1-4)-(6-O-acétyl-2-O-(3-bromo-4-méthoxy)benzyl-3-O-méthyl-α-D-glucopyranosyl)-(1-4)-(5-(benzyloxy)-oxypipéridine-1,3-dicarboxylate de dibenzyle (3S, 4R, 5R)) (n° 43)

Un mélange du composé **40** (197.7 mg, 197.7 µmol, 1.14 équivalent molaire) obtenu à l'étape 5.k, et du composé **42** (210.5 mg, 173.2 µmol, 1.0 molequiv.) obtenu à l'étape 6.b, est traité selon la méthode 2 pour conduire, après purification sur colonne Sephadex^{®} LH-20, puis sur gel de silice (toluène - acétone 1:1), au composé **43** (176 mg, 50%).
Spectre de masse (ESI) *m*/*z* 2075.0 [(M + Na)⁺].

### Etape 6.d : Préparation du (benzyl 2-O-acétyl-4-O-lévulinoyl-3-O-méthyl-α-L-idopyranosyluronate)-(1-4)-(6-O-acétyl-3-O-méthyl-α-D-glucopyranosyl)-(1-4)-(benzyl 2-O-acétyl-3-O-méthyl-α-L-idopyranosyluronate)-(1-4)-(6,O-acétyl,3-O-méthyl-α-D-glucopyrânosyl)-(1-4)-(5-(benzyloxy)-4-oxypipéridine-1,3-dicarboxylate de dibenzyle (3S, 4R, 5R)) (n° 44)

A une solution du composé **43** (69 mg, 33.6 µmol) obtenu à l'étape 6.c, dans l'acétonitrile (3 mL), est ajouté, à 0 °C et sous atmosphère d'argon, de tétrachlorure zirconium (ZrCl₄) (39 mg, 5 équivalents molaire). Après 45min d'agitation à température ambiante, le mélange est concentré sous vide, et le résidu est dilué par de l'acétate d'éthyle, lavé à l'eau, et après séchage, filtration et concentration, le résidu est purifié sur gel de silice (3:7 acétone - toluène) pour donner le composé **44** (52 mg, 89%).
Spectre de masse (ESI) *m*/*z* 1676.7 [(M + Na)⁺].

### Etape 6.e : Préparation du (acide 3-O-méthyl-α-L-idopyranosyluronigue)-(1-4)-(3-O-méthyl-α-D-glucopyranosyl)-(1-4)-(acide 3-O-méthyl-α-L-idopyranosyluronique)-(1-4)-(3-O-méthyl-α-D-glucopyranosyl)-(1-4)-(5-(benzyloxy)-4-oxypipéridine-1-carboxylate de benzyle-3-acide carboxylique (3S; 4R, 5R)) (n° 45)

Le composé **44** (18 mg, 11 µmol) obtenu à l'étape 6.d, est traité selon la METHODE 3 pour conduire, après purification, au composé **45** (5.8 mg, 47%) pouvant être partiellement estérifié sur les groupements acide carboxylique.
Spectre de masse (ESI) *m*/*z* 1118.4 [(M + H)⁺].

### Etape 6.f : Préparation du (3-O-méthyl-2,4-di-O-sodium sulfonato-α-L-idopyranosyluronate de sodium)-(1-4)-(3-O-méthyl-2,6-di-O-sodium sulfonato-α-D-glucopyranosyl)-(1-4)-(3-O-méthyl-2-O-sodium sulfonato-α-L-idopyranosyluronate de sodium)-(1-4)-(3-O-méthyl-2,6-di-O-sodium sulfonato-α-D-glucopyranosyl)-(1-4)-(5-(benzyloxy)-4-oxypipéridine-1-carboxylate de benzyle-3-carboxylate de sodium (3S, 4R, 5R)) (n° 46)

Le composé **45** (7 mg, 6.26 µmol) obtenu à l'étape 6.e, est traité selon la METHODE 4 pour donner, après purification, le composé **46** (10 mg, 77%) pouvant être partiellement estérifié sur les groupements acide carboxylique.
Spectre de masse (ESI) *m*/*z* 1897.0 [(M + Na - H)⁺].

### PREPARATION 7 :

### Synthèse du (Benzyl 2-O-acétyl-4-O-lévulinoyl-3-O-benzyl-α-L-idopyranosyluronate)-(1-4)-(6-O-acétyl-2-O-(4-méthoxy)benzyl-3-O-benzyl-α,β-D-glucopyranose trichloroacétimidate) (n° 57)

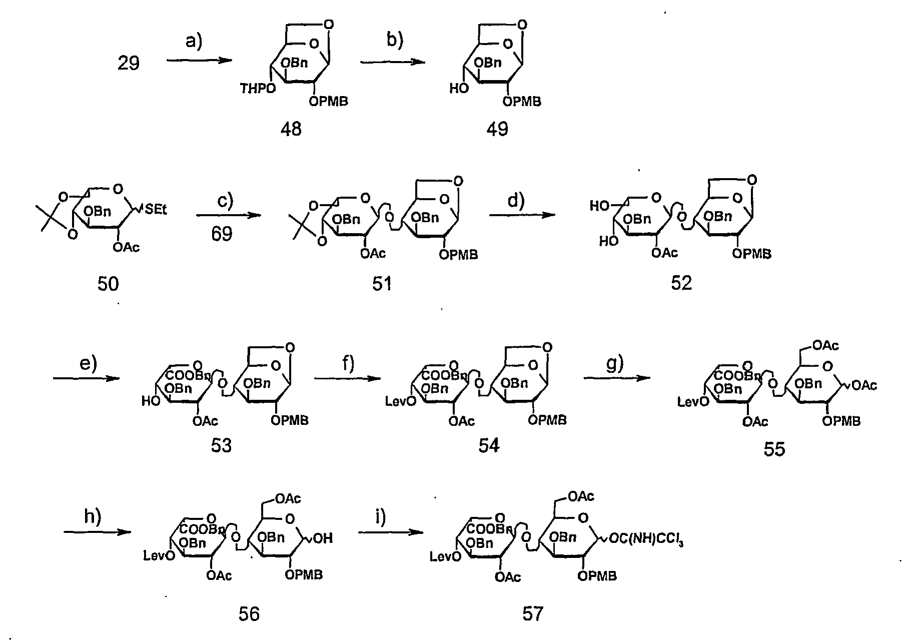

### Etape 7.a : Préparation du 1,6-anhydro-4-O-(tétrahydropyran-2-yl)-2-O-(4-méthoxy)benzyl-3-O-benzyl-β-D-glucopyranose (n° 48)

A une solution de **29** (19.96 g, 54.5 mmol) obtenu à l'étape 5.a, dans le DMF (300 mL) est ajouté, à 0 °C, du bromure de benzyle (5.1 mL), puis de l'hydrure de sodium (4.6 g). A la fin de l'addition, le mélange est placé à température ambiante pendant 16h, puis du méthanol (18 mL) est additionné à 0 °C, et après 1h d'agitation à température ambiante, le milieu est dilué à l'acétate d'éthyle (600 mL), lavé à l'eau (300 mL), séché (Na₂SO₄), filtré, et concentré. Le résidu est purifié par chromatographie flash (5:95 acétate d'éthyle - éther diisopropylique), pour fournir **48** (20.6 g, 83%).
Spectre de masse (ESI) *m*/*z* 479.3 [(M + Na)⁺].

### Etape 7.b : Préparation du 1,6-anhydro-2-O-(4-méthoxy)benzyl-3-O-benzyl-β-D-glucopyranose (n° 49)

Le composé **48** (20.6 g, 45.2 mmol) obtenu à l'étape 7.a, est traité comme pour la synthèse du composé **31** (étape 5.c), pour conduire, après purification sur silice (3:7 acétate d'éthyle - cyclohexane), à **49** (14.4 g, 86%).
Spectre de masse (ESI) *m*/*z* 395.4 [(M + Na)⁺].

### Etape 7.c : Préparation du (2-O-acétyl-4,6-O-isopropylidène-3-O-benzyl-α,β-L-idopyranosyl)-(1-4)-(1,6-anhydro-2-O-(4-méthoxy)benzyl-3-O-benzyl-β-D-glucopyranose) (n° 51)

Le composé 50 (préparé selon la méthode décrite par C. Tabeur et al. pour le dérivé 2-O-benzoylé, Carbohydr.Res. (1996), 281, 253-276) (16.91 g, 42.6 mmol) et le composé 49 (14.44 g, 38.8 mmol) obtenu à l'étape 7.b, sont mis à réagir comme pour la synthèse de 34 (étape 5.e), pour conduire, après purification sur silice (15:85 acétate d'éthyle - éther diisopropylique) au composé 51 (17.05g, 62% (56% alpha-L)).
Spectre de masse (ESI) *m*/*z* 729.3 [(M + Na)⁺].

### Etape 7.d : Préparation du (2-O-acétyl-3-O-benzyl-α-L-idopyranosyl)-(1-4)-(1,6-anhydro-2-O-(4-méthoxy)benzyl-3-O-benzyl-β-D-glucopyranose) (n° 52)

Le composé 51 (12.38, 17.51 mmol) obtenu à l'étape 5.c, est traité comme pour la synthèse de 35 (étape 5.f) pour fournir, après purification sur silice (4:1 toluene-acetone), 52 (10.85 g, 93%).
Spectre de masse (ESI) *m*/*z* 689.3 [(M + Na)⁺].

### Etape 7.e : Préparation du (Benzyl 2-O-acétyl-3-O-benzyl-α-L-idopyranosyluronate)-(1-4)-(1,6-anhydro-2-O-(4-méthoxy)benzyl-3-O-benzyl-α-D-glucopyranose) (n° 53)

Le composé 52 (10.85 g, 16.27 mmol) obtenu à l'étape 7.d, est traité selon la METHODE 1 pour conduire, après purification sur silice (3:7 acétone - cyclohexane), à 53 (8.44 g, 61 %).
Spectre de masse (ESI) *m*/*z* 793.3 [(M + Na)⁺].

### Etape 7.f : Préparation du (Benzyl 2-O-acétyl-3-O-benzyl-4-O-lévulinoyl-α-L-idopyranosyluronate)-(1-4)-(1,6-anhydro-2-O-(4-méthoxy)benzyl-3-O-benzyl-β-D-glucopyranose) (n° 54)

Le composé **53** (3.2 g, 3.77 mmol) obtenu à l'étape 7.e, est traité comme pour la synthèse de **37** (étape 5.h), pour fournir **54** (3.17 g, 89%) après purification sur silice (acétone-toluène 1:4).
Spectre de masse (ESI) *m*/*z* 891.3 [(M + Na)⁺].

### Etape 7.a : Préparation du (Benzyl 2-O-acéyl-4-O-lévulinoyl-3-O-benzyl-α-L-idopyranosyluronate)-(1-4)-(1,6-di-O-acétyl-2-O-(4-méthoxy)benzyl-3-O-benzyl-α,β-D-glucopyranose) (n° 55)

Le composé **54** (1.45 g, 1.53 mmol) obtenu à l'étape 7.f, est traité comme pour la synthèse de **9** mais à 0 °C pendant 1 h pour conduire, après purification sur silice (toluène - acétone 85:15), à 55 (1.25 g, 78%).
Spectre de masse (ESI) *m*/*z* 993.3 [(M + Na)⁺].

### Etape 7.h : Synthèse du (Benzyl 2-O-acétyl-4-O-lévulinoyl-3-O-benzyl-α-L-idopyranosyluronate)-(1-4)-(6-O-acétyl-2-O-(4-méthoxy)benzyl-3-O-benzyl-α,β-D-glucopyranose) (n° 56)

Le composé **55** obtenu à l'étape 7.g, est traité comme pour la synthèse de 10 (étape 2.c), pour conduire, après purification sur silice (éther diéthylique - dichlorométhane 25:75), à **56** (429 mg, 56%).
Spectre de masse (ESI) *m*/*z* 951.3 [(M + Na)⁺].

### Etape 7.i : Préparation du (Benzyl 2-O-acétyl-4-O-lévulinoyl-3-O-benzyl-α-L-idopyranosyluronate)-(1-4)-(6-O-acétyl-2-O-(4-méthoxy)benzyl-3-O-benzyl-α,β-D-glucopyranose trichloroacétimidate) (n° 57)

Le composé **56** (429 mg, 426 µmol) obtenu à l'étape 7.h, est traité comme pour la synthèse de **11** (étape 2.d) pour fournir, après purification sur silice (acétate d'éthyle - cyclohexane 1:1), le dérivé 57 (396 mg, 80%).
RMN ¹H (CDCl₃) δ 6.45 (d, H-1α), 5.89 (d, H-1β).

### PREPARATION 8 :

### Préparation du (Benzyl 2-O-acétyl-4-O-allyl-3-O-benzyl-α-L-idopyranosyluronate)-(1-4)-(6-O-acétyl-2-O-(4-méthoxy)benzyl-3-O-benzyl-α,β-D-glucopyranose trichloroacétimidate) (n° 62)

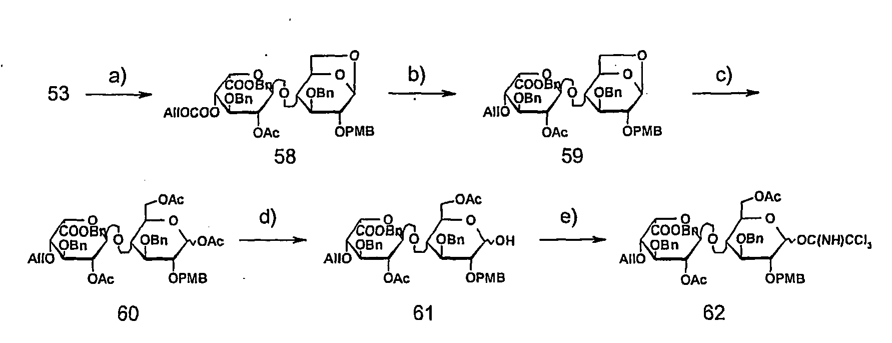

### Etape 8.a : Préparation du (Benzyl 2-O-acétyl-4-O-allyloxycarbonyl-3-O-benzyl-α-L-idopyranosyluronate)-(1-4)-(1,6-anhydro-2-O-(4-méthoxy)benzyl-3-O-benzyl-β-D-glucopyranose) (n° 58)

A une solution de **53** (0.800 g, 0.9414 mmol) obtenu à l'étape 7.e, dans le THF (9.4 mL) sont ajoutés, à 0 °C et sous atmosphère d'argon, de la pyridine (759 µL, 9.41 mmol), du DMAP (115 mg, 0.94 mmol), et du chloroformate d'allyle (995 µL, 9.41 mmol) en solution dans du THF (2.35 mL). L'agitation est maintenue pendant la nuit, puis le mélange réactionnel est dilué à l'acétate d'éthyle, lavé au KHSO₄ à 10%, à l'hydrogénocarbonate de sodium 2%, à l'eau, séché (Na₂SO₄), filtré, et concentré. Le résidu est purifié par chromatographie flash (1:9 acétone - toluène), pour fournir **58** (0.809 g, 92%).
Spectre de masse (ESI) *m*/*z* 877.3 [(M + Na)⁺].

### Etape 8.b : Préparation du (Benzyl 2-O-acétyl-4-O-allyl-3-O-benzyl-α-L-idopyranosyluronate)-(1-4)-(1,6-anhydro-2-O-(4-méthoxy)benzyl-3-O-benzyl-β-D-glucopyranose) (n° 59)

A une solution dans du THF (6 mL) du composé 58 (0.805 g, 0.862 mmol) obtenu à l'étape 8.a, sont additionnés successivement du palladium diacétate (3.9 mg, 0.017 mmol) et de la triphénylphosphine (22.6 mg, 0.086 mmol), sous atmosphère d'argon. La température du mélange est portée à 90 °C pendant 15 min, puis le milieu est concentré sous vide et purifié sur silice (15:85 acétone - toluène) pour conduire à **59** (0.587 g, 66%).
Spectre de masse (ESI) *m*/*z* 833.4 [(M + Na)⁺].

### Etape 8.c : Préparation du (Benzyl 2-O-acétyl-4-O-allyl-3-O-benzyl-α-L-idopyranosyluronate)-(1-4)-(1,6-di-O-acétyl-2-O-(4-méthoxy)benzyl-3-O-benzyl-α,β-D-glucopyranose) (n° 60)

Le composé **59** (0.587 g, 0.660 mmol) obtenu à l'étape 8.b, est traité comme pour la synthèse du composé **55** (étape 7.g), pour fournir, après purification sur silice (9:1 acétone - toluène), **60** (0.37 g, 57%)
Spectre de masse (ESI) *m*/*z* 936.4 [(M + Na)⁺].

### Etape 8.d : Préparation du (Benzyl 2-O-acétyl-4-O-allyl-3-O-benzyl-α-L-idopyranosyluronate)-(1-4)-(6-O-acétyl-2-O-(4-méthoxy)benzyl-3-O-benzyl-α,β-D-glucopyranose) (n° 61)

Le composé **60** obtenu à l'étape 8.c, est traité comme pour la synthèse de 10 (étape 2.c), pour conduire, après purification sur silice (cyclohexane-acétate d'éthyle 2:3), à **61** (240 mg, 70%).
Spectre de masse (ESI) *m*/*z* 893.4 [(M + Na)⁺].

### Etape 8.e : Préparation du (Benzyl 2-O-acétyl-4-O-allyl-3-O-benzyl-α-L-idopyranosyluronate)-(1-4)-(6-O-acétyl-2-O-(4-méthoxy)benzyl-3-O-benzyl-(α,β-D-glucopyranose trichloroacétimidate) (n° 62)

Le composé **61** (234 mg, 246 µmol) obtenu à l'étape 8.d, est traité comme pour la synthèse de 11 (étape 2.d), pour fournir, après purification sur silice (86:14 acétone - toluène), le dérivé **62** (249 mg, 93%).
RMN ¹H (CDCl₃) δ 6.40 (d, H-1α), 5.81 (d, H-1β).

Les synthèses des **PREPARATIONS 9** et **10** peuvent être schématisées comme suit :

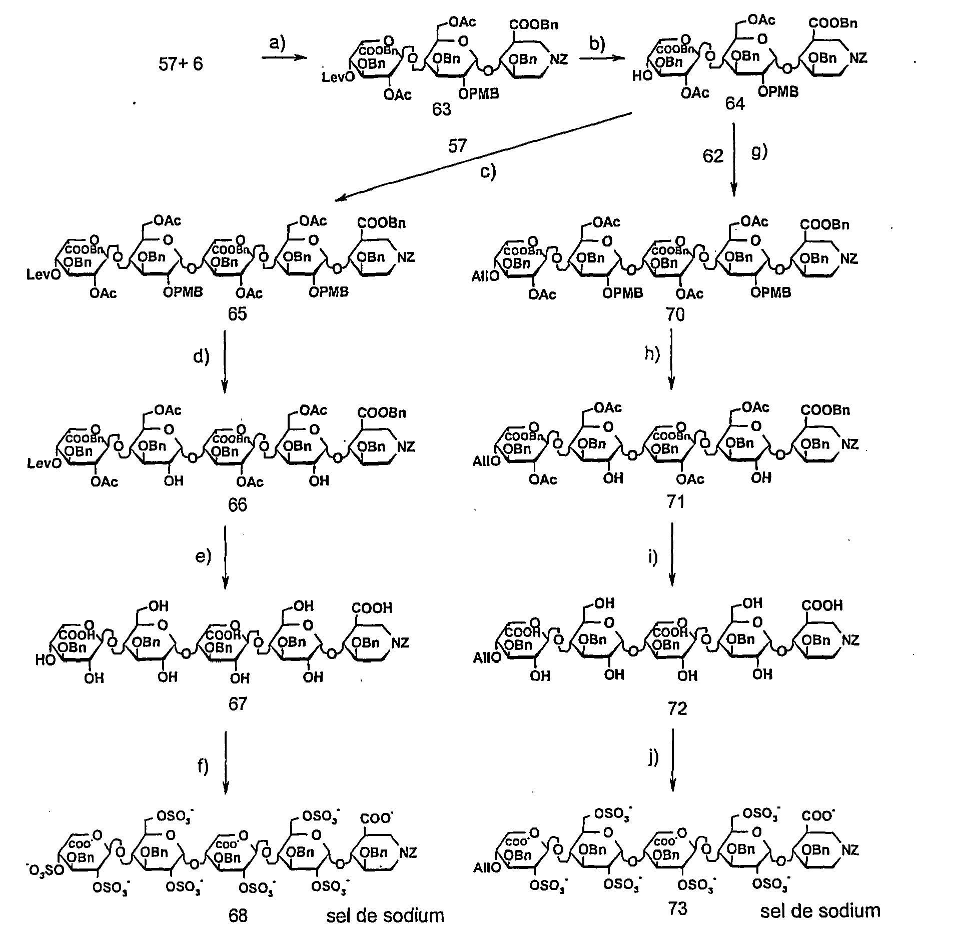

### PREPARATION 9 :

### Synthèse du (3-O-benzyl-2,4-di-O-sodium sulfonato-α-L-idopyranosyluronate de sodium)-(1-4)-(3-O-benzyl-2,6-di-O-sodium sulfonato-α-D-glucopyranosyl)-(1-4)-(3-O-benzyl-2-O-sodium sulfonato-α-L-idopyranosyluronate de sodium)-(1-4)-(3-O-benzyl-2,6-di-O-sodium sulfonato-α-D-glucopyranosyl)-(1-4)-(5-(benzyloxy)-4-oxypipéridine-1-carboxylate de benzyle-3-carboxylate de sodium (3S, 4R, 5R)) (n° 68)

### Etape 9 au Préparation du (benzyl 2-O-acétyl-4-O-lévulinoyl-3-O-benzyl-α-L-idopyranosyluronate)-(1-4)-(6-O-acétvl-2-O-(-4-méthoxy)benzyl-3-O-benzyl-α-D-glucopyranosyl)-(1-4)-(5-(benzyloxy)-4-oxypipéridine-13-dicarboxylate de dibenzyle (3S, 4R, 5R)) (n° 63)

Le composé **57** (396 mg, 0.34 mmol) obtenu à l'étape 7.i, et le composé 6 (405 mg, 0.85 mmol) obtenu à l'étape 1.e, sont traités selon la METHODE 2 pour conduire, après purification, à 63 (369 mg, 73%).
RMN ¹H (CDCl₃) δ 5.29 (d, H-1 Glc^{II}), 5.13 (d, H-1 IdoUA^{III}).

### Etape 9.b : Préparation du (benzyl 2-O-acétyl-3-O-benzyl-α-L-idopyranosyluronate)-(1-4)-(6-O-acétyl-2-O-(4-méthoxy)benzyl-3-O-benzyl-α-D-glucopyranosyl)-(1-4)-(5-(benzyloxy)-4-oxypipéridine-1,3-dicarboxylate de dibenzyle (3S, 4R, 5R)) (n° 64)

Le composé **63** (372 mg, 0.254 mmol) obtenu à l'étape 9.a, est traité comme pour la synthèse de 42 (étape 6.b), pour fournir, après purification sur silice (acétate d'éthyle - cyclohexane 2:3), le composé **64** (301 mg, 87%).
RMN ¹H (CDCl₃) δ 5.29 (d, H-1 Glc^{II}), 5.10 (d, H-1 IdoUA^{III}), 3.97 (dd, H-4 IdoUA^{III}).

### Etape 9.c : Préparation du (benzyl 2-O-acétyl-4-O-lévulinoyl-3-O-benzyl-α-L-idopyranosyluronate)-(1-4)-2-O-acétyl-2-O-(4-méthoxy)benzyl-3-O-benzyl-α-D-glucopyranosy)-(1-4)-(benzyl 2-O-acétyl-3-O-benzyl-α-L-idopyranosyluronate)-(1-4)-(6-O-acétyl-2-O-(4-méthoxy)benzyl-3-O-benzyl-α-D-glucopyranosyl)-(1-4)-(5-(benzyloxy)-4-oxypipéridine-1,3-dicarboxylate de dibenzyle (3S, 4R, 5R)) (n° 65)

Les composés **57** (124 mg, 0.108 mmol) obtenu à l'étape 7.i, et **64** (150 mg, 0.110 mmol) obtenu à l'étape 9.b, sont traités selon la METHODE 2 pour conduire, après purification, à **65** (90 mg, 35%).
RMN ¹H (CDCl₃) δ 5.28 (d, H-1 Glc^{II}), 5.16 (d, H-1 IdoUA^{III}), 5.13 (d, H-1 IdoUA^{V}), 4.74 (d, H-1 Glc^{IV}).

### Etape 9.d : Préparation du (benzyl 2-O-acétyl-4-O-lévulinoyl-3-O-benzyl-α-L-idopyranosyluronate)-(1-4)-(6-O-acétyl-3-O-benzyl-α-D-glucopyranosyl)-(1-4)-(benzyl 2-O-acétyl-3-O-benzyl-α-L-idopyranosyluronate)-(1-4)-(6-O-acétyl-3-O-benzyl-α-D-glucopyranosyl)-(1-4)-4-(benzyloxy)-4-oxypipéridine-1,3-dicarboxylate de dibenzyle (3S, 4R, 5R)) (n° 66)

Le composé **65** (45 mg, 0.19 mmol) obtenu à l'étape 9.c, est traité comme pour la synthèse de **44** (étape 6.d), pour fournir, après purification sur silice (acétate d'éthyle - cyclohexane 3:2), le composé 66 (34 mg, 91%).
Spectre de masse (ESI) *m*/*z* 1982.0 [(M + Na)⁺].

### Etape 9.e : Préparation de l'(acide 3-O-benzyl-α-L-idopyranosyluronique)-(1-4)-(3-O-benzyl-α-D-glucopyranosyl)-(1-4)-(acide 3-O-benzyl-α-L-idopyranosyluronique)-(1-4)-(3-O-benzyl-α-D-glucopyranosyl)-(1-4)-(5-(benzyloxy)-4-oxypipéridine-1-carboxylate de benzyle-3-acide carboxylique (3S, 4R, 5R)) (n° 67)

Le composé **66** (25 mg, 12.8 µmol) obtenu à l'étape 9.d, est traité .selon la METHODE 3. Le mélange réactionnel est acidifié par de l'acide chlorhydrique 6N (pH 2), puis déposé sur colonne de LH-20 (100 mL) équilibrée dans un mélange 9:1 DMF/eau. Les fractions contenant le produit sont ensuite concentrées et purifiées sur silice (dichlorométhane - méthanol 7:3) pour fournir **67** (10.4 mg, 59%) pouvant être partiellement estérifié sur les groupements acides carboxylique.
Spectre de masse (ESI) *m*/*z* 1422.7 [(M + H)⁺].

### Etape 9.f : Préparation du (3-O-benzyl-2,4-di-O-sodium sulfonato-α-L-idopyranosyluronate de sodium)-(1-4)-(3-O-benzyl-2,6-di-O-sodium sulfonato-α-D-glucopyranosyl)-(1-4)-(3-O-benzyl-2-O-sodium sulfonato-α-L-idopyranosyluronate de sodium)-(1-4)-(3-O-benzyl-2,6-di-O-sodium sulfonato-α-D-glucopyranosyl)-(1-4)-(5-(benzyloxy)-4-oxypipéridine-1-carboxylate de benzyle-3-carboxylate de sodium (3S, 4R, 5R)) (n° 68)

Le composé **67** (10.4 mg, 9 µmol) obtenu à l'étape 9.e, est traité selon la METHODE 4 pour fournir **68** qui est engagé directement dans l'étape suivante.

### PREPARATION 10 :

### Synthèse du (4-O-allyl-3-O-benzyl-2-O-sodium sulfonato-α-L-idopyranosyluronate de sodium)-(1-4)-(3-O-benzyl-2,6-di-O-sodium sulfonato-α-D-glucopyranosyl)-(1-4)-(3-O-benzyl-2-O-sodium sulfonato-α-L-idopyranosyluronate de sodium)-(1-4)-(3-O-benzyl-2,6-di-O-sodium sulfonato-α-D-glucopyranosyl)-(1-4)-(5-(benzyloxy)-4-oxypipéridine-1-carboxylate de benzyle-3-carboxylate de sodium (3S, 4R, 5R)) (n°73)

### Etape 10.g : Préparation du (benzyl 2-O-acétyl-4-O-allyl-3-O-benzyl-α-L-idopyranosyluronate)-(1 -4)-(6-O-acétyl-2-O-(4-méthoxy)benzyl-3-O-benzyl-α-D-glucopyranosyl)-(1-4)-(benzyl 2-O-acétyl-3,O-benzyl-α-L-idopyranosyluronate)-(1-4)-(6-O-acétyl-2-O-(4-méthoxybenzyl-3-O-benzyl-α-D-glucopyranosyl)-(1-4)-(5-(benzyloxy)-4-oxypipéridine-1,3-dicarboxylate de dibenzyle (3S, 4R, 5R)) (n°70)

Le composé 62 (244 mg, 0.223 mmol) obtenu à l'étape 8.e et le composé **64** (138 mg, 0.101 mmol) obtenu à l'étape 9.b, sont traités selon la METHODE 2 pour conduire, après purification, à **70** (100 mg, 43%).
RMN ¹H (CDCl₃) δ 5.28 (d, H-1 Glc^{II}), 5.25 (d, H-1 IdoUA^{V}), 5.16 (d, H-1 IdoUA^{III}), 4.72 (d, H-1 Glc^{IV}).

### Etape 10.h : Préparation du (benzyl 2-O-acétyl-4-O-allyl-3-O-benzyl-α-L-idopyranosyluronate)-(1-4)-(6-O-acétyl-3-O-benzyl-α-D-glucopyranosyl)-(1-4)-(benzyl 2-O-acétyl-3-O-benzyl-α-L-idopyranosyluronate)-(1-4)-(6-O-acétyl-3-O-benzyl-α-D-glucopyranosyl)-(1-4)-(5-(benzyloxy)-4-oxypipéridine-1,3-dicarboxylate de dibenzyle (3S, 4R, 5R)) (n° 71)

Le composé **70** (92 mg, 40.0 µmol) obtenu à l'étape 10.g, est traité comme pour la synthèse du composé **44** (étape 6.d), pour fournir, après purification sur silice (acétone - toluène 17:83), le composé **71** (44 mg, 59%).
Spectre de masse (ESI) *m*/*z* 1924.0 [(M + Na)⁺].

### Etape 10.i : Préparation du (acide 4-O-allyl-3-O-benzyl-α-L-idopyranosyluronique)-(1-4)-(3-O-benzyl-α-D-glucopyranosyl)-(1-4)-(acide 3-O-benzyl-α-L-idopyranosyluronique)-(1-4)-(3-O-benzyl-α-D-glucopyranosyl)-(1-4)-(5-(benzyloxy)-4-oxypipéridine-1-carboxylate de benzyle-3-acide carboxylique (3S, 4R, 5R)) (n°72)

Le composé **71** (41 mg, 21.6 µmol) obtenu à l'étape 10.h, est traité selon la méthode 3. Le mélange réactionnel est déposé sur colonne de LH-20 (210 mL) équilibrée dans un mélange 1:1 dichlorométhane - éthanol. Les fractions contenant le produit sont ensuite concentrées et purifiées sur silice pour fournir **72** (30.3 mg, 96%) pouvant être partiellement estérifié sur les groupements acides carboxylique
Spectre de masse (ESI) *m*/*z* 1462.4 [(M + H)⁺].

### Etape 10.j : Préparation du (4-O-allyl-3-O-benzyl-2-O-sodium sulfonato-α-L-idopyranosyluronate de sodium)-(1-4)-(3-O-benzyl-2,6-di-O-sodium sulfonato-α-D-glucopyranosyl)-(1-4)-(3-O-benzyl-2-O-sodium sulfonato-α-L-idopyranosyluronate de sodium)-(1-4)-(3-O-benzyl-2 6-di-O-sodium-sulfonato-α-D-glucopyranosyl)-(1-4)-(5-(benzyloxy)-4-oxypipéridine-1-carboxylate de benzyle-3-carboxylate de sodium (3S,4R, 5R)) (n°73)

Le composé **72** (10.0 mg, **6.44** µmol) obtenu à l'étape 10.i, est traité selon la méthode 4 pour fournir **73** qui est engagé directement dans l'étape suivante.

### PREPARATION 11 :

### Synthèse du méthyl (2-N-sodium sulfonato-2,4-didésoxy-4-formyl-3,6-di-O-sodium sulfonato-α-D-glucopyranosyl)-(1-4)-(sodium 2-O-sodium sulfonato-α-L-idopyranosyluronate)-(1-4)-(2-N-sodium sulfonato-2-désoxy-6-O-sodium sulfonato-α-D-glucopyranoside) (n° 89)

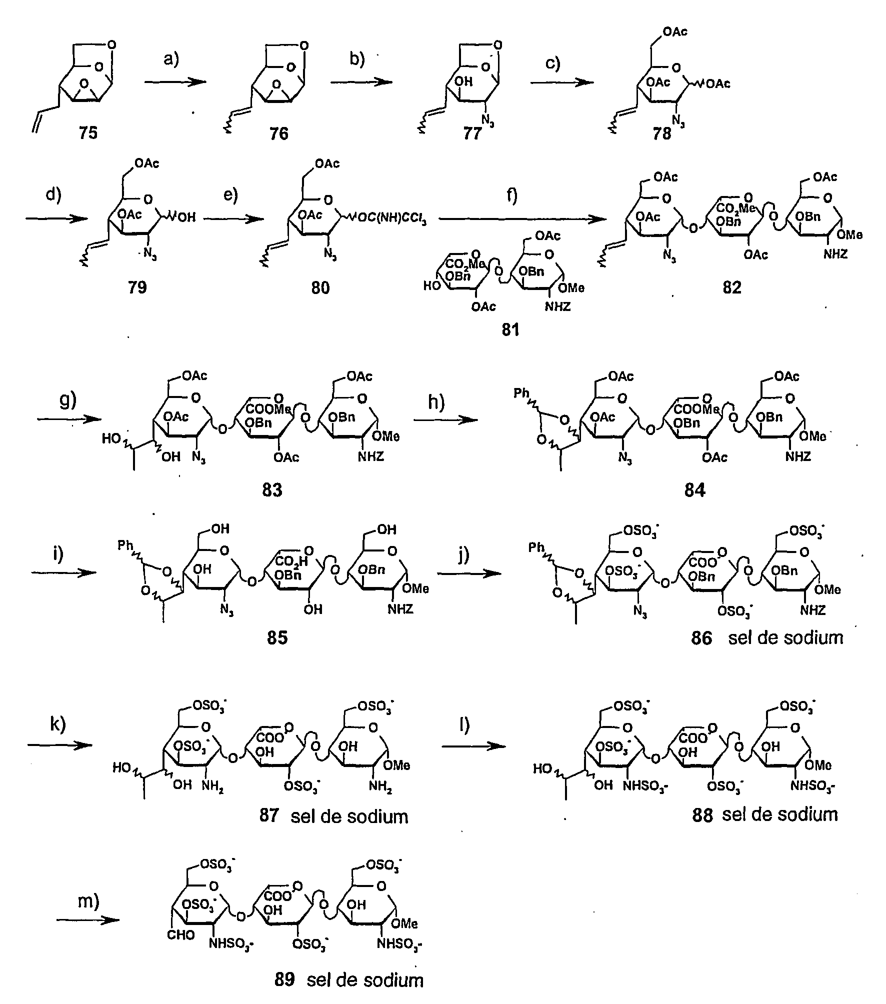

### Etape 11.a : Préparation du 1.6:2,3-di-anhydro-4-désoxy-4-(prop-1-ène-1-yl)-β-D-mannopyranose (n° 76)

A une solution d'époxyde **75** (1.2 g, 7.14 mmol) (préparé selon AG Kelly and JS Roberts, J. Chem. Soc., Chem. Commun., (1980), Vol.288) dans de l'éthanol (56.5 mL), est ajouté, sous argon, du trichlorure de rhodium monohydraté (202 mg, 0.15 équivalent molaire). Après 1 h25 d'agitation à 75°C, le milieu réactionnel est versé sur 250 mL d'eau glacée, puis après 5 min d'agitation, le produit est extrait à l'éther diéthylique, séché (Na₂SO₄), et concentré. Le résidu est ensuite purifié sur silice (éther diisopropylique - cyclohexane 45:55) et les fractions contenant le composé **76** sont partiellement concentrées **(76** est volatile).
RMN ¹H (CDCl₃) δ 3.42 (dd, H-2), 3.00 (dd, H-3), 2.64 (dd, H-4).

### Etape 11.b : Préparation du 1,6-anhydro-2-azido-2,4-didésoxy-4-(prop-1-ène-1-yl)-β-D-glucopyranose (n° 77)

Le composé **76** obtenu à l'étape 11.a est dissous dans un mélange diméthylformamide - eau (40 mL, 4:1), puis de l'azoture de sodium (7.0 g) est ajouté, et le mélange est porté à reflux pendant 10.5h. Le milieu réactionnel est ensuite extrait à l'acétate d'éthyle, lavé à l'eau puis par une solution aqueuse de chlorure de sodium saturé, séché (Na₂SO₄), concentré et purifié sur gel de silice pour donner le composé **77** (674 mg, 48%).
RMN ¹H (CDCl₃) δ 5.8-5.6 (m, 2H, CH=CH).

### Etape 11.c : Préparation du 1,3,6-tri-O-acétyl-2-azido-2,4-didésoxy-4-(prop-1-ène-1-yl)-α,β-D-glucopyranose (n° 78)

Le composé **77** (3.5 g, 16.57 mmol) obtenu à l'étape 11.b, est traité comme pour la synthèse du composé **9** (étape 2.b) pour conduire, après purification, au composé 78 (5.88 g, 100%).
Spectre de masse (ESI) *m*/*z* 378 [(M + Na)⁺].

### Etape 11.d : Préparation du 3,6-di-O-acétyl-2-azido-2,4-didésoxy-4-(prop-1-ène-1-yl)-α,β-D-glucopyranose (n° 79)

A une solution du composé **78** (5.88 g, 16.5 mmol) obtenu à l'étape 11.c, dans le tétrahydrofurane (140 mL) est ajoutée de l'éthanolamine (4.0 mL, 4 équivalents molaire), à 0 °C. Après 16h à +4 °C, le milieu est dilué à l'acétate d'éthyle, acidifié (HCl 1N), lavé à l'eau, séché (Na₂SO₄), et concentré pour conduire, après purification, au composé **79** (4.66 g, 90%).
Spectre de masse (ESI) *m*/*z* 336 [(M + Na)⁺].

### Etape 11.e : Préparation du 3,6-di-O-acétyl-2-azido-2,4-didésoxy-4-prop-1-ène-1-yl)-α,β-D-glucopyranose trichloroacétimidate (n° 80)

A une solution du composé **79** (4.66 g, 14.9 mmol) obtenu à l'étape 11.d, dans du dichlorométhane (285 mL) est ajouté, sous argon, du carbonate de potassium (K₂CO₃) (3.34 g, 1.6 équivalent molaire), puis du CCl₃CN (7.6 mL, 5 équivalents molaires). Après 17h d'agitation magnétique, le mélange réactionnel est filtré, concentré et purifié sur silice pour donner le composé 80 (5.65 g, 83%).
RMN ¹H (CDCl₃) δ 8.77 (s, NH (isomère α)), 5.70 (dd, H-3), 2.64 (d, H-1β).

### Etape 11.f : Préparation du méthyl (3,6-di-O-acétyl-2-azido-2,4-didésoxy-4-(prop-1-ène-1-yl)-α-D-glucopyranosyl)-(1-4)-(méthyl 2-O-acétyl-3-O-benzyl-α-L-idopyranosyluronate)-(1-4)-(6-O-acétyl-3-O-benzyl-2-benzyloxycarbonylamino-2-désoxy-α-D-glucopyranoside) (n° 82)

Le composé **80** (5.65 g, 12.3 mmol) obtenu à l'étape 11.e, et le composé **81** (préparé selon J.C. Jacquinet et al., Carbohydr. Res. 130 (1984), 221-241) (11.54 g, 1.2 équivalent molaire) sont mis à réagir selon la METHODE 2 pour conduire après purification, au composé **82** (9.39 g, 71 %).
Spectre de masse (ESI) *m*/*z* 1077.5 [(M + H)⁺].

### Etape 11.g : Préparation du méthyl (3,6-di-O-acétyl-2-azido-2,4-didésoxy-4-(1,2-dihydroxypropyl)-α-D-glucopyranosyl)-(1-4)-(méthyl 2-O-acétyl-3-O-benzyl-α-L-idopyranosyluronate)-(1-4)-(6-O-acétyl-3-O-benzyl-2-benzyloxycarbonylamino-2-désoxy-α-D-glucopyranoside) (n° 83)

A une solution du composé **82** (513 mg, 0.476 mmol) obtenu à l'étape 11.f, dans un mélange 1:1 tétrahydrofurane - dichlorométhane (8 mL) sont ajoutés de la N-méthylmorpholine N-oxide monohydrate (NMO) (1.11 g, 20 équivalents molaire) et du tétraoxyde d'osmium (OsO₄) 4% à dans l'eau (8.35 mL, 1 équivalent molaire). Après 3 jours d'agitation à température ambiante, un mélange 1:1 de dichlorométhane - eau est ajouté, ainsi qu'une solution à 37.5% d'hydrogénosulfite de sodium (NaHSO₃), et l'agitation est maintenue 30 min supplémentaires. Le mélange réactionnel est extrait au dichlorométhane, purifié sur silice, et la fraction contenant du produit de départ est remise à réagir dans les conditions ci-dessus jusqu'à consommation complète. Après purification, on obtient finalement le composé 83 (293 mg, 66%).
Spectre de masse (ESI) *m*/*z* 1111.4 [(M + H)⁺].

### Etape 11.h : Préparation du méthyl (3,6-di-O-acétyl-2-azido-2,4-didésoxy-4-(5-méthyl-2-phényl-1,3-dioxolan-4-yl)-α-D-glucopyranosyl)-(1-4)-(méthyl 2-O-acétyl-3-O-benzyl-α-L-idopyranosyluronate)-(1-4)-(6-O-acétyl-3-O-benzyl-2-benzyloxycarbonylamino-2-désoxy-α-D-glucopyranoside) (n° 84)

A une solution du composé 83 (518 mg, 0.466 mmol) obtenu à l'étape 11.g, dans l'acétonitrile sont ajoutés, sous argon, du CSA (21.6 mg, 0.2 molequiv), et du benzylidène diméthylacétal (160 µL, 2.3 équivalents molaire). Après 1h30 d'agitation, le milieu est neutralisé avec de la triéthylamine, concentré à sec, puis purifié sur silice pour donner le composé **84** (454 mg, 74%).
Spectre de masse (ESI) *m*/*z* 1199.5 [(M + H)⁺].

### Etape 11.i : Préparation du méthyl (2-azido-2,4-didésoxy-4-(5-méthyl-2-phényl-1,3-dioxolan-4-yl)-α-D-glucopyranosyl)-(1-4)-(acide 3-O-benzyl-α-L-idopyranosyluronique)-(1-4)-(3-O-benzyl-2-benzyloxycarbonylamino-2-désoxy-α-D-glucopyranoside) (n° 85)

Le composé **84** (215 mg, 0.18 mmol) obtenu à l'étape 11.h, est traité selon la méthode 3. Après 16h d'agitation magnétique à température ambiante, le milieu est dilué au méthanol (12.5 mL), puis une solution aqueuse de soude 4N (11.5 mL) est ajoutée à 0 °C. Le mélange est agité 4h à 0 °C, acidifié (pH 5) par de l'acide chlorhydrique 6N, puis est extrait au dichlorométhane, lavé au Na₂SO₃ 5%, et enfin au chlorure de sodium saturé. Après séchage et concentration, le résidu est purifié sur silice pour conduire au composé **85** (157 mg, 86%).
Spectre de masse (ESI) *m*/*z* 1017.3 [(M + H)⁺].

### Etape 11.i : Préparation du méthyl (2-azido-2,4-didésoxy-4-(5-méthyl-2-phényl-1,3-dioxolan-4-yl)-3,6-di-O-sodium sulfonato-α-D-glucopyranosyl)-(1-4)-(sodium 3-O-benzyl-2-O-sodium sulfonato-α-L-idopyranosyluronate)-(1-4)-(3-O-benzyl-2-benzyloxycarbonylamino-2-désoxy-6-O-sodium sulfonato-α-D-glucopyranoside) (n° 86)

Le composé **85** (160 mg, 0.157 mmol) obtenu à l'étape 11.i, est traité selon la METHODE 4 pour fournir le composé **86** (215 mg, 95%).
Spectre de masse (ESI) *m*/*z* 689.1 [(M + H - 3Na)²⁻].

### Etape 11.k : Préparation du méthyl (2-amino-2,4-didésoxy-4-(1,2-dihydroxypropyl)-3,6-di-O-sodium sulfonato-α-D-glucopyranosyl)-(1-4)-(sodium 2-O-sodium sulfonato-α-L-idopyranosyluronate)-(1-4)-(2-amino-2-désoxy-6-O-sodium sulfonato-α-D-glucopyranoside) (n° 87)

Le composé 86 (210 mg, 0.145 mmol) obtenu à l'étape 11.j est traité selon la méthode 5 sans acide acétique pour donner le composé **87** (108 mg, 73%). Si nécessaire, la réaction est reconduite plusieurs fois jusqu'à disparition totale des protons benzyliques par RMN.
Spectre de masse (ESI) *m*/*z* 996.1 [(M + H - Na)⁻].

### Etape 11.l : Préparation du méthyl (2-N-sodium sulfonato-2,4-didésoxy-4-(1,2-dihydroxypropyl)-3,6-di-O-sodium sulfonato-α-D-glucopyranosyl)-(1-4)-(sodium 2-O-sodium sulfonato-α-L-idopyranosyluronate)-(1-4)-(2-N-sodium sulfonato-2-désoxy-6-O-sodium sulfonato-α-D-glucopyranoside) (n° 88)

A une solution du composé **87** (106 mg, 0.104 mmol) obtenu à l'étape 11.k, dans l'eau (7 mL), est ajouté, à 0 °C, du complexe pyridine.SO₃ (662 mg, 4.16 mmol) tout en maintenant le pH à 9.3 avec de la soude 1 N. La température est ensuite remontée à température ambiante, le milieu réactionnel est agité 16h en maintenant le pH à 9.3, puis est purifié sur une colonne de gel Sephadex^{®} G-25 équilibrée par une solution de chlorure de sodium 0.2 M.Après rassemblement des fractions contenant le produit et concentration, le résidu est purifié par la même colonne Sephadex^{®} G-25 éluée à l'eau, pour donner le composé **88** (116 mg, 91%).
Spectre de masse (ESI) *m*/*z* 1199.8 [(M + H - Na)].

### Etape 11.m : Préparation du méthyl (2-N-sodium sulfonato-2,4-didésoxy-4-formyl-3,6-di-O-sodium sulfonato-α-D-glucopyranosyl)-(1-4)-(sodium 2-O-sodium sulfonato-(α-L-idopyranosyluronate)-(1-4)-(2-N-sodium sulfonato-2-désoxy-6-O-sodium sulfonato-α-D-glucopyranoside) (n° 89)

Du périodate de sodium (22.1 mg, 1.1 équivalent molaire) est ajouté à une solution du composé **88** (115 mg, 94 µmol) obtenu à l'étape 11.l, dans l'eau (1.9 mL). Après 1h d'agitation magnétique, le milieu réactionnel est purifié sur une colonne de gel Sephadex^{®} G-15 équilibrée en eau pour conduire au composé **89** (107 mg, 96%).

### PREPARATION 12 :

### Synthèse du (2-acétamido-3,4-di-O-benzyl-2-désoxy-6-O-sodium sulfonato-α-D-glucopyranosyl)-(1-4)-(5-(benzyloxy)-4-oxypipéridine-1-carboxylate de benzyle-3-carboxylate de sodium (3S, 4R, 5R)) (n° 97)

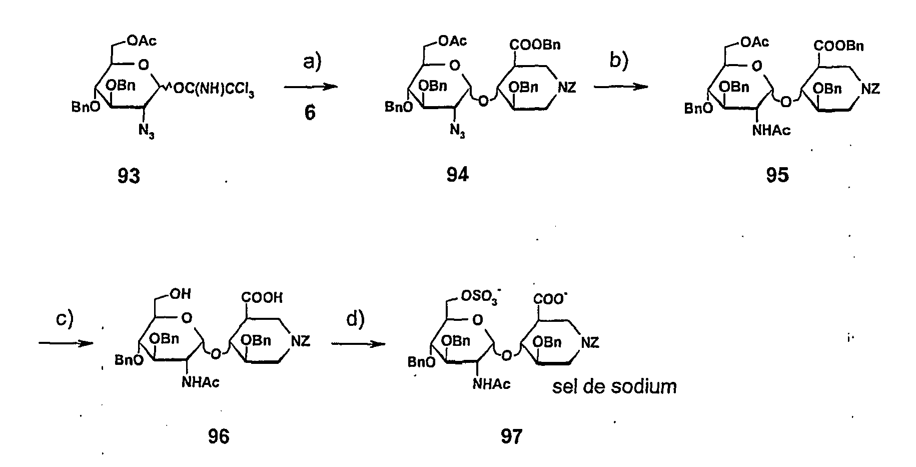

### Etape 12,a : Préparation du (6-O-acétyl-2-azido-3,4-di-O-benzyl-2-désoxy-α-D-glucopyranosyl)-(1-4)-(5-(benzyloxy)-4-oxypipéridine-1,3-dicarboxylate de dibenzyle (3S, 4R, 5R)) (n° 94)

Le composé **93** (préparé selon R. Verduyn et al., Recl. Trav. Chim. Pays-Bas, 109 (1990), 12, 591) (361 mg, 0.631 mmol) et le composé **6** (200 mg, 0.421 mmol) obtenu à l'étape 1.e, sont traités selon la méthode 2 pour fournir, après purification, le composé **94** (224 mg, 60%).
Spectre de masse (ESI) *m*/*z* 885.4 [(M + H)⁺].

### Etape 12.b : Préparation du (6-O-acétyl-2-acétamido-3,4-di-O-benzyl-2-désoxy-α-D-glucopyranosyl)-(1-4)-(5-(benzyloxy)-4-oxypipéridine-1,3-dicarboxylate de dibenzyle (3S, 4R, 5R)) (n° 95)

Le composé **94** (56.3 mg, 63.6 µmol) obtenu à l'étape 12.a, est traité comme pour la synthèse de **17** (étape 3.f), pour conduire au composé **95** (54.5 mg, 95%). Spectre de masse (ESI) *m*/*z* 901.2 [(M + H)⁺].

### Etape 12.c : Préparation du (2-acétamido-3,4-di-O-benzyl-2-désoxy-α-D-glucopyranosyl)-(1-4)-(5-(benzyloxy)-4-oxypipéridine-1-carboxylate de benzyle-3-acide carboxylique (3S, 4R, 5R)) (n° 96)

Le composé 95 (101 mg, 50.9 µmol) obtenu à l'étape 12.b, est traité selon la METHODE 3. Le résidu est engagé brut dans l'étape suivante.

### Etape 12.d : Préparation du (2-acétamido-3,4-di-O-benzyl-2-désoxy-6-O-sodium sulfonato-α-D-glucopyranosyl)-(1-4)-(5-(benzyloxy)-4-oxypipéridine-1-carboxylate de benzyle-3-carboxylate de sodium- (3S, 4R, 5R)) (n° 97)

Le composé **96** brut obtenu à l'étape 12.c, est traité d'après la METHODE 4, pour fournir le composé **97** (35 mg, 88% (2 étapes)), pouvant être partiellement estérifié sur la fonction acide carboxylique.
Spectre de masse (ESI) *m*/*z* 847.2 [(M - H)⁻].

### PREPARATION 13 :

### Synthèse du 3-(benzyloxy)-5-[(benzyloxy)méthyl]-4-hydroxypipéridine-1-carboxylate de benzyle (3R, 4R, 5R) (n° 100)

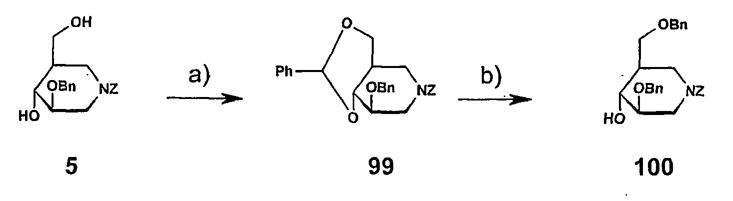

### Etape 13.a : Préparation du 8-(benzyloxy)-2-phényltétrahydro-4H-[1,3]dioxino[5,4-c]pyridine-6(5H)-carboxylate de benzyle (4aR, 8R, 8aR) (n° 99)

A une solution du composé **5** (250 mg, 0.67 mmol) dans l'acétonitrile (13.4 mL) est ajouté de l'acide camphorsulfonique (31 mg, 0.2 moléquiv) puis du benzaldéhyde diméthylacétal (0.23 mL, 2.3 moléquiv). Après 1h d'agitation magnétique à température ambiante, le milieu réactionnel est neutralisé par de la triéthylamine, concentré, et purifié sur gel de silice (15:85 acétate d'éthyle - cyclohexane) pour fournir le composé **99** (281 mg, 91%).
Spectre de masse (ESI) *m*/*z* 482.2 [(M + Na)⁺].

### Etape 13.b : Préparation du 3-(benzyloxy)-5-[(benzyloxy)méthyl]-4-hydroxypipéridine-1-carboxylate de benzyle (3R, 4R 5R) (n° 100)

A une solution du composé **99** (141 mg, 0.31 mmol) obtenu à l'étape 13.a dans du dichlorométhane (1.2 mL), sont ajoutés successivement, à 0 °C, sous argon, du triéthylsilane (0.20 mL, 4 équivalents molaire), de l'acide trifluoroacétique (0.09 mL, 4 équivalents molaire), et de l'anhydride trifluoroacétique (3 µL, 0.07 équivalents molaire). La température est maintenue à 0 °C pendant 5 min, puis le milieu réactionnel est placé à température ambiante pendant 3.5 h. Le mélange réactionnel est alors neutralisé par une solution aqueuse d'hydrogénocarbonate de sodium, à l'eau, et la phase organique est séchée (Na₂SO₄), filtrée, et concentrée sous vide. Le résidu est purifié sur gel de silice pour fournir le composé 100 (76 mg, 54%).
Spectre de masse (ESI) *m*/*z* 462.3 [(M + H)⁺].

### PREPARATION 14 :

### Synthèse du (3-O-benzyl-2,4-di-O-sodium sulfonato-α-L-idopyranosyluronate de sodium)-(1-4)-(2-acétamido-3-O-benzyl-2-désoxy-6-O-sodium sulfonato-α-D-glucopyranosyl)-(1-4)-(3-O-benzyl-2-O-sodium sulfonato-α-L-idopyranosyluronate de sodium)-(1-4)-(2-acétamido-3-O-benzyl-2-désoxy-6-O-sodium sulfonato-α-D-glucopyranosyl)-(1-4)-(3-(benzyloxy)-5-[(benzyloxy)méthyl]-4-oxypipéridine-1-carboxylate de benzyle (3R, 4R, 5R)) (n° 104)

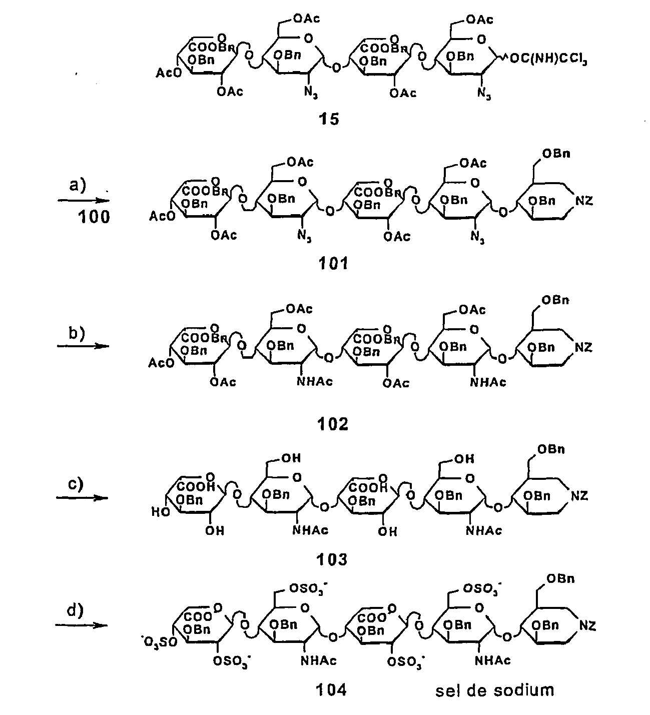

### Etape 14.a : Préparation du (benzyl 2,4-di-O-acétyl-3-O-benzyl-α-L-idopyranosyluronate)-(1-4)-(6-O-acétyl-2-azido-3-O-benzyl-2-désoxy-α-D-glucopyranosyl)-(1-4)-(benzyl 2-O-acétyl-3-O-benzyl-α-L-idopyranosyluronate)-(1-4)-(6-O-acétyl-2-azido-3-O-benzyl-2-désoxy-α-D-glucopyranosyl)-(3-(benzyloxy)-5-[(benzyloxy)méthyl]-4-oxypipéridine-1-carboxylate de benzyle (3R, 4R, 5R)) (n° 101)

Les composés **15** (123 mg, 0.075 mmol) et **100** (69 mg, 0.149 mmol) sont traités selon la METHODE 2 pour fournir, après purification, le composé **101** (117 mg, 80%). Rapport α/β 55/45.
Spectre de masse (ESI) *m*/*z* 1962 [(M + Na)⁺].

### Etape 14.b : Préparation du (benzyl 2,4-di-O-acétyl-3-O-benzyl-α-L-idopyranosyluronate)-(1-4)-(6-O-acétyl-2-acétamido-3-O-benzyl-2-désoxo-α-D-glucopyranosyl)-(1-4)-(benzyl 2-O-acétyl-3-O-benzyl-α-L-idopyranosyluronate)-(1-4)-(6-O-acétyl-2-acétamido-3-O-benzyl-2-désoxy-α-D-glucopyranosyl)-(1-4)-(3-(benzyloxy)-5-[(benzyloxy)méthyl]-4-oxypipéridine-1-carboxylate de benzyle (3R, 4R, 5R)) (n° 102)

Le composé **101** (117 mg, 60 µmol) est dissous dans la pyridine (1 mL), puis de l'acide thioacétique (1 mL, 225 équivalents molaire) est ajouté à 0 °C. Le milieu réactionnel est agité 17 h à température ambiante, puis est concentré et purifié sur gel de silice (4:96 éthanol - toluène), pour conduire au composé **102** (50 mg, 42%). Spectre de masse (ESI) *m*/*z* 1971.9 [(M + H)⁺].

### Etape 14.c : Préparation de l'acide 3-O-benzyl-α-L-idopyranosyluronique-(1-4)-(2-acétamido-3-O-benzyl-2-désoxy-α-D-glucopyranosyl)-(1-4)-(acide 3-O-benzyl-α-L-idopyranosyluronique)-(1-4)-(2-acétamido-3-O-benzyl-2-désoxy-α-D-glucopyranosyl)-(1-4)-(3-(benzyloxy)-5-[(benzyloxy)méthyl]-4-oxypipéridine-1-carboxylate de benzyle (3R, 4R, 5R)) (n° 103)

Le composé **102** (50 mg, 25 µmol) est traité selon la METHODE 3 pour conduire au dérivé **103.**
Spectre de masse (ESI) *m*/*z* 1581.7 [(M + H)⁺].

### Etape 14.d : Préparation du (3-O-benzyl-2,4-di-O-sodium sulfonato-α-L-idopyranosyluronate de sodium)-(1-4)-(2-acétamido-3-O-benzyl-2-désoxy-6-O-sodium sulfonato-α-D-glucopyranosyl)-(1-4)-(3-O-benzyl-2-O-sodium sulfonato-α-L-idopyranosyluronate de sodium)-(1-4)-(2-acétamido-3-O-benzyl-2-désoxy-6-O-sodium sulfonato-α-D-glucopyranosyl)-(1-4)-(3-(benzyloxy)-5-[(benzyloxy)méthyl]-4-oxypipéridine-1-carboxylate de benzyle (3R, 4R, 5R)) (n° 104)

Le composé **103** est traité d'après la METHODE 4, pour fournir le composé **104** (43 mg, 80% (2 étapes)).
Spectre de masse (ESI) *m*/*z* 2134.3 [(M - Na)].

### PREPARATION 15 :

### Synthèse du (benzyl 2-O-acétyl-3-O-benzyl-4-O-phénylpropyl-α-L-idopyranosyluronate)-(1-4)-(6-O-acétyl-2-azido-3-O-benzyl-2-désoxy-α,β-D-glucopyranose trichloroacétimidate) (n° 114)

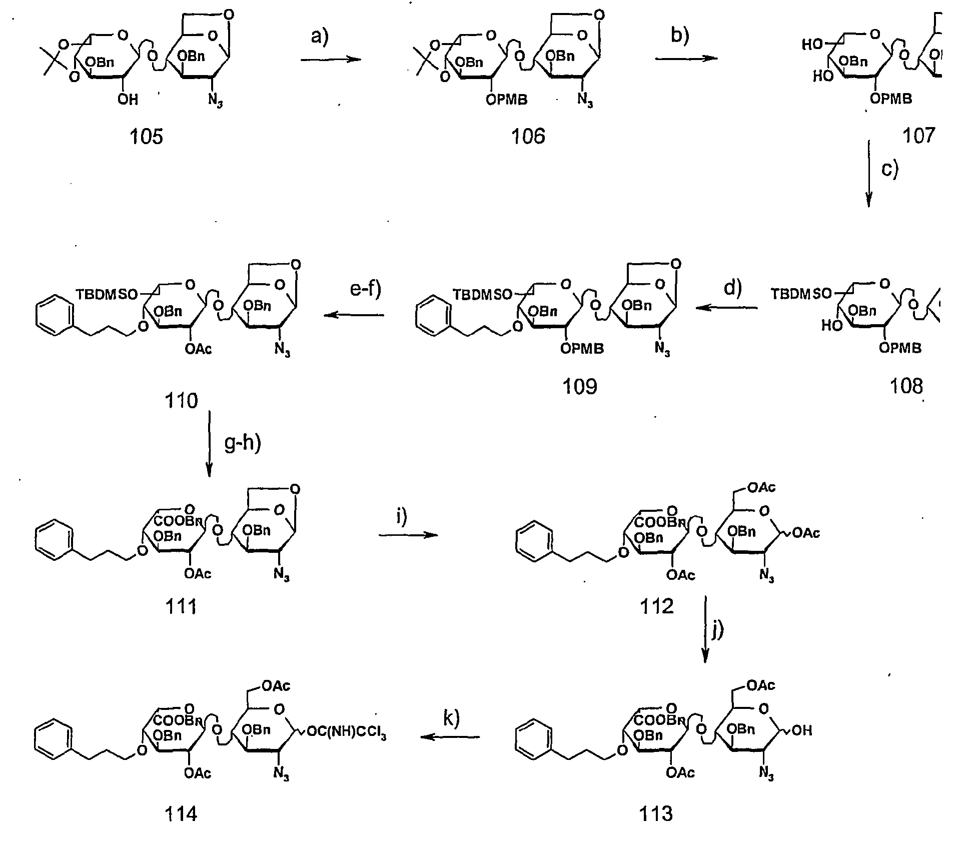

### Etape 15.a : Préparation du (4,6-O-isopropylidène-3-O-benzyl-2-O-(4-méthoxy)benzyl-α-L-idopyranosyl)-(1-4)-(1,6-anhydro-2-azido-3-O-benzyl-2-désoxy-β-D-glucopyranose) (n° 106)

A une solution du composé 105 (63.2 g, 111 mmol) (préparé selon C.A.A. van Boeckel et al., J. Carbohydrate Chemistry (1985) 4 (3), 293-321) dans du DMF (445 mL) est ajouté, à 0 °C et sous argon, du NaH (6.93 g, 1.3 équivalent molaire) puis du chlorure de para-méthoxybenzyle (24 mL, 1.6 équivalent molaire). Après 2 h d'agitation magnétique, du méthanol est additionné (9 mL), le milieu réactionnel est concentré sous vide, le brut réactionnel est dilué à l'acétate d'éthyle, lavé à l'eau, séché (Na₂SO₄), filtré et concentré. Le résidu obtenu est purifié sur silice (acétate d'éthyle - cyclohexane 15:85) pour fournir 106.
Spectre de masse (ESI) *m*/*z* 707.3 [(M + NH₄)⁺].

### Etape 15.b : Préparation du (3-O-benzyl-2-O-(4-méthoxy)benzyl-α-L-idopyranosyl)-(1-4)-(1,6-anhydro-2-azido-3-O-benzyl-2-désoxy-β-D-glucopyranose) (n° 107)

Le composé **106** obtenu à l'étape précédente est mis en présence d'acide acétique à 80% dans l'eau. Après 15 h d'agitation magnétique, le mélange réactionnel est refroidi par de la glace, dilué (dichlorométhane) et neutralisé à l'hydrogénocarbonate de sodium. La phase organique est séchée (Na₂SO₄), filtrée et concentrée. Le résidu obtenu est purifié sur silice (acétate d'éthyle - cyclohexane 3:7) pour fournir **107** (63.2 g, 88%, 2 étapes).
Spectre de masse (ESI) *m*/*z* 672.3 [(M + Na)⁺].

### Etape 15.c : Préparation du (3-O-benzyl-6-O-tert-butyldiméthylsilyl-2-O-(4-méthoxy)benzyl-α-L-idopyranosyl)-(1-4)-(1,6-anhydro-2-azido-3-O-benzyl-2-désoxy-β-D-glucopyranose) (n° 108)

Le composé **107** (64.2 g) est mis en solution dans du dichlorométhane (200 mL). A 0 °C et sous argon, sont additionnés successivement de la triéthylamine (30.3 mL, 2.2 équivalents molaire), du 4-diméthylaminopyridine (1.21 g, 0.1 équivalent molaire), et du chlorure de *tert*-butyldiméthylsilyle (17.04 g, 1.1 équivalent molaire). Après 4 h d'agitation magnétique, 10% de chlorure de *tert*-butyldiméthylsilyle sont rajoutés, et après une heure, le milieu réactionnel est dilué par du dichlorométhane, lavé à l'eau, séché (Na₂SO₄), filtré et concentré. Le résidu obtenu est purifié sur silice (acétate d'éthyle - cyclohexane 15:85) pour fournir **108**.
Spectre de masse (ESI) *m*/*z* 786.3 [(M + Na)⁺].

### Etape 15.d : Préparation du (3-O-benzyl-6-O-tert-butyldiméthylsilyl-2-O-(4-méthoxy)benzyl-4-O-phénylpropyl-α-L-idopyranosyl)-(1-4)-(1,6-anhydro-2-azido-3-O-benzyl-2-désoxy-β-D-glucopyranose) (n° 109)

A une solution du composé **108** dans du diméthylformamide (485 mL) est ajouté, à 0 °C et sous argon, du bromure de phénylpropyle (74 mL, 5 équivalents molaire) puis du NaH (7 g, 1.5 équivalent molaire). Après 5.5 h d'agitation magnétique, du méthanol est additionné (50 mL), le milieu réactionnel est concentré sous vide, le brut réactionnel est dilué à l'acétate d'éthyle, lavé à l'eau, séché (Na₂SO₄), filtré et concentré. Le résidu obtenu est purifié sur silice (acétate d'éthyle - cyclohexane 15:85) pour fournir 109 (49.3 g, 58%, 2 étapes).
Spectre de masse (ESI) *m*/*z* 904.3 [(M + Na)⁺].

### Etape 15.e-f : Préparation du (2-O-acétyl-3-O-benzyl-6-O-tert-butyldiméthylsilyl-4-O-phénylpropyl-α-L-idopyranosyl)-(1-4)-(1,6-anhydro-2-azido-3-O-benzyl-2-désoxy-_ α-D-glucopyranose) (n° 110)

A une solution de **109** (49.3 g, 55.9 mmol) dans du dichlorométhane (2.2 L) est ajouté de l'eau (112 mL) puis, à 0 °C, du DDQ (19.03 g, 1.5 équivalent molaire). Après 3 h d'agitation à 0 °C, une solution d'hydrogénocarbonate de sodium est additionnée. La phase organique est séchée (Na₂SO₄), filtrée et concentrée. Le résidu obtenu est dissous dans de la pyridine (335 mL), puis de l'anhydride acétique (28 mL) et du 4-diméthylaminopyridine (682 mg) sont additionnés. Après 16 h d'agitation magnétique, le mélange réactionnel est concentré sous vide et le résidu obtenu est purifié sur silice (acétate d'éthyle - cyclohexane 15:85) pour fournir **110** (34.4 g, 77%, 2 étapes).
Spectre de masse (ESI) *m*/*z* 826.4 [(M + Na)⁺].

### Etape 15.g-h : Préparation du (benzyl 2-O-acétyl-3-O-benzyl-4-O-phénylpropyl-α-L-idopyranosyluronate)-(1-4)-(1,6-anhydro-2-azido-3-O-benzyl-2-désoxy-β-D-glucopyranose) (n° 111)

A une solution de **110** (25.17 g, 31.3 mmol) dans l'acétone (1.46 L) est ajoutée, à 0 °C, une solution aqueuse 3.5 M d'acide sulfurique (45 mL) contenant de l'anhydride chromique (10 g). Après 3 h d'agitation magnétique à 0 °C, le milieu réactionnel est dilué au dichlorométhane, lavé à l'eau, séché, filtré et concentré pour fournir un brut réactionnel qui est engagé directement dans l'étape suivante. Le résidu précédemment obtenu est mis en solution dans du diméthylformamide (230 mL) et de l'hydrogénocarbonate de potassium (16.7 g, 5 équivalents molaires) ainsi que du bromure de benzyle (39.8 mL, 10 équivalents molaires) sont additionnés. Le mélange réactionnel est agité 16 h à température ambiante, puis est dilué à l'acétate d'éthyle, lavé à l'eau, séché, filtré, concentré et purifié sur gel de silice (acétate d'éthyle - toluène 1:4) pour fournir le composé **111** (22.6 g, 91%, 2 étapes).
Spectre de masse (ESI) *m*/*z* 811.3 [(M + NH₄)⁺].

### Etape 15.i : Préparation du (benzyl 2-O-acétyl-3-O-benzyl-4-O-phénylpropyl-α-L-idopyranosyluronate)-(1-4)-(1,6-di-O-acétyl-2-azido-3-O-benzyl-2-désoxy-α,β-D-glucopyranose) (n° 112)

A une solution du composé **111** (1.11 g, 1.39 mmol) dans de l'anhydride acétique (13.2 mL, 100 équivalents molaire), est additionné, à 0 °C, de l'acide trifluoroacétique (TFA) (1.14 mL, 11 équivalents molaire). Après retour à température ambiante, le mélange réactionnel est agité pendant 3.5 h, puis est concentré, coévaporé au toluène, et purifié sur gel de silice (85:15 toluène - acétate d'éthyle), pour donner le composé **112** (1.15 g, 93%).
Spectre de masse (ESI) *m*/*z* 918.3 [(M + Na)⁺].

### Etape 15.j : Préparation du (benzyl 2-O-acétyl-3-O-benzyl-4-O-phénylpropyl-α-L-idopyranosyluronate)-(1-4)-(6-O-acétyl-2-azido-3-O-benzyl-2-désoxy-α,β-D-glucopyranose) (n° 113)

A une solution du composé **112** (1.13 g, 1.26 mmol) dans de l'éther diéthylique (51 mL), est additionnée, à 0 °C, de la benzylamine (BnNH₂) (5.25 mL, 38 équivalents molaire). Après 5h15 d'agitation à température ambiante, le milieu est acidifié avec de l'HCl 1N, puis est extrait à l'éther diéthylique, séché (Na₂SO₄), concentré, et purifié sur gel de silice (35:65 acétate d'éthyle - cyclohexane) pour conduire à **113** (0.97 g, 90%).
Spectre de masse (ESI) *m*/*z* 854.3 [(M + H)⁺].

### Etape 15.k : Préparation du (benzyl 2-O-acétyl-3-O-benzyl-4-O-phénylpropyl-α-L-idopyranosyluronate)-(1-4)-(6-O-acétyl-2-azido-3-O-benzyl-2-désoxy-α,β-D-glucopyranose trichloroacétimidate) (n° 114)

A une solution du composé **113** (0.95 g, 1.12 mmol) dans le dichlorométhane (21.2 mL), sont additionnés, sous argon, du carbonate de césium (Cs₂CO₃) (0.583 g, 1.6 équivalent molaire), puis du trichloroacétonitrile (CCl₃CN) (0.56 mL, 5.0 équivalents molaire). Après 35 min d'agitation, le mélange réactionnel est filtré, puis concentré. Le résidu est purifié sur gel de silice (25:75 acétate d'éthyle - cyclohexane) pour donner 114 (995 mg, 90%).
Spectre de masse (ESI) *m*/*z* 1021.5 [(M + Na)⁺].

### PREPARATION 16 :

### Synthèse du (benzyl 3-O-benzyl-4-O-phénylpropyl-2-O-sodium sulfonato-α-L-idopyranosyluronate)-(1-4)-(2-acétamido-3-O-benzyl-2-désoxy-6-O-sodium sulfonato-α-D-glucopyranosyl)-(1-4)-(benzyl 3-O-benzyl-2-O-sodium sulfonato-α-L-idopyranosyluronate)-(1-4)-(2-acétamido-3-O-benzyl-2-désoxy-6-O-sodium sulfonato-α,D-glucopyranosyl)-(1-4)-(5-(benzyloxy)-4-oxypipéridine-1,3-dicarboxylate de dibenzyle (3S, 4R, 5R)) (n° 122)

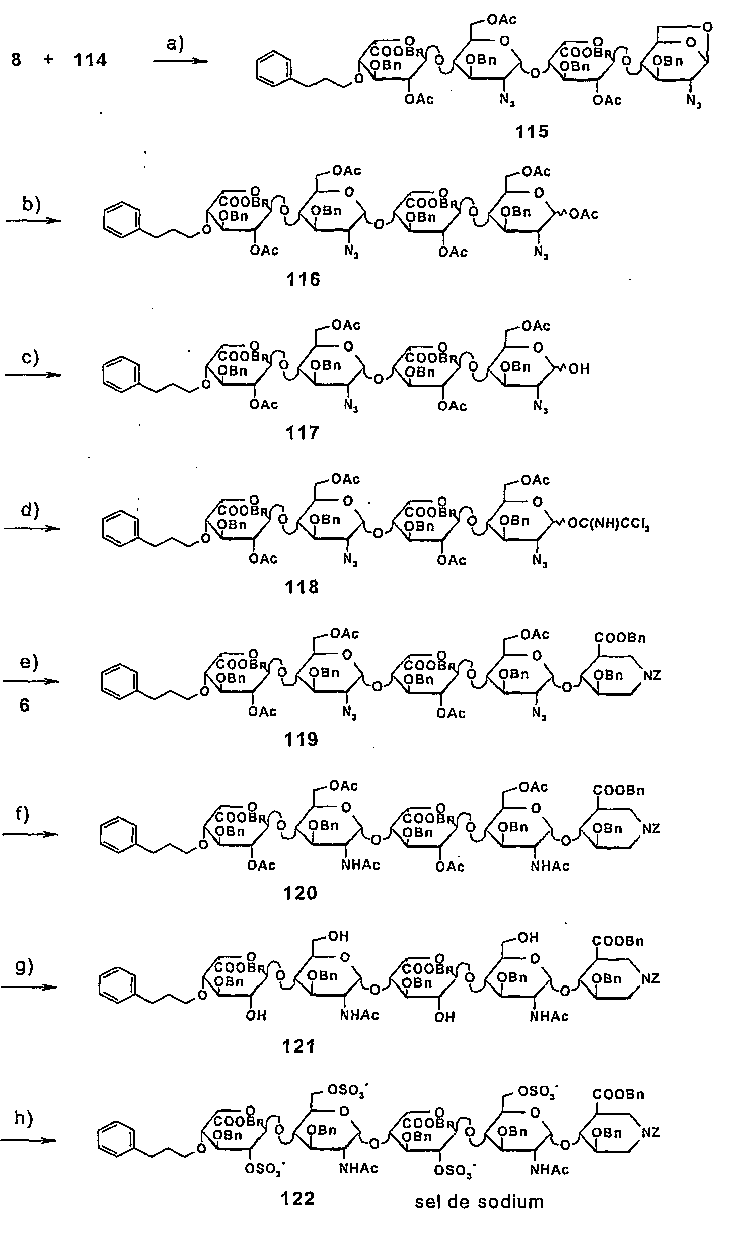

### Etape 16.a : Préparation du (benzyl 2-O-acétyl-3-O-benzyl-4-O-phénylpropyl-α-L-idopyranosyluronate)-(1-4)-(6-O-acétyl-2-azido-3-O-benzyl-2-désoxy-α-D-glucopyranosyl)-(1-4)-(benzyl 2-O-acétyl-3-O-benzyl-α-L-idopyranosyluronate)-(1-4)-(1,6-anhydro-2-azido-3-O-benzyl-2-désoxy-β-D-glucopyranose) (n° 115)

Le composé **114** (990 mg, 0.99 mmol) et le composé **8** (1.15 g, 1.7 mmol) sont traités selon la méthode 2 pour fournir, après purification, le composé **115** (623 mg, 42%).
Spectre de masse (ESI) *m*/*z* 1533.8 [(M + Na)⁺].

### Etape 16.b : Préparation du (benzyl 2-O-acétyl-3-O-benzyl-4-O-phénplpropyl-α-L-idopyranosyluronate)-(1-4)-(6-O-acétyl-2-azido-3-O-benzyl-2-désoxy-α-D-glucopyranosyl)-(1-4)-(benzyl 2-O-acétyl-3-O-benzyl-α-L-idopyranosyluronate)-(1-4)-(1,6-di-O-acétyl-2-azido-3-O-benzyl-2-désoxy-α,β-D-glucopyranose) (n° 116)

Le composé **115** (590 mg, 0.39 mmol) est traité comme pour la synthèse du composé **112** pour conduire, après purification sur gel de silice (7:3 cyclohexane - acétate d'éthyle), à **116** (609 mg, 97%).
Spectre de masse (ESI) *m*/*z* 1636.2 [(M + Na)⁺].

### Etape 16.c : Préparation du (benzyl 2-O-acétyl-3-O-benzyl-4-O-phénylpropyl-α-L-idopyranosyluronate)-(1-4)-(6-O-acétyl-2-azido-3-O-benzyl-2-désoxy-α-D-glucopyranosyl)-(1-4)-(benzyl 2-O-acétyl-3-O-benzyl-α-L-idopyranosyluronate)-(1-4)-(6-O-acétyl-2-azido-3-O-benzyl-2-désoxy-α,β-D-glucopyranose) (n° 117)

Le composé **116** (592 mg, 0.367 mmol) est traité comme pour la synthèse du composé **113** pour donner, après purification sur gel de silice (65:35 cyclohexane - acétate d'éthyle), le composé **117** (530 mg, 92%).
Spectre de masse (ESI) *m*/*z* 1593.9 [(M + Na)⁺].

### Etape 16.d : Préparation du (benzyl 2-O-acétyl-3-O-benzyl-4-O-phénylpropyl-α-L-idopyranosyluronate)-(1-4)-(6-O-acétyl-2-azido-3-O-benzyl-2-désoxy-α-D-glucopyranosyl)-(1-4)-benzyl 2-O-acétyl-3-O-benzyl-α-L-idopyranosyluronate)-(1-4)-(6-O-acétyl-2-azido-3-O-benzyl-2-désoxy-α,β-D-glucopyranose trichloroacétimidate) (n° 118)

Le composé **117** (511 mg, 0.325 mmol) est traité comme pour la synthèse du composé **114** pour fournir, après purification sur gel de silice (7:3 cyclohexane - acétate d'éthyle), 118 (495 mg, 89%).
Analyse élémentaire calculée pour C₈₅H₉₀Cl₃N₇O₂₅: C, 59.49 ; H, 5.29 ; N, 5.71. Trouvée : C, 59.49 ; H, 5.50 ; N, 5.48.

### Etape 16.e : Préparation du (benzyl 2-O-acétyl-3-O-benzyl-4-O-phénylpropyl-α-L-idopyranosyluronate)-(1-4)-(6-O-acétyl-2-azido-3-O-benzyl-2-désoxy-α-D-glucopyranosyl)-(1-4)-(benzyl 2-O-acétyl-3-O-benzyl-α-L-idopyranosyluronate)-(1-4)-(6-O-acétyl-2-azido-3-O-benzyl-2-désoxy-α-D-glucopyranosyl)-(1-4)-(5-(benzyloxy)-4-oxypipéridine-1,3-dicarboxylate de dibenzyle (3S, 4R, 5R)) (n° 119)

Les composés **118** (479 mg, 0.279 mmol) et **6** (255 mg, 0.536 mmol), sont traités selon la METHODE 2 pour fournir, après purification, le composé **119** (375 mg, 66%).
Analyse élémentaire calculée pour C₁₁₁H₁₁₇N₇O₃₀: C, 59.49 ; H, 5.29 ; N, 5.71. Trouvée : C, 59.49 ; H, 5.50 ; N, 5.48.

### Etape 16.f : Préparation du (benzyl 2-O-acétyl-3-O-benzyl-4-O-phénylpropyl-α-L-idopyranosyluronate)-(1-4)-(6-O-acétyl-2-acétamido-3-O-benzyl-2-désoxy-α-D-glucopyranosyl)-(1-4)-(benzyl 2-O-acétyl-3-O-benzyl-α-L-idopyranosyluronate)-(1-4)-(6-O-acétyl-2-acétamido-3-O-benzyl-2-désoxy-α-D-glucopyranosyl)-(1-4)-(5-(benzyloxy)-4-oxypipéridine-1,3-dicarboxylate de dibenzyle (3S, 4R, 5R)) (n° 120)

Le composé **119** (180 mg, 88.7 µmol) est dissous dans la pyridine (1.4 mL), puis de l'acide thioacétique (1.4 mL, 225 équivalents molaire) est ajouté à 0 °C. Le milieu réactionnel est agité 17h à température ambiante, puis est concentré et purifié sur gel de silice (4:1 toluène - acétone), pour conduire au composé **120** (153 mg, 84%).
Spectre de masse (ESI) *m*/*z* 2084.8 [(M + Na)⁺].

### Etape 16.g : Préparation du (benzyl 3-O-benzyl-4-O-phénylpropyl-α-L-idopyranosyluronate)-(1-4)-(2-acétamido-3-O-benzyl-2-désoxy-α-D-glucopyranosyl)-(1-4)-(benzyl 3-O-benzyl-α-L-idopyranosyluronate)-(1-4)-(2-acétamido-3-O-benzyl-2-désoxy-α-D-glucopyranosyl)-(1-4)-(5-(benzyloxy)-4-oxvpipéridine-1,3-dicarboxylate de dibenzyle (3S, 4R, 5R)) (n° 121)

Le composé **120** (190 mg, 93.6 µmol) est traité selon la METHODE 3.
Le polyol obtenu est dissous dans du diméthylformamide (4.4 mL), et de l'hydrogénocarbonate de potassium (85 mg, 10 équivalents molaire) ainsi que du bromure de benzyle (202 µL, 20 équivalents molaire) sont additionnés à 0 °C. Le mélange réactionnel est agité à température ambiante pendant 16 h, puis purifié sur colonne LH-20.
Une purification sur gel de silice (acétate d'éthyle - cyclohexane 2:3) permet d'obtenir **121** (108 mg, 62% (2 étapes)).
Spectre de masse (ESI) *m*/*z* 1884.2 [(M + Na)⁺].

### Etape 16.h : Préparation du (benzyl 3-O-benzyl-4-O-phénylpropyl-2-O-sodium sulfonato-α-L-idopyranosyluronate)-(1-4)-(2-acétamido-3-O-benzyl-2-désoxy-6-O-sodium sulfonato-α-D-glucopyranosyl)-(1-4)-(benzyl 3-O-benzyl-2-O-sodium sulfonato-α-L-idopyranosyluronate)-(1-4)-(2-acétamido-3-O-benzyl-2-désoxy-6-O-sodium sulfonato-a-D-glucopyranosyl)-(1-4)-(5-(benzyloxy)-4-oxypipéridine-1,3-dicarboxylate de dibenzyle (3S, 4R, 5R)) (n° 122)

Le composé **121** (41 mg, 21.6 µmol) est traité d'après la METHODE 4, pour fournir le composé **122** (49 mg, 99%).
Spectre de masse (ESI) *m*/*z* 2301.0 [(M - H)⁻].

Les exemples qui suivent illustrent la préparation de composés de l'invention sans la limiter. Les spectres de masse et R.M.N. confirment les structures des composés obtenus.

### EXEMPLE 1 :

### Synthèse du (2,4-di-O-sodium sulfonato-α-L-idopyranosyluronate de sodium)-(1-4)-(2-acétamido-2-désoxy-6-O-sodium sulfonato-α-D-glucopyranosyl)-(1-4)-(2-O-sodium sulfonato-α-L-idopyranosyluronate de sodium)-(1-4)-(2-acétamido-2-désoxy-6-O-sodium sulfonato-α-D-glucopyranosyl)-(1-4)-(5-(hydroxy)-4-oxypipéridine-3-carboxylate de sodium (3S, 4R, 5R)) (composé n° 20)

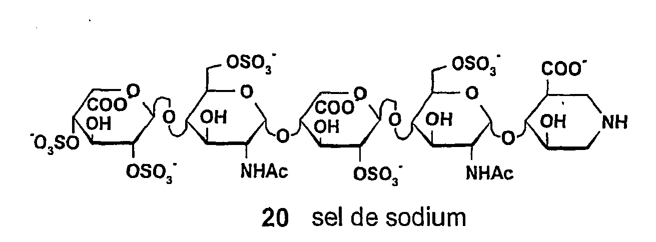

Le composé **19** de la **PREPARATION 3** (50 mg, 24.02 µmol) est traité selon la méthode 5 pour conduire au composé 20 (26 mg, 72%).
RMN ¹H (D₂O) δ 5.23 (d, H-1 Glc^{II}), 5.13 (d, H-1 IdoUA^{III}), 5.10 (d, H-1 IdoUA^{V}), 5.09 (d, H-1 Glc^{IV}), 3.62, 3.04, 2.64, 2.47 (4m, 4H, H-2, H-2', H-6, H-6' pip^{I}).

### EXEMPLE 2 :

### Synthèse du 2-acétamido-2-désoxy-6-O-sodium sulfonato-α-D-glucopyranosyl)-(1-4)-(5-(hydroxy)-4-oxypipéridine-3-carboxylate de sodium (3S, 4R, 5R)) (composé n° 21)

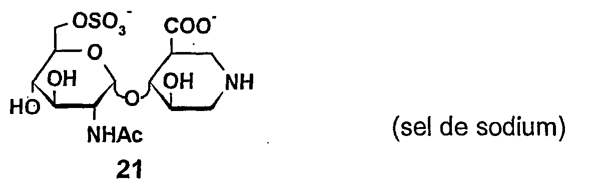

Le composé **97** de la PREPARATION 12 est traité selon la méthode 5 pour conduire au composé **21.**
RMN ¹H (D₂O) δ 5.21 (d, H-1 Glc^{II}), 3.40, 3.40, 3.40, 3.10 (4m, 4H, H-2, H-2', H-6, H-6'pip^{I}).

### EXEMPLE 3 :

### Synthèse du (2,4-di-O-sodium sulfonato-α-L-idopyranosyluronate de sodium)-(1-4)-(2-acétamido-2-désoxy-6-O-sodium sulfonato-α-D-glucopyranosyl)-(1-4)-(5-(hydroxy)-4-oxypipéridine-3-carboxylate de sodium (3S, 4R, 5R)) (composé n° 22)

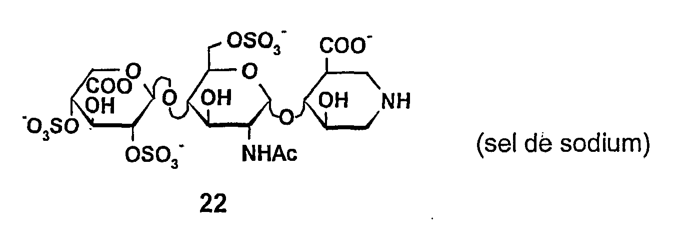

De la même manière a été préparé le composé **22.**
RMN ¹H (D₂O) δ 5.19 (d, H-1 Glc^{II}), 5.15 (d, H-1 IdoUA^{III}), 3.27, 3.23, 3.09, 2.89 (4m, 4H, H-2, H-2', H-6, H-6' pip^{I}).

### EXEMPLE 4 :

### Synthèse du (2-acétamido-2-désoxy-6-O-sodium sulfonato-α-D-glucopyranosyl)-(1-4)-(2-O-sodium sulfonato-α-L-idopyranosyluronate de sodium)-(1-4)-(2-acétamido-2-désoxy-6-O-sodium sulfonato-α-D-glucopyranosyl)-(1-4)-(5-(hydroxy)-4-oxypipéridîne-3-carboxylate de sodium (3S ,4R, 5R)) (Composé n°23)

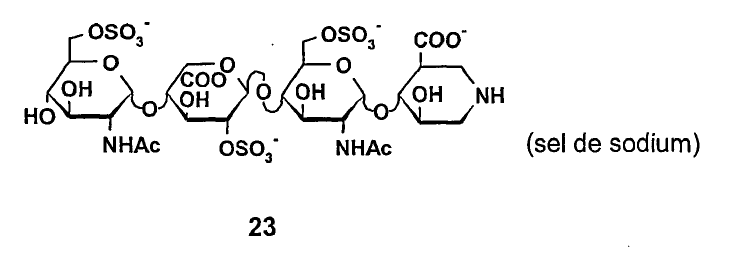

De la même manière a été préparé le composé **23.**
RMN ¹H (D₂O) δ 5.26 (d, H-1 Glc^{II}), 5.14 (d, H-1 IdoUA^{III}), 5.09 (d, H-1 Glc^{IV}).

### EXEMPLE 5 :

### Synthèse du (2,4-di-O-sodium sulfonato-α-L-idopyranosyluronate de sodium)-(1-4)-(2-N-sodium sulfonato-2-désoxy-6-O-sodium sulfonato-α-D-glucopyranosyl)-(1-4)-(2-O-sodium sulfonato-α-L-idopyranosyluronate de sodium)-(1-4)-(2-N-sodium sulfonato-2-désoxy-6-O-sodium sulfonato-a-D-glucopyranosyl)-(1-4)-(5-(hydroxy)-4-oxypipéridine-3-carboxylate de sodium (3S, 4R, 5R)) (composé n°27)

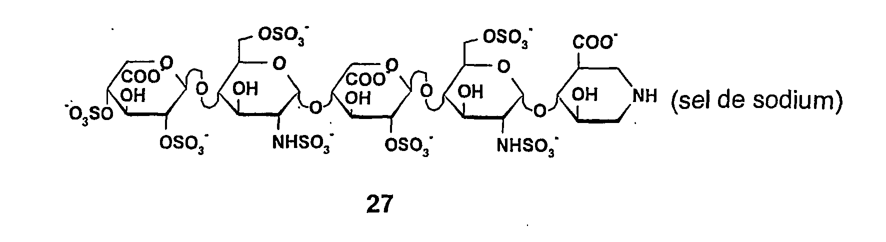

Le composé **26** de la **PREPARATION 4** (7.0 mg, 3.28 µmol) est traité selon la méthode 5 pour conduire au composé 27 (2.9 mg, 55%).
RMN ¹H (D₂O) δ 5.53 (d, H-1 Glc^{II}), 5.44 (d, H-1 Glc^{IV}), 5.23 (d, H-1 IdoUA^{III}), 5.21 (d, H-1 IdoUA^{V}), 3.09, 3.07, 2.58, 2.44 (4m, 4H, H-2, H-2', H-6, H-6' pip^{I}).

### EXEMPLE 6 :

### Synthèse du (3-O-méthyl-2,4-di-O-sodium sulfonato-α-L-idopyranosyluronate de sodium)-(1-4)-(3-O-méthyl-2,6-di-O-sodium sulfonato-α-D-glucopyranosyl)-(1-4)-(3-O-méthyl-2-O-sodium sulfonato-α-L-idopyranosyluronate de sodium)-(1-4)-(3-O-méthyl-2,6-di-O-sodium sulfonato-α-D-glucopyranosyl)-(1-4)-(5-(hydroxy)-4-oxypipéridine-3-carboxylate de sodium (3S, 4R, 5R)) (composé n°47)

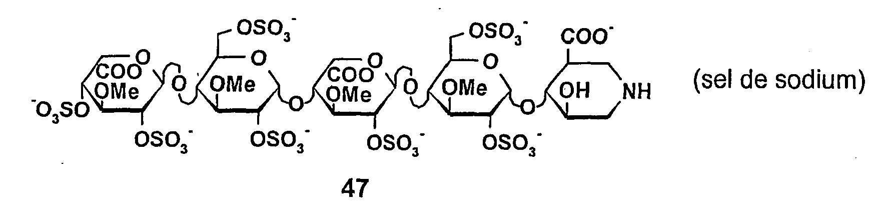

Le composé **46** de la **PREPARATION 6** est traité selon la méthode 5 pour donner, après purification, le composé 47 (5 mg, 57%).
Spectre de masse (ESI) *m*/*z* 1650.9 [(M - Na + H)⁻].

### EXEMPLE 7 :

### Synthèse du (2,4-di-O-sodium sulfonato-α-L-idopyranosyluronate de sodium)-(1-4)-(2,6-di-O-sodium sulfonato-α-D-glucopyranosyl)-(1-4)-(2-O-sodium sulfonato-α-L-idopyranosyluronate de sodium)-(1-4)-(2,6-di-O-sodium sulfonato-α-D-glucopyranosyl)-(1-4)-(5-(hydroxy)-4-oxypipéridine-3-carboxylate de sodium (3S, 4R, 5R)) (composé n° 69)

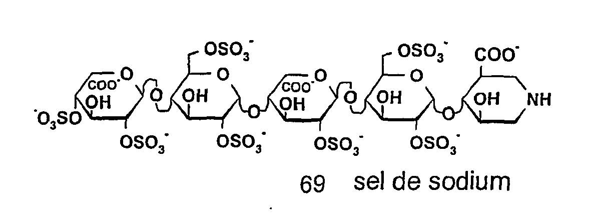

Le composé brut **68** de la **PREPARATION 9,** est traité selon la METHODE 5 pour fournir 69 (3.8 mg, 25%, deux étapes).
RMN ¹H (D₂O) δ 5.62 (d, H-1 Glc^{IV}), 5.52 (d, H-1 Glu^{II}), 5.21 (d, H-1 IdoUA^{V}), 4.99 (d, H-1 IdoUA^{III}).

### EXEMPLE 8 :

### Synthèse du (4-O-propyl-2-O-sodium sulfonato-α-L-idopyranosyluronate de sodium)-(1-4)-(2,6-di-O-sodium sulfonato-α-D-glucopyranosyl)-(1-4)-(2-O-sodium sulfonato-α-L-idopyranosyluronate de sodium)-(1-4)-(2,6-di-O-sodium sulfonato-α-D-glucopyranosyl)-(1-4)-(5-(hydroxy)-4-oxypipéridine-3-carboxylate de sodium (3S, 4R, 5R)) (composé n°74)

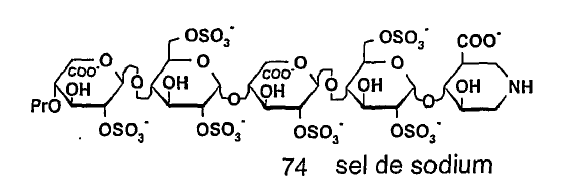

Le composé brut **73** de la **PREPARATION 10,** est traité selon la METHODE 5 pour fournir **74** (6.0 mg, 62%, deux étapes).
RMN ¹H (D₂O) δ 5.68 (d, H-1 Glc^{IV}), 5.55 (d, H-1 Glu^{II}), 5.21 (d, H-1 IdoUA^{V}), 5.18 (d, H-1 IdoUA^{III}).

### EXEMPLE 9 :

### Synthèse du méthyl (2,4-di-O-sodium sulfonato-α-L-idopyranosyluronate de sodium)-(1-4)-(2-acétamido-2-désoxy-6-O-sodium sulfonato-α-D-glucopyranosyl)-(1-4)-(5-hydroxy-4-oxypipéridin-1-yl-3-carboxylate de sodium (3S, 4R, 5R))-(1-4)-(2-N-sodium sulfonato-2,4-didésoxy-4-methyl-3,6-di-O-sodium sulfonato-α-D-glucopyranosyl)-(1-4)-(sodium 2-O-sodium sulfonato-α-L-idopyranosyluronate)-(1-4)-(2-N-sodium sulfonato-2-désoxy-6-O-sodium suifonato-α-D-glucopyranoside) (composé n° 90)

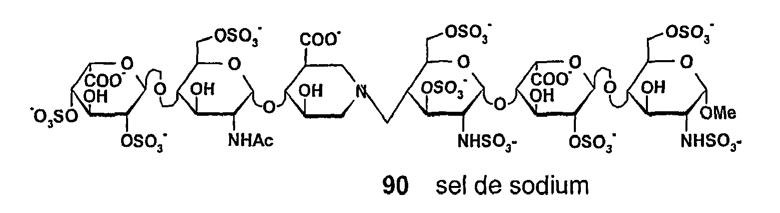

A une solution du composé **89** (24 mg, 26.9 µmol) de la **PREPARATION 11,** et du composé **22** (61 mg, 1.9 équivalent molaire) de **l'EXEMPLE 3** dans du tampon phosphate (pH 7) est ajouté, à 0 °C, du cyanoborohydrure de sodium (4.4 mg, 2.4 molequiv). Après 8h d'agitation à 0 °C, le milieu réactionnel est placé à température ambiante pendant 16h, puis purifié successivement sur une colonne de gel Sephadex^{®} G-25 éluée par du chlorure de sodium 0.2 M suivi de la même colonne Sephadex^{®} G-25 éluée à l'eau pour donner le composé **90** (17 mg, 31%).
Spectre de masse (ESI) *m*/*z* 2029.3 [(M + H - Na)⁻].

### EXEMPLE 10 :

### Synthèse du méthyl (2,4-di-O-sodium sulfonato-α-L-idopyranosyluronate de sodium)-(1-4)-(2-acétamido-2-désoxy-6-O-sodium sulfonato-α-D-glucopyranosyl)-(1-4)-(5-hydroxy-4-oxypipéridin-1-yl-3-carboxylate de sodium (3S, 4R, 5R))-(1-4)-(2-N-sodium sulfonato-2,4-didésoxy-4-methyl-6-O-sodium sulfonato-α-D-glucopyranosyl)-(1-4)-(sodium 2-O-sodium sulfonato-α-L-idopyranosyluronate)-(1-4)-(2-N-sodium sulfonato-2-désoxy-6-O-sodium sulfonato-α-D-gtucopyranoside) (composé n° 91)

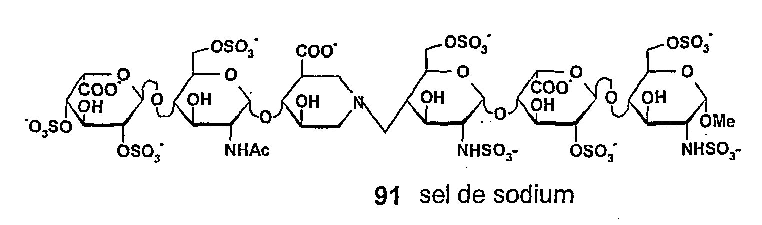

De la même manière a été préparé le composé **91**.
Spectre de masse (ESI) *m*/*z* 1927.1 [(M + H - Na)⁻].

### EXEMPLE 11 :

### Synthèse du méthyl (5-hydroxy-4-oxypipéridin-1-yl-3-carboxylate de sodium (3S, 4R, 5R))-(1-4)-(2-N-sodium sulfonato-2,4-didésoxy-4-methyl-3,6-di-O-sodium sulfonato-α-D-glucopyranosyl)-(1-4)-(sodium 2-O-sodium sulfonato-α-L-idopyranosyluronate)-(1-4)-(2-N-sodium sulfonato-2-désoxy-6-O-sodium sulfonato-α-D-glucopyranoside) (composé n° 92)

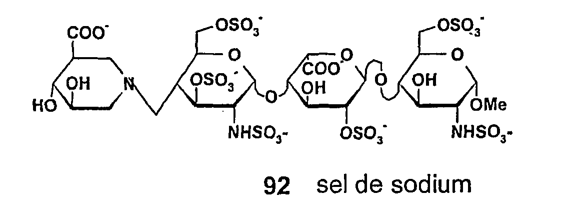

De la même manière a été préparé le composé **92**.
Spectre de masse (ESI) *m*/*z* 1321.1 [(M + H - Na)⁻].

### EXEMPLE 12 :

### Préparation du (2-acétamido-2-désoxy-6-O-sodium sulfonato-α-D-glucopyranosyl)-(1-4)-(5-hydroxy-4-oxypipéridin-1-méthyl-3-carboxylate de sodium (3S, 4R, 5R)) (composé n° 98)

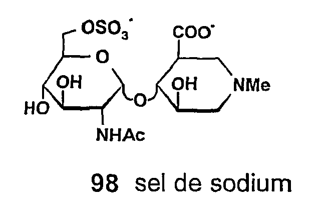

Le composé **97** de la **PREPARATION 12** (20 mg, 21.3 µmol), est traité selon la METHODE 5 dans un mélange 3:1:1 méthanol - acide acétique - eau sous courant d'hydrogène pour conduire au composé 98 (6.0 mg, 57%).
Spectre de masse (ESI) *m*/*z* 456.8 [(M - H)⁻].

### EXEMPLE 13 :

### Synthèse du (2,4-di-O-sodium sulfonato-α-L-idopyranosyluronate de sodium)-(1-4)-(2-acétamido-2-désoxy-6-O-sodium sulfonato-α-D-glucopyranosyl)-(1-4)-(2-O-sodium sulfonato-α-L-idopyranosyluronate de sodium)-(1-4)-(2-acétamido-2-désoxy-6-O-sodium sulfonato-α-D-glucopyranosyl)-(1-4)-(5-(hydroxy)-3-hydroxyméthyl-4-oxypipéridine-(3R, 4R, 5R)) (composé n° 123)

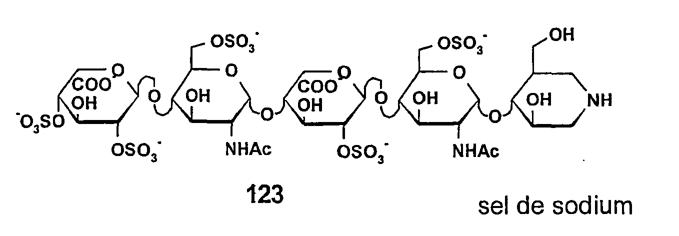

Le composé **104** (41 mg, 19 µmol) est traité selon la méthode 5 pour conduire au composé **123** (10.7 mg, 38%).
RMN ¹H (D₂O) δ 5.28 (d, H-1 Glc^{II}), 5.20 (d, H-1 IdoUA^{III}), 5.17 (d, H-1 IdoUA^{V}), 5.15 (d, H-1 Glc^{IV}), 3.21, 3.19, 2.68, 2.59 (4m, 4H, H-2, H-2', H-6, H-6' pip^{I}).

### EXEMPLE 14 :

### Synthèse du (4-O-phénylpropyl-2-O-sodium sulfonato-α-L-idopyranosyluronate de sodium)-(1-4)-(2-acétamido-2-désoxy-6-O-sodium sulfonato-α-D-glucopyranosyl)-(1-4)-(2-O-sodium sulfonato-α-L-idopyranosyluronate de sodium)-(1-4)-(2-acétamido-2-désoxy-6-O-sodium sulfonato-α-D-glucopyranosyl)-(1-4)-(5-(hydroxy)-4-oxypipéridine-3-carboxylate de sodium (3S, 4R, 5R)) (composé n° 124)

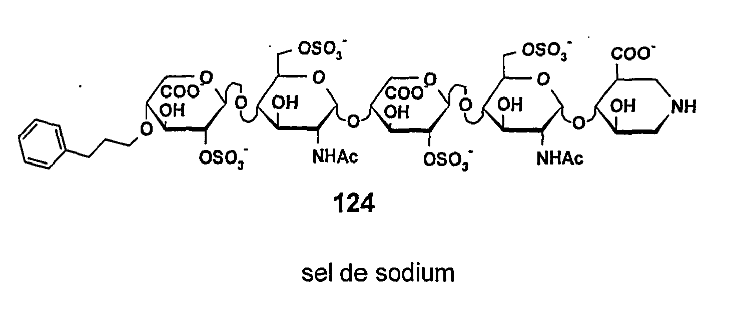

Le composé **122** est traité selon la méthode 5 pour conduire au composé **124.**
RMN ¹H (D₂O) δ 5.28 (d, H-1 Glc^{II}), 5.21 (d, H-1 IdoUA^{V}), 5.16 (d, H-1 IdoUA^{III}), 5.16 (d, H-1 Glc^{IV}), 3.23, 3.20, 2.93, 2.75 (4m, 4H, H-2, H-2', H-6, H-6' pip^{I}).

## Revendications

1. Composés de formule générale (I) : 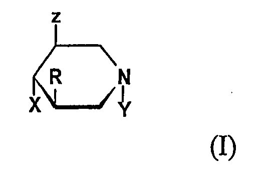 dans laquelle :
R représente un atome d'hydrogène, un radical hydroxyle, un radical -OSO₃⁻, un radical -O-(C₁-C₅)alkyle ou un radical -O-aralkyle ;
Z représente un radical COO⁻ ou un radical hydroxyle
X représente un radical hydroxyle ou une unité saccharidique de formule A : 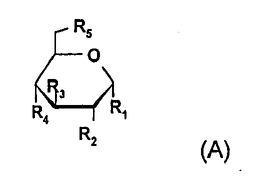 dans laquelle :
- R₁ représente un atome d'oxygène, permettant à A de se lier à l'unité azasucre ou à une autre unité saccharidique,
- R₂ représente un radical -NH₂, un radial -NHCO(C₁-C₅)alkyle, un radical -NHCOaryle, un radical -NHSO₃⁻, un radical hydroxyle, un radical -O-(C₁-C₅)alkyle, un radical -O-aralkyle ou un radical -OSO₃⁻,
- R₃ représente un radical hydroxyle, un radical -OSO₃⁻, un radical -O-(C₁-C₅)alkyle ou un radical O-aralkyle,
- R₄ représente un radical hydroxyle, un radical -OSO₃⁻, un radical -O-(C₁-C₅)alkyle, un radical -O-aralkyle ou une unité saccharidique de formule B: 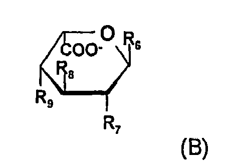 dans laquelle :
- R₆ représente un atome d'oxygène, permettant à B de se lier à une autre unité saccharidique de formule A :
- R₇ et R₈ ont la même définition que R₃ tel que défini précédemment,
- R₉ représente un groupe hydroxyle, un radical -OSO₃⁻, un radical
- O-(C₁-C₅)alkyle, un radical -O-aralkyle ou une unité saccharidique de formule A telle que définie précédemment,
- R₅ a la même définition que R₃ tel que défini précédemment ;
Y représente un atome d'hydrogène, un radical (C₁-C₅)alkyle ou une unité saccharidique de formule D 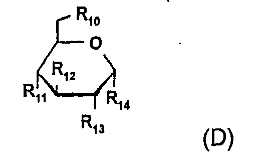 dans laquelle :
- R₁₀, R₁₂ et R₁₃ ont respectivement les mêmes définitions que R₅, R₃ et R₂ tels que définis précédemment,
- R₁₁ représente :
• un radical (C₁-C₃)alkylène permettant à D de se greffer sur l'unité azasucre ou
• un atome d'oxygène permettant à D de se greffer sur une autre unité saccharidique,
- R₁₄ représente un radical -O-(C₁-C₅)alkyle ou un radical de formule -O-E dans laquelle E représente un radical de formule : 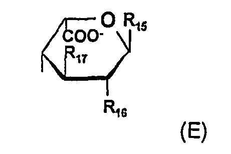 dans laquelle :
- R₁₅ représente un radical -O-(C₁-C₅)alkyle, un radical -O-aralkyle ou une unité saccharidique de formule D dans laquelle R₁₁ représente un atome d'oxygène,
- R₁₆ et R₁₇ ont la même définition que R₃ tel que défini précédemment,
à la condition toutefois que lorsque X et R représentent chacun un radical hydroxyle, Y ne représente pas un atome d'hydrogène,
et étant entendu que le nombre d'unités saccharidiques comportées par le composé de formule (I) est compris entre 1 et 10,
sous forme libre ou sous forme de sels formés avec une base ou un acide pharmaceutiquement acceptables ainsi que sous forme de solvates ou d'hydrates.

2. Composés selon la revendication 1 de formule générale (I) : 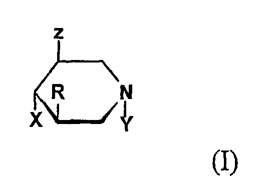 dans laquelle :
R représente un atome d'hydrogène, un radical hydroxyle, un radical -OSO₃⁻, un radical -O-(C₁-C₅)alkyle ou un radical -O-aralkyle ;
Z représente un radical COO⁻ ou un radical hydroxyle
X représente un radical hydroxyle ou une unité saccharidique de formule A : 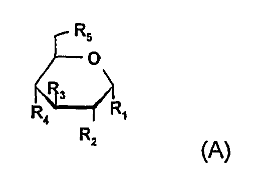 dans laquelle :
- R₁ représente un atome d'oxygène,
- R₂ représente un radical -NHCOCH₃, un radical -NHSO₃⁻, un radical -OSO₃⁻,
- R₃ représente un radical hydroxyle ou un radical -O-(C₁-C₅)alkyle,
- R₄ représente un radical hydroxyle, un radical -O-aralkyle ou une unité saccharidique de formule B : 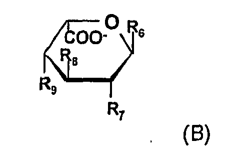 dans laquelle :
- R₆ représente un atome d'oxygène,
- R₇ représente un radical -OSO₃⁻,
- R₈ représente un radical hydroxyle, un radical -O-(C₁-C₅)alkyle ou un radical -O-aralkyle,
- R₉ représente un radical -OSO₃⁻, un radical -O-aralkyle, un radical -O-(C₁-C₅)alkyle ou une unité saccharidique de formule A telle que définie précédemment,
- R₅ représente un radical -OSO₃⁻ ;
Y représente un atome d'hydrogène ou une unité saccharidique de formule D : 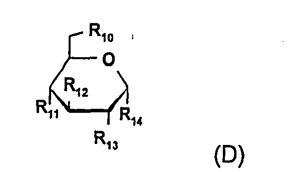 dans laquelle :
- R₁₀ a la même définition que R₅ tel que défini précédemment,
- R₁₂ représente un radical hydroxyle ou un radical -OSO₃⁻,
- R₁₃ représente un radical -NHSO₃⁻,
- R₁₁ représente un radical méthylène lié à l'unité azasucre ou un atome d'oxygène lié à E,
- R₁₄ un radical -OCH₃ ou un radical de formule -O-E dans laquelle E représente un radical de formule : 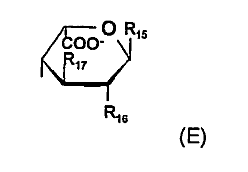 dans laquelle :
- R₁₅ représente une unité D dans laquelle R₁₁ représente un atome d'oxygène permettant de lier E à D,
- R₁₆ représente un radical -OSO3⁻,
- R₁₇ représente un radical hydroxyle,
étant entendu que le nombre d'unités saccharidiques comportées par le composé de formule générale (I) est compris entre 2 et 10,
sous forme libres ou sous forme de sels avec une base ou un acide pharmaceutiquement acceptables ainsi que sous forme de solvats ou d'hydrates.

3. Composés de formule générale (I) selon l'une quelconque des revendications 1 ou 2 dans laquelle Y est un atome d'hydrogène, et Z un radical COO⁻.

4. Composés selon l'une quelconque des revendications 1 à 3, choisis parmi:
• (2,4-di-O-sodium sulfonato-α-L-idopyranosyluronate de sodium)-(1-4)-(2-acétamido-2-désoxy-6-O-sodium sulfonato-α-D-glucopyranosyl)-(1-4)-(2-O-sodium sulfonato-α-L-idopyranosyluronate de sodium)-(1-4)=(2-acétamido-2-désoxy-6-O-sodium sulfonato-α-D-glucopyranosyl)-(1-4)-(5-(hydroxy)-4-oxypipéridine-3-carboxylate de sodium (3S, 4R, 5R)),
• (2,4-di-O-sodium sulfonato-α-L-idopyranosyluronate de sodium)-(1-4)-(2-N-sodium sulfonato-2-désoxy-6-O-sodium sulfonato-α-D-glucopyranosyl)-(1-4)-(2-O-sodium sulfonato-α-L-idopyranosyluronate de sodium)-(1-4)-(2-N-sodium sulfonato-2-désoxy-6-O-sodium sulfonato-α-D-glucopyranosyl)-(1-4)-(5-(hydroxy)-4-oxypipéridine-3-carboxylate de sodium (3S, 4R, 5R)),
• (3-O-méthyl-2,4-di-O-sodium sulfonato-α-L-idopyranosyluronate de sodium)-(1-4)-(3-O-méthyl-2,6-di-O-sodium sulfonato-α-D-glucopyranosyl)-(1-4)-(3-O-méthyl-2-O-sodium sulfonato-α-L-idopyranosyluronate de sodium)-(1-4)-(3-O-méthyl-2,6-di-O-sodium sulfonato-α-D-glucopyranosyl)-(1-4)-(5-(hydroxy)-4-oxypipéridine-3-carboxylate de sodium (3S, 4R, 5R)),
• (2,4-di-O-sodium sulfonato-α-L-idopyranosyluronate de sodium)-(1-4)-(2,6-di-O-sodium sulfonato-α-D-glucopyranosyl)-(1-4)-(2-O-sodium sulfonato-α-L-idopyranosyluronate de sodium)-(1-4)-(2,6-di-O-sodium sulfonato-α-D-glucopyranosyl)-(1-4)-(5-(hydroxy)-4-oxypipéridine-3-carboxylate de sodium (3S, 4R, 5R)),
• (4-O-propyl-2-O-sodium sulfonato-α-L-idopyranosyluronate de sodium)-(1-4)-(2,6-di-O-sodium sulfonato-α-D-glucopyranosyl)-(1-4)-(2-O-sodium sulfonato-α-L-idopyranosyluronate de sodium)-(1-4)-(2,6-di-O-sodium sulfonato-α-D-glucopyranosyl)-(1-4)-(5-(hydroxy)-4-oxypipéridine-3-carboxylate de sodium (3S, 4R, 5R)).
• (2,4-di-O-sodium sulfonato-α-L-idopyranosyluronate de sodium)-(1-4)-(2-acétamido-2-désoxy-6-O-sodium sulfonato-α-D-glucopyranosyl)-(1-4)-(2-O-sodium sulfonato-α-L-idopyranosyluronate de sodium)-(1-4)-(2-acétamido-2-désoxy-6-O-sodium sulfonato-α-D-glucopyranosyl)-(1-4)-(3-(hydroxy)-5-hydroxyméthyl-4-oxypipéridine-(3R, 4R, 5R))
• (4-O-phénylpropyl-2-O-sodium sulfonato-α-L-idopyranosyluronate de sodium)-(1-4)-(2-acétamido-2-désoxy-6-O-sodium sulfonato-α-D-glucopyranosyl)-(1-4)-(2-O-sodium sulfonato-α-L-idopyranosyluronate de sodium)-(1-4)-(2-acétamido-2-désoxy-6-O-sodium sulfonato-α-D-glucopyranosyl)-(1-4)-(5-(hydroxy)-4-oxypipéridine-3-carboxylate de sodium (3S, 4R, 5R))

5. Compositions pharmaceutiques contenant, en tant que principe actif, un composé de formule générale (I) selon l'une quelconque des revendications 1 à 4, éventuellement en association avec un ou plusieurs excipients inertes et appropriés.

6. Composition pharmaceutique selon la revendication 5, utile dans le traitement de maladies dans lesquelles les héparanases sont impliquées.

7. Composition pharmaceutique selon la revendication 5, utile dans le traitement des carcinomes ayant un degré de vascularisation important, tels que le carcinome de poumon, sein, prostate et oesophage, les cancers induisant des métastases tels que le cancer du colon et le cancer de l'estomac, des mélanomes, des gliomes, des lymphomes ou des leucémies.

8. Composition pharmaceutique selon la revendication 5, utile dans le traitement de maladies cardiovasculaires telles que l'athérosclérose, la resténose post angioplastie, de maladies liées aux complications apparaissant suite à la pose de prothèse endovasculaires et/ou de pontages aorto-coronariens ou d'autres greffes vasculaires de l'hypertrophie cardiaque ou de complications vasculaires du diabète comme les rétinopathies diabétiques.

9. Composition pharmaceutique selon la revendication 5, utile dans le traitement de maladies inflammatoires chroniques comme l'arthrite rhumatoïde ou les IBD.

10. Composition pharmaceutique selon la revendication 5, utile dans le traitement de la dégénérescence maculaire.

## Claims

1. Compounds of general formula (I): 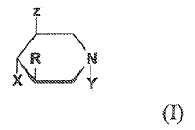 in which:
R represents a hydrogen atom, a hydroxyl radical, an -OSO₃⁻ radical, an -O-(C₁-C₅)alkyl radical or an O-aralkyl radical;
Z represents a COO⁻ radical or a hydroxyl radical;
X represents a hydroxyl radical or a saccharide unit of formula A: 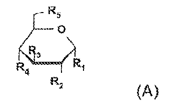 in which:
- R₁ represents an oxygen atom, allowing A to bind to the azasugar unit or to another saccharide unit,
- R₂ represents an -NH₂ radical, an -NHCO(C₁-C₅)-alkyl radical, an -NHCOaryl radical, an -NHSO₃⁻ radical, a hydroxyl radical, an -O-(C₁-C₅)alkyl radical, an -O-aralkyl radical or an -OSO₃⁻ radical,
- R₃ represents a hydroxyl radical, an -OSO₃⁻ radical, an -O-(C₁-C₅)alkyl radical or an 5 -O-aralkyl radical,
- R₄ represents a hydroxyl radical, an -OSO₃⁻ radical, an -O-(C₁-C₅)alkyl radical, an -O-aralkyl radical or a saccharide unit of formula B: 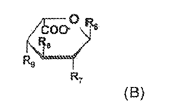 in which:
- R₆ represents an oxygen atom, allowing B to bind to another saccharide unit of formula A,
- R₇ and R₈ have the same definition as R₃ as defined above,
- R₉ represents a hydroxyl group, an -OSO₃⁻ radical, an -O-(C₁-C₅)alkyl radical, an
- O-aralkyl radical or a saccharide unit of formula A as defined above,
- R₅ has the same definition as R₃ as defined above;
Y represents a hydrogen atom, a (C₁-C₅)alkyl radical or a saccharide unit of formula D 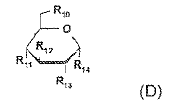 in which:
- R₁₀, R₁₂ and R₁₃ have the same definitions as R₅, R₃ and R₂ respectively as defined above,
- R₁₁, represents:
• a (C₁-C₃)alkylene radical allowing D to attach to the azasugar unit, or
• an oxygen atom allowing D to attach to another saccharide unit,
- R¹⁴ represents an -O-(C₁-C₅) alkyl radical or a radical of formula -O-E in which E represents a radical of formula: 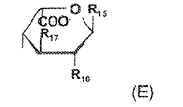 in which;
- R₁₅ represents an -O-(C₁-C₅)alkyl radical, an -O-aralkyl radical or a saccharide unit of formula D in which R₁₁ represents an oxygen atom,
- R₁₆ and R₁₇ have the same definition as R₃ as defined above,
provided, however, that when X and R each represent a hydroxyl radical, Y does not represent a hydrogen atom,
and it being understood that the number of saccharide units of which the compound of formula (I) is composed is between 1 and 10,
in free form or in the form of salts formed with a pharmaceutically acceptable base or acid, and in the form of solvates or hydrates.

2. Compounds according to Claim 1, of general formula (I): 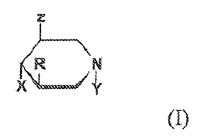 in which:
R represents a hydrogen atom, hydroxyl radical, an -OSO₃⁻ radical, an -O-(C₁-C₅)alkyl radical or an -O-aralkyl radical;
Z represents a COO⁻ radical or a hydroxyl radical;
X represents a hydroxyl radical or a saccharide unit of formula A: 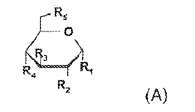 in which:
- R₁ represents an oxygen atom,
- R₂ represents an -NHCOCH₃ radical, an -NHSO₃⁻ radical, an -OSO₃ radical,
- R₃ represents a hydroxyl radical or an
- O-(C₁-C₅)alkyl radical,
- R₄ represents a hydroxyl radical, an -O-aralkyl radical or a saccharide unit of formula B: 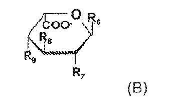 in which:
- R₆ represents an oxygen atom,
- R₇ represents an -OSO₃⁻ radical,
- R₈ represents a hydroxyl radical, an -O-(C₁-C₅)alkyl radical or an -O-aralkyl radical,
- R₉ represents an -OSO₃⁻ radical, an -O-aralkyl radical, an -O-(C₁-C₅)alkyl radical or a saccharide unit of formula A as defined above,
- R₅ represents an -OSO₃⁻ radical;
Y represents a hydrogen atom or a saccharide unit of formula D: 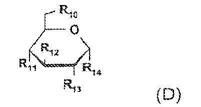 in which:
R₁₀ has the same definition as R₅ as defined above,
- R₁₂ represents a hydroxyl radical or an -OSO₃⁻ radical,
- R₁₃ represents an -NHSO₃⁻ radical,
- R₁₁ represents a methylene radical linked to an azasugar unit or an oxygen atom linked to E,
- R₁₄ an -OCH₃ radical or a radical of formula -O-E in which E represents a radical of formula: 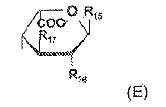 in which:
- R₁₅ represents a D unit in which R₁₁ represents an oxygen atom allowing E to be lined to D,
- R₁₆ represents an -OSO₃⁻ radical,
- R₁₇ represents a hydroxyl radical,
it being understood that the number of saccharide units of which the compound of formula (I) is composed is between 2 and 10,
in free form or in the form of salts with a pharmaceutically acceptable base or acid, and in the form of solvates or hydrates.

3. Compounds of general formula (I) according to either of Claims 1 and 2, in which Y is a hydrogen atom and Z a COO⁻ radical.

4. Compounds according to any one of Claims 1 to 3, chosen from:
• (2,4-di-O-sodium sulphonato-α-L-idopyranosyluronate of sodium)-(1-4)-(2-acetamido-2-deoxy-6-O-sodium sulphonato-α-D-glucopyratiosyl)-(1-4)-(2-O-sodium sulphonato-α-L-idopyranosyluronate of sodium)-(1-4)-(2-acetamido-2-deoxy-S-O-sodium) sulphonato-α-D-glucopyranosyl)-(1-4)-(5-(hydroxy)-4-oxypiperidine-3-carboxylate of sodium (3S, 4R, 5R))
• (2,4-di-O-sodium sulphonato-α-L-idopyranosyluronate of sodium)-(1-4)-(2-N-sodium sulphonato-2-deoxy-6-O-sodium sulphonato-α-D-glucopyranosyl)-(1-4)-(2-O-sodium sulphonato-α-L-idopyranosyluronate of sodium)-(1-4)-(2-N-sodium sulphonato-2-deoxy-6-O-sodium sulphonato-α-D-glucopyranogyl)-(1-4)-(5-(hydroxy)-4-oxypiperidine-3-carboxylate of sodium (3S, 4R, 5R))
• (3-O-methyl-2,4-di-O-sodium sulphonato-α-L-idopyranosyluronate of sodium)-(1-4)-(3-O-methyl-2,6-di-O-sodium sulphonato-α-D-glucopyranosyl)-(1-4)-(3-O-methyl-2-O-sodium sulphonato-α-L-idopyranosyluronate of sodium) (1-4)-(3-O-methyl-2,6-di-O-sodium sulphonato-α-D-glucopyranosyl)-(1-4)-(5-(hydroxy)-4-oxypiperidine-3-carboxylate of sodium (3S, 4R, 5R))
• (2,4-di-O-sodium sulphonato-α-L-idopyranosyluronate of sodium)-(1-4)-(2,6)-di-O-sodium sulphonato-α-D-glucopyranosyl)-(1-4)-(2-O-sodium sulphonato-α-L-idopyranosyluronate of sodium)-(1-4)-(2,6-di-O-sodium sulphonato-α-D-glucopyranosyl)-(1-4)-(5-(hydroxy)-4-oxypiperidine-3-carboxylate of sodium (3S, 4R, 5R))
• (4-O-propyl-2-O-sodium sulphonato-α-L-idopyranosyluronate of sodium)-(1-4)-(2,6-di-O-sodium sulphonato-α-D-glucopyransyl-(1-4)-(2-O-sodium sulphonato-α-L-idopyranosyluronate of sodium)-(1-4)-(2,6-di-O-sodium sulphonato-α-D-glucopyranosyl)-(1-4)-(5-(hydroxy)-4-oxypiperidine-3-carboxylate of sodium (3S, 4R, 5R))
• (2,4-di-O-sodium sulphonato-α-L-idopyranosyluronate of sodium)-(1-4)-(2-acetamido-2-deoxy-6-O-sodium sulphonato-α-D-glucopyranosyl)-(1-4)-(2-O-sodium sulphonato-α-L-idopyranosyluronate of sodium)-(1-4)-(2-acetamido-2-deoxy-6-O-sodium sulphonato-ϕ-D-glucopyranosyl)-(1-4)-(3-(hydroxy)-5-hydroxymethyl-4-oxypiperidine (3R, 4R, 5R))
• (4-O-phenylpropyl-2-O-sodium sulphonato-α-L-idopyranosylurenate of sodium)-(1-4)-(2-acetamido-2-deoxy-6-O-sodium sulphonato-α-D-glucopyranosyl)-(1-4)-(2-O-sodium sulphonato-α-L-idopyranosyluronate of sodium)-(1-4)-(2-acetamido-2-deoxy-6-O-sodium sulphonato-α-D-glucopyranosyl)-(1-4)-(5-(hydroxy)-4-oxypiperidine-3-carboxylate of sodium (3S, 4R, 5R)).

5. Pharmaceutical compositions containing, as active ingredient, a compound of general formula (I) according to any one of Claims 1 to 4, optionally combined with one or more inert and appropriate excipients.

6. pharmaceutical composition according to Claim 5, useful in the treatment of diseases in which heparanases are involved.

7. Pharmaceutical composition according to Claim 5, useful in the treatment of carcinomas having a high degree of vascularization, such as lung, breast, prostate and oesophageal carcinoma, cancers which induce metastases such as colon cancer and stomach cancer, melanomas, glyomas, lymphomas and leukaemias.

8. Pharmaceutical composition according to Claim 5, useful in the treatment of cardiovascular diseases such as atherosclerosis, post-angioplasty restenosis, diseases linked to complications which appear following the fitting of endovascular prostheses and/or aortocoronary bypass surgery or other vascular transplants, cardiac hypertrophy or vascular complications of diabetes such as diabetic retinopathies.

9. Pharmaceutical, composition according to Claim 5, useful in the treatment of chronic inflammatory diseases such as rheumatoid arthritis or IBDs.

10. Pharmaceutical composition according to Claim 5, useful in the treatment of macular degeneration.

## Patentansprüche

1. Verbindung der allgemeinen Formel (I): 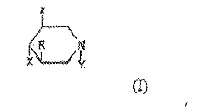 worin:
R für ein Wasserstoffatom, einen Hydroxylrest, einen Rest -OSO₃⁻, einen -O-(C₁-C₅)-Alkylrest oder einen -O-Aralkylrest steht;
Z für einen Rest COO⁻ oder einen Hydroxylrest steht;
X für einen Hydroxylrest oder eine Saccharideinheit der Formel A steht: 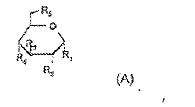
- worin:
- R₁ für ein Sauerstoffatom steht, so dass A an die Azazuckereinheit oder an eine andere Saccharideinheit binden kann,
- R₂ für einen Rest -NH₂, einen -NHCO-(C₁-C₅)Alkylrest, einen -NHCOArylrest, einen Rest-NHSO₃ , einen Hydroxylrest, einen -O-(C₁-C₅) Alkylrest, ein -O-Aralkylrest oder einen Rest - OSO₃⁻ steht,
- R₃ für einen Hydroxylrest, einen Rest -OSG₃⁻, einen -O-(C₁-C₅)Alkylrest oder einen -O-Aralkylrest steht,
- R₄ für einen Hydroxylrest, einen Rest -OS0₃⁻, einen -O-(C₁-C₅)Alkylrest, einen -O-Aralkylrest oder eine Saccharideinheit der Formel B steht: 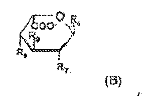
- worin:
- R₆ für ein Sauerstoffatom steht, so dass B an eine andere Saccharideinheit der Formel A binden kann,
- R₇ und R₈ dieselbe Definition haben, wie der vorstehend definierte Rest R₃,
- R₉ für eine Hydroxylgruppe, einen Rest -OSO₃⁻, einen -O-(C₁-C₅)Alkylrest, einen -O-Aralkylrest oder eine Saccharideinheit der Formel A, wie vorstehend definiert, steht,
- R₅ dieselbe Definition hat, wie der vorstehend definierte Rest R₃;
Y für ein Wasserstoffatom, einen (C₁,-C₅)Alkylrest oder eine Saccharideinheit der Formel D steht 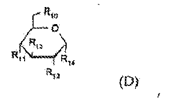
- worin:
- R₁₀, E₁₂ und R₁₃ jeweils dieselben Definitionen haben, wie die vorstehend definierten Reste R₅, R₃ beziehungsweise R₂,
- R₁₁ für:
• einen (C₁-C₃)Alkylenrest, so dass sich D an die Azazuckereinheit anheften kann, oder
• ein Sauerstoffatom, so dass sich D an eine andere Saccharideinheit anheften kann,
steht,
- R₁₄ für einen -O-(C₁-C₅)Alkylrest oder einen Rest der Formel -O-E steht, worin E für einen Rest der folgenden Formel steht: 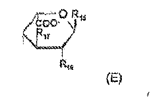 worin:
- R₁₅ für einen -O-(C₁-C₅)Alkylrest, einen -O-Aralkylrest oder eine Saccharideinheit der Formel D steht, worin R₁₁ für ein Sauerstoffatom steht,
- R₁₆ und R₁₇ dieselbe Definition haben, wie der vorstehend definierte Rest R₃,
jedoch mit der Maßgabe, dass, wenn X und R jeweils für einen Hydroxylrest stehen, Y nicht für ein Wasserstoffatom steht,
und wobei selbstverständlich ist, dass die Anzahl der Saccharideinheiten, die die vebindung der Formel (I) trägt, zwischen 1 und 10 liegt,
in freier Form oder in Form von Salzen, die mit einer pharmazeutisch annehmbaren Base oder Säure gebildet worden sind, sowie in Form von Solvaten oder Hydraten.

2. Verbindungen nach Anspruch 1 der allgemeinen Formel (I): 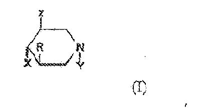 worin:
R für ein Wasserstoffatom, einen Hydroxylrest, einen Rest -OSO₃⁻, einen -O-(C₁-C₅)-Alkylrest oder einen -O-Aralkylrest steht;
Z für einen Rest COO⁻ oder einen Hydroxylrest steht;
X für einen Hydroxylrest oder eine Saccharideinheit der Formel A steht: 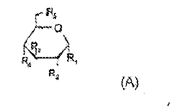
- worin:
- R₁ für ein Sauerstoffatom steht,
- R₂ für einen Rest -NHCOCH₃, einen Rest -NHSO₃⁻, einen Rest -OSO₃⁻ steht,
- R₃ für einen Hydroxylrest oder einen -O-(C₁-C₅)Alkylrest steht,
- R₄ für einen Hydroxylrest, einen -O-aralkylrest oder eine Saccharideinheit der Formel B steht: 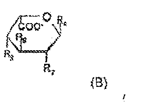
- worin:
- R₆ für ein Sauerstoffatom steht,
- R₇ für einen Rest -OSO₃ steht,
- R₈ für einen Hydroxylrest, einen -O-(C₁-C₈)Alkylrest oder einen -O-Aralkylrest steht,
- R₉ für einen Rest -OSO₃⁻, einen -O-Aralkylrest, einen -O-(C₁-C₅) Alkylrest oder eine Saccharideinheit der Formel A, wie vorstehend definiert, steht,
- R₅ für einen Rest -OSO₃ steht,
Y für ein wasserstoffatom oder eine Saccharideinheit der Formel D steht: 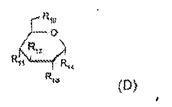
- worin:
- R₁₀ dieselbe Definition hat, wie der vorstehend definierte Rest R₅,
- R₁₂ für einen Hydroxylrest oder einen Rest -OSO₃⁻ steht,
- R₁₃ für einen Rest -NHSO₃⁻ steht,
- R₁₁ für einen Methylenrest, der an die Azazuckereinheit gebunden ist, oder ein an E gebundenes Sauerstoffatom steht,
- R₁₄ ein Rest -OCH₃ oder ein Rest der Formel -O-E, worin E für einen Rest der folgenden Formel steht: 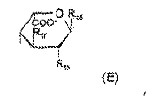
- worin:
- R₁₅ für eine Einheit D steht, worin R₁₁ für ein Sauerstoffatom steht, so dass E an D gebunden werden kann,
- R₁₆ für einen Rest -OSO₃⁻ steht,
- R₁₇ für einen Hydroxylrest steht,
wobei selbstverständlich ist, dass die Anzahl der Saccharideinheiten, die die Verbindung der Formel (I) trägt, zwischen 2 und 10 liegt,
in freier Form oder in Form von Salzen mit einer pharmazeutisch annehmbaren Base oder Säure sowie in Form von Solvaten oder Hydraten.

3. Verbindungen der allgemeinen Formel (I) nach einem der Ansprüche 1 oder 2, worin Y ein Wasserstoffatom und Z ein Rest COO⁻ ist.

4. verbindungen nach einem der Ansprüche 1 bis 3, die ausgewählt sind aus:
• (2,4-Di-O-natriumsufonato-α-L-idopyranosyluronat, Natriumsalz) (1-4)-(2-acetamido-2-desoxy-6-O-natriumsulfonato-α-D-glucopyranosyl)-(1-4)-(2-O-natriumsulfonato-α-L-idopyranosyluronat, Natriumsalz)-(1-4)-(2-acetamido-2-desoxy-6-O-natriumsulfonato-α-D-glucopyranosyl)-(1-4)-(5-(hydroxy)-4-oxypiperidin-3-carboxylat, Natriumsalz (3S, 4R, 5R));
• (2,4-Di-O-natriumsulfonato-α-L-idopyranosyluronat, Natriumsalz)-(1-4)-(2-N-natriumsulfonato-2-desoxy-6-O-natriumsulfonato-α-D-glucopyranosyl)-(1-4)-(2-O-natriumsulfonato-α-L-idopyranosyluronat, Natriumsalz)-(1-4)-(2-N-natriumsulfonato-2-desoxy-6-O-natriumsulfonato-α-D-glucopyranosyl)-(1-4)-(5-(hydroxy)-4-oxypiperidin-3-carboxylat, Natriumsalz (3S,4R,5R));
• (3-O-Methyl-2,4-di-O-natriumsulfonato-α-L-idopyranosyluronat, Natriumsalz)-(1-4)-(3-O-methyl-2,6-di-O-natriumsulfonato-α-D-glucopyranosyl)-(1-4)-(3-O-methyl-2-O-natriumsulfonato-α-L-idopyranosyluronat, Natriumsalz)-(1-4)-(3-O-methyl-2,5-di-O-natriumsulfonato-α-D-glucopyranosyl)-(1-4)-(5-(hydroxy)-4-oxypiperidin-3-carboxylat, Natriumsalz (3S,4R,5R));
• (2,2-Di-O-natriumsulfonato-α-L-idopyranosyluronat, Natriumsalz)-(1-4)-(2,6)-di-O-natriumsulfonato-α-D-glucopyranosyl)-(1-4)-(2-O-natriumsulfonato-α-L-idopyranosyluronat, Natriumsalz)-(1-4)-(2,6-di-O-natriumsulfonato-α-D-glucopyranosyl) - (1-4) - (5-(hydroxy)-4-oxypiperidin-3-carboxylat, Natriumsalz (3S,4R,5R));
• (4-O-Propyl-2-O-natriumsulfonato-α-L-idopyranosyluronat, Natriumsalz)-(1-4)-(2,6-di-O-natriumsulfonato-α-D-glucopyranosyl-(1-4)-(2-O-natriumsulfonato-α-L-idopyranosyluronat, Natriumsalz)-(1-4)-(2,6-di-O-natriumsulfonato-α-D-glucopyranosyl)-(1-4)- 5-(hydroxy)-4-oxypiperidin-3-carboxylal, Natriumsalz (3S,4R,5R));
• (2,4-Di-O-natriumsulfonato-α-L-idopyranosyluronat, Natriumsalz)-(1-4)-(2-acetamido-2-desoxy-6-O-natriumsulfonato-α-D-glucopyranosyl)-(1-4)-(2-O-natriumsulfonato-α-L-idopyranosyluronat, Natriumsalz)-(1-4)-(2-acetamido-2-desoxy-6-O-natriumsulfonato-α-D-glucopyranosyl)-(1-4)-(3-(hydroxy)-5-hydroxymethyl-4-oxypiperidin-(3R,4R,5R)) und
• (4-O-Phenylpropyl-2-O-natriumsulfonato-α-L-idopyranosyluronat, Natriumsalz)-(1-4)-(2-acetamido-2-desoxy-6-O-natriumsulfonato-α-D-glucopyranosyl)-(1-4)-(2-O-natriumsulfonato-α-L-idopyranosyluronat, Natriumsalz)-(1-4)-(2-acetamido-2-desoxy-6-O-natriumsulfonato-α-D-glucopyranosyl)-(1-4)-(5-(hydroxy)-4-oxypiperidin-3-carboxylat, Natriumsalz (3S,4R,5R)).

5. Pharmazeutische Zusammensetzungen, die als Werkstoff eine Verbindung der allgemeinen Formel (I) nach einem der Ansprüche 1 bis 4, gegebenenfalls in Kombination mit einem oder mehreren inerten und geeigneten Excipienten, umfasst.

6. Pharmazeutische Zusammensetzung nach Anspruch 5, die sich zur Behandlung von Krankheiten eignet, bei denen Heparanasen impliziert werden.

7. Pharmazeutische Zusammensetzung nach Anspruch 5, die zur Behandlung von Karzinomen mit einem erheblichen Grad an Vaskularisierung, wie Lungen-, Brust-, Prostata- und Ösophaguskarzinom, Krebserkrankungen, die Metastasen induzieren, wie Enddarmkrebs und Magenkrebs, Melanome, Gliome, Lymphome und Leukämien, geeignet ist.

8. Pharmazeutische Zusammensetzung nach Anspruch 5, die zur Behandlung von kardiovaskulären Krankheiten, wie Atherosklerose, Restenose nach Angiolastie, von Krankheiten in Verbindung mit Komplikationen, die nach dem Legen von endovaskulären Prothesen und/oder von aortokoronaren Bypässen oder anderen Vefäßtransplantaten auftreten, von Herzhypertrophie oder Gefäßkomplikationen von Diabetes, wie diabetische Retinopathien, geeignet ist.

9. Pharmazeutische Zusammensetzung nach Anspruch 5, die zur Behandlung von chronischen Entzündungskrankheiten, wie rheumatoide Arthritis oder IBD, geeignet ist.

10. Pharmazeutische Zusammensetzung nach Anspruch 5, die zur Behandlung von Nakuladegeneration geeignet ist.
